(19) Europäisches Patentamt
European Patent Office
Office européen des brevets

(11) EP 1 627 008 B1

(12) EUROPÄISCHE PATENTSCHRIFT

(45) Veröffentlichungstag und Bekanntmachung des Hinweises auf die Patenterteilung:
**28.01.2015 Patentblatt 2015/05**

(21) Anmeldenummer: **04726174.8**

(22) Anmeldetag: **07.04.2004**

(51) Int Cl.:
*C08G 77/388* (2006.01) *C08G 77/452* (2006.01)

(86) Internationale Anmeldenummer:
**PCT/EP2004/050472**

(87) Internationale Veröffentlichungsnummer:
**WO 2004/090007 (21.10.2004 Gazette 2004/43)**

# (54) REAKTIVE AMINO- UND/ODER AMMONIUM-POLYSILOXANVERBINDUNGEN

REACTIVE AMINO- AND/OR AMMONIUM-POLYSILOXANE COMPOUNDS

COMPOS S DE POLYSILOXANE AMINO ET/OU AMMONIUM R ACTIFS

(84) Benannte Vertragsstaaten:
**AT BE BG CH CY CZ DE DK EE ES FI FR GB GR HU IE IT LI LU MC NL PL PT RO SE SI SK TR**

(30) Priorität: **11.04.2003 DE 10316662**

(43) Veröffentlichungstag der Anmeldung:
**22.02.2006 Patentblatt 2006/08**

(73) Patentinhaber: **Momentive Performance Materials GmbH**
**51368 Leverkusen (DE)**

(72) Erfinder:
- **LANGE, Horst**
  **44879 Bochum (DE)**
- **WAGNER, Roland**
  **53757 St. Augustin (DE)**
- **WITOSSEK, Anita**
  **40764 Langenfeld (DE)**
- **STACHULLA, Karl-Heinz**
  **51379 Leverkusen (DE)**
- **SOCKEL, Karl-Heinz**
  **51373 Leverkusen (DE)**
- **MOELLER, Annette**
  **51381 Leverkusen (DE)**

(74) Vertreter: **Gille Hrabal**
**Postfach 18 04 09**
**40571 Düsseldorf (DE)**

(56) Entgegenhaltungen:
**DE-A- 10 003 322** **DE-A- 10 036 533**
**US-A- 5 998 650**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 627 008 B1

## Beschreibung

Die Erfindung betrifft reaktive, funktionelle Gruppen aufweisende Amino- und/oder Ammonium-Polysiloxanverbindungen und Salze davon, insbesondere polyquaternäre Polysiloxancopolymere und deren Salze, Verfahren zu ihrer Herstellung, ihre Verwendung zur Oberflächen-Behandlung von Substraten, wie natürlichen oder synthetischen Fasern oder faserartigen Substraten, insbesondere als waschbeständige hydrophile Weichmacher, sowie ihre Verwendung in kosmetischen Formulierungen, und Formulierungen, die die reaktiven Amino- und/oder Ammonium-Polysiloxan-verbindungen und deren Salze enthalten.

Aminogruppen enthaltende Polysiloxane sind als textile Weichmacher bekannt (EP 441530).

Die Einführung von durch Ethylenoxid-/Propylenoxideinheiten modifizierten Aminostrukturen als Seitenketten bewirkt eine Verbesserung des Hydrophilierungs-effekts (US 5591880, US 5650529). Die Alkylenoxideinheiten erlauben hierbei die gezielte Einstellung der hydrophilen-hydrophoben Balance.

Es ist weiterhin vorgeschlagen worden, $\alpha,\omega$-epoxymodifizierte Siloxane mit $\alpha,\omega$-aminofunktionalisierten Alkylenoxiden umzusetzen, und diese Produkte als hydrophile Weichmacher einzusetzen (US 5807956, US 5981681).

In Weiterentwicklung dieses Gedankens sind Blockcopolymere beschrieben worden, die durch Reaktion $\alpha,\omega$-epoxymodifizierter Siloxane und $\alpha,\omega$-epoxyfunktionalisierter Alkylenoxide mit primären Amine entstehen (US 6475568). Die primären Amine dienen als Kopplungsglieder zwischen den zwei Epoxyspezies.

Zur Verbesserung der Substantivität sind Versuche unternommen worden, quartäre Ammoniumgruppen in alkylenoxidmodifizierte Siloxane einzuführen.

Die Reaktion von $\alpha,\omega$-Diepoxiden mit tertiären Aminen in Gegenwart von Säuren liefert alkylenoxidfreie $\alpha,\omega$-diquartäre Siloxane, welche zu Haarpflegezwecken eingesetzt werden können (DE-PS 3719086).

In US 6242554 werden $\alpha,\omega$-difunktionelle Siloxanderivate beschrieben, die jeweils über eine separate quartäre Ammonium- und Alkylenoxideinheit verfügen.

Streng kammartige alkylenoxidmodifizierte Polysiloxanquats sind ebenfalls beschrie-ben worden (US 5098979, US 5153294, US 5166297). Die Hydroxylgruppen von kammartig, d.h. in den Polysiloxanseitenketten substituierten Polyethersiloxanen werden mit Epichlorhydrin oder Chloressigsäure in die entsprechenden Halogenderivate überführt. Anschließend erfolgt eine Quaternierung mit tertiären Aminen.

Die Reaktion von $\alpha,\omega$-Diepoxysiloxanen mit di-tertiären Aminen in Gegenwart von Säuren liefert alkylenoxidfreie langkettige polyquaternäre Polysiloxane (EP 282720). Analoge Verbindungen auf Basis von $\alpha,\omega$-chlorpropylsubstituierten Siloxanen sind in DE 3340708 beschrieben. Aromatische Derivate, bevorzugt auf Basis Imidazol, werden in US 6240929 behandelt. Die Nutzung dieser polyquaternierten aroma-tischen Strukturen soll die Auswaschbeständigkeit aus Haaren unter Einwirkung milder Shampoos verbessern.

Lineare Siloxancopolymere, die Alkylenoxid- und Quatstrukturen enthalten, werden in DE 198 17 776 beansprucht.

Keiner der behandelten Vorschläge lieferte allerdings eine befriedigende Lösung für das Problem, den durch Silicone möglichen weichen Griff und die ausgeprägte Hydrophilie nach Erstausrüstung eines Textilmaterials auch dann zu erhalten, wenn dieses dem Angriff aggressiver Detergenzienformulierungen im Verlauf wiederholter Waschprozesse bei gegebenenfalls erhöhter Temperatur ausgesetzt wird. Polyquaternierte Polysiloxane werden in DE 100 51 258 als Weichmacher für Waschmittel und in DE 100 36 533 zusätzlich als Weichmacher für die textile Erstausrüstung beansprucht.

In den Schriften WO 02/10256, WO 02/10257, WO 02/10259 der Anmelderin werden alkylenoxidmodifizierte polyquaternierte Polysiloxane als Weichmacher in Waschmitteln und für die textile Erstausrüstung behandelt. Gemäß dieser Patenten kann die Hydrophilie durch gezielten Einbau von u.a. Alkylenoxideinheiten gesteigert und gleichzeitig eine exzellente Weichheit und Substantivität erreicht werden.

In den nicht vorveröffentlichten Schriften der Anmelderin DE 102 51 525.5 und DE 102 51 526.3 wird vorgeschlagen, durch Oligomerisierung der Quatstrukturen zwischen den Siliconblöcken eine höhere Ladungsdichte einzustellen, was sich positiv auf Substantivität und Hydrophilie auswirkt.

Vernetzte und/oder verzweigte Strukturen sind ebenfalls vorgeschlagen worden. Gemäß US 6177511 erfolgt eine Vernetzung durch Kombination von Aminosiloxanen mit polyfunktionellen Acrylsäurederivaten in einer Michael-Addition analogen Reaktion. Quaternäre Ammoniumgruppen können als Acrylatderivaten eingebracht werden.

Verzweigte alkylenoxidmodifizierte Polysiloxanpolyquats sind aus $\alpha,\omega$-OH terminierten Polysiloxanen und Trialkoxysilanen durch Kondensation synthetisiert worden. Die quartäre Ammoniumstruktur wird über das Trialkoxysilan eingebracht, wobei das quartäre Stickstoffatom durch Alkylenoxideinheiten substituiert ist (US 5602224).

In einer nicht vorveröffentlichten Schrift der Anmelderin (DE 102 12 470.1) wird durch gezielten Einbau von tri- und höherfunktionellen Aminen oder alkylenoxidmodifizierten Aminen, bzw. den Einbau tri- und höherfunktioneller Alkylierungsmittel auf z.B. Basis von Epoxiden oder Halogencarbonsäureestern in die Strukturen gemäß WO 02/10256, WO 02/10257, WO 02/10259 eine Verzweigung/Vernetzung erreicht, die das Eigenschaftsniveau, betreffend Substantivität, Weichheit und Hydrophilie weiter steigert.

Vernetzende Siloxansysteme sind ebenfalls bekannt. Gemäß DE 42 11 256 können Aminosiloxane in Emulsion mit Silanen oder Siloxanen vernetzt werden, die über mindestens eine Carbonsäureanhydridgruppe verfügen. Stabilität der Formulierung und besonders die Hydrophilie der Ausrüstung sind nicht hinreichend. Alternativ kann eine Vernetzung ebenfalls erreicht werden durch Reaktion mit Epichlorhydrin oder Diepoxiden (WO 01/27232).

Selbst vernetzende Emulsionen auf Basis von Aminosiloxanen mit terminalen Trialkoxysilylstrukturen, die Bestandteil des Polyorganosiloxangerüstes sind, werden in der US 4661577 und der US 2002 0028900 beschrieben. Diese Strukturen enthalten jedoch nur eine Polyorganosiloxanhauptkette und erlauben daher keine maßgeschneiderte Gestaltung und Abstimmung von Löslichkeit, Weichheit, Hydrophilie und Substatitivität. Das Molekulargewicht kann nicht unabhängig von der Größe der Polysiloxanhauptkette oder einer gegebenenfalls vorhandenen Polyetherseitenkette erhöht werden.

Vernetzungsfähige Siliconquats werden in DE 32 36 466 beschrieben. Die Umsetzung von OH-terminierten Siloxanen mit quaternäre Ammoniumstrukturen enthaltenden Alkoxysilanen liefert reaktive, kammartig substituierte Zwischenprodukte, die allein oder mit geeigneten Vernetzungsagenzien, wie Trialkoxysilanen, auf der Faseroberfläche zu waschbeständigen Schichten vernetzen sollen.

Keine der vorgeschlagenen Lösungen liefert eine Antwort auf die Frage, wie insbesondere die in den WO 02/10256, WO 02/10257, WO 02/10259 als permanente hydrophile Weichmacher für Textilien beschriebenen polyquaternären Polysiloxan-Verbindungen in ihrer Effizienz, insbesondere der Substantivität, weiter signifikant gesteigert werden können.

Es ist somit Aufgabe der Erfindung, hoch effiziente hydrophile Amino- und/oder Ammonium-Polysiloxanverbindungen, insbesondere polyquaternäre Polysiloxancopolymer-Verbindungen (Siliconquats), deren Herstellung und Verwendung als waschbeständige hydrophile Weichmacher zur textilen Erstausrüstung zu beschreiben, wobei die Siliconquats den Textilien nach entsprechender Applikation einen silicontypischen weichen Griff und eine ausgeprägte Hydrophilie verleihen und dieses Eigenschaftsbild auch nach Einwirkung von Detergenzienformulierungen während wiederholter Waschprozesse bei gegebenenfalls erhöhter Temperatur nicht verloren geht. Es ist eine weitere Aufgabe der Erfindung, die Verwendung dieser Siliconquats als separate Weichmacher nach bzw. als Weichmacher in auf nichtionogenen oder anionischen/nichtionogenen Tensiden beruhenden Formulierungen zur Wäsche von Fasern und Textilien, als Bügelhilfe, Mittel zur Verhinderung bzw. Rückgängigmachung von Textilverknitterungen, sowie Mittel zur Ausrüstung von Papier zu beschreiben.

Die Aufgabenstellunung ergibt sich auch für Polysiloxancopolymere, die in kosmetischen Formulierungen für die Haut- und Haarpflege verwendet und die auf diese Weise in ihrer Substantvität verbessert werden können.

Ebenso ist die Fixierung von oberflächenaktiven Polymeren der zuvor genannten Strukturen auf harten Oberflächen eine nicht zufriedenstellend gelöste Aufgabe, die auf diesem Weg eine Lösung erfährt.

Es wurde überraschend gefunden, daß die Einführung von reaktiven, in wässrigen Emulsionen bei 20 °C stabilen Gruppen in Amino- und/oder Ammonium-Polysiloxanverbindungen, wie z.B. polyquaternäre Polysiloxancopolymere, wie sie z.B. in der WO 02/10256, WO 02/10257, WO 02/10259, DE10036533, DE100 36 522, EP-A-282720, US 6240929, DE 33 40 708 und den noch nicht veröffentlichten Deutschen Patentanmeldungen DE 102 12 470.1, DE 102 51 525.5 und DE 102 51 526.3 beschrieben sind, völlig überraschend bei der Behandlung natürlicher oder synthetischer Fasern oder faserartiger Substrate (wie z.B. Papier) zu einer deutlich gesteigerten Permanenz der Hydrophilie bei unverändert weichem Griff der Fasern führt.

Die vorliegende Erfindung stellt somit bereit:

Amino- und/oder Ammonium-Polysiloxanverbindungen und Salze davon, wie in den Patentansprüchen 1 bis 10 definiert.

Amino- und/oder Ammonium-Polysiloxanverbindung und Salze davon im Sinne der Erfindung sind Verbindungen, die mindestens eine bevorzugt mindestens zwei, noch bevorzugter mehr als vier Amino- und oder Ammoniumgruppen und bevorzugt mindestens eine, noch bevorzugter mindestens zwei, noch bevorzugter mindestens vier Polyorganosiloxanblöcke je Molekül aufweisen. Bevorzugt enthalten die genannten Verbindungen mindestens eine, bevorzugt mindestens zwei und noch bevorzugter mindestens vier quaternäre Ammoniumgruppen je Molekül. Die Verbindungen bestehen bevorzugt aus wiederkehrenden Einheiten, die die genannten Amino- und/oder Ammoniumgruppen und die genannten Polyorganosiloxanblöcke enthalten.

Bei den genannten funktionellen Gruppen der Formel (I) und (VIIIa) handelt es sich um reaktive Gruppen, die beim Aktivieren, d.h. beim Einwirken von Temperaturen von zweckmäßig mindestens 40 °C, vorzugsweise 60 °C, bevorzugter 80 °C, noch bevorzugter mindestens etwa 100 °C beispielsweise unter Kondensation, Umlagerung, Additionsreaktion und/oder Reaktion mit einer Substrat-, bevorzugt einer Faseroberfläche oder unter Vernetzung bzw. Reaktion mit sich selbst reagieren.

Im Fall von Verbindungen mit den Gruppen der Formel (VIIIa) kann in einer bevorzugten Ausführungsform die Aktivierung der Reaktivgruppen unter Zusatz von Säuren oder Basen, wie nachfolgend beschrieben, herbeigeführt

werden.

Reaktive Gruppe bedeutet hier also Gruppen der Formel (I) oder (VIIIa) in den erfindungsgemäßen Molekülen, die in der Lage sind gegebenenfalls unter Aktivierung zusätzliche inter- oder intramolekulare Bindungen aufzubauen, die das Molekulargewicht erhöhen, wie z.B. eine Vernetzung, oder Bindungen mit dem Substrat herzustellen.

Es zeigt sich überraschend, dass die genannten funktionellen Gruppen in den erfindungsgemäßen Amino- und/oder Ammonium-Polysiloxanverbindungen und ihren Salzen in wässrigen Emulsionen bei 20 °C stabil sind, so dass hervorragend für die Anwendung der Amino- und/oder Ammonium-Polysiloxanverbindungen in wässrigen Emulsionen geeignet sind. So zeigen die erfindungsgemäß verwendeten reaktiven Gruppen in Wasser bei 20 °C eine Halbwertszeit von zweckmäßig mindestens etwa 20 Tagen, bevorzugt mindestens etwa 30 Tagen, bevorzugter mindestens etwa 40 Tage. Die Anwesenheit bzw. die Konzentration der reaktiven Gruppen in den wäßrigen Emulsionen, kann dabei in an sich bekannter Weise beispielsweise durch IR- und NMR-spektroskopische Methoden bestimmt werden. Die Reaktivität der funktionellen bzw. reaktiven Gruppen kann durch die Anwesenheit von Katalysatoren erhöht werden. Katalysatoren sind Säuren oder Basen. Die Anwendung der Katalysatoren zur Aktivierung erfolgt bevorzugt bei Anwesenheit der Reaktivgruppen der Formel (VIIIa) zweckmäßig unmittelbar vor der Anwendung, z.B. durch Zugabe der Katalysatoren zu einer wässrigen Mischung der erfindungsgemäßen Verbindungen, die die Fasersubstrate enthält. Geeignete Säuren schließen z.B. anorganische Säure, wie z.B. Salzsäure, Schwefelsäure, Phosphorsäure etc. oder organische C1-C22-Carbonsäuren ein. Geeignete Basen schließen z.B. anorganische Basen, wie Alkalimetallhydroxide, Erdalkalimetallhydroxide, Ammoniak, und organische Basen, wie z.B. C1-C22 Alkylamine ein.

Die Gruppe der Formel (I) wird im folgenden gelegentlich als Uretdion-Gruppe bezeichnet. Die Gruppe der Formel (VIIIa) wird im folgenden gelegentlich als Alkoxysilyl-Gruppe bezeichnet. Bevorzugt enthalten die erfindungsgemäßen Verbindungen nur reaktive Gruppen der Formel (I) oder nur reaktive Gruppen der Formel (VIIIa). Es können aber auch Verbindungen, die sowohl reaktive Gruppen der Formel (I) als auch reaktive Gruppen der Formel (VIIIa) enthalten, erfindungsgemäß umfasst sein. Die Auswahl der bevorzugten Gruppen erfolgt in Abhängigkeit der Anwendung. Bevorzugt enthalten die erfindungsgemäßen Verbindungen die reaktive Gruppe der Formel (I).

Bei den erfindungsgemäßen Verbindungen handelt es sich um solche Ver-bindungen, die mindestens drei Einheiten, bevorzugt mindestens 4, noch bevorzugter mindestens 6 Einheiten, ausgewählt aus den Einheiten Q und V aufweisen, worin Q mindestens eine zwei-, drei- und/oder vierwertige Amino- und/oder Ammonium-gruppe wie im Patentanspruch 1 definiert ist, die nicht über ein Carbonylkohlenstoffatom an V gebunden ist, und V mindestens eine organische Einheit wie im Patentanspruch 1 definiert ist, die über Kohlenstoff mit den Q-Einheiten verbunden ist, mit der Maßgabe, dass mindestens eine der Einheiten V einen Polyorganosiloxanrest enthält. Bevorzugt enthalten die erfindungsgemäßen Verbindungen mindestens zwei, bevorzugter mindestens vier, noch bevorzugter min-destens sechs Einheiten V, die einen Polyorganosiloxanrest enthalten. In einer weiteren bevor-zugten Ausführungsform enthalten die erfindungsgemäßen Verbindungen mindestens zwei, bevorzugt mindestes vier, noch bevorzugter mindestens sechs Einheiten Q.

Die Einheit Q wird ausgewählt aus der Gruppe, die besteht aus:

$-NR^1-$,

$-N^+R^1_2-$,

einem gesättigten oder ungesättigten, gegebenenfalls mit weiteren Substituenten substituierten diaminofunktionellen Heterocyclus der Formeln:

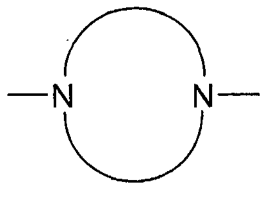

,

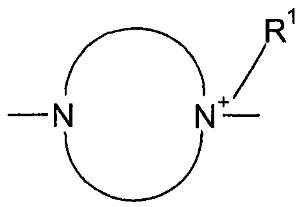

und

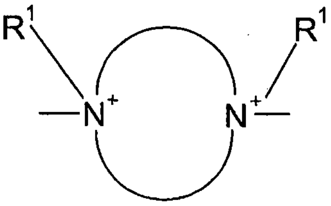

,

sowie
einem aromatischen, gegebenenfalls substituierten diaminofunktionellen Heterocyclus der Formel:

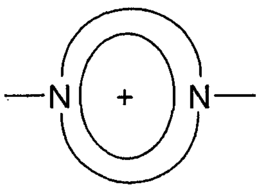

einem dreiwertigen Rest der Formel:

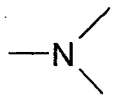

,

einem dreiwertigen Rest der Formel:

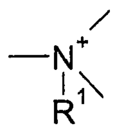

,

oder
einem vierwertigen Rest der Formel,

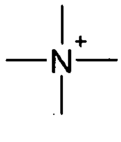

worin $R^1$ jeweils Wasserstoff oder einen einwertigen organischen Rest darstellt, welcher auch die Anwesenheit von Silizium zulässt und worin, wenn mehrere Gruppen $R^1$ vorliegen, diese gleich oder verschieden voneinander sein können. $R^1$ wird bevorzugt aus Resten $R^2$ ausgewählt.

Die Einheiten Q sind nicht mit Carbonylkohlenstoffatomen der V-Einheiten verbunden. Bevorzugt enthalten die erfindungsgemäßen Verbindungen als Q-Einheiten quaternäre Ammoniumgruppen. Quaternäre Ammoniumgruppen sind positiv geladenen Stickstoffatome, die mit vier Kohlenstoffatomen verbunden sind. Die Bildung der quaternären Ammoniumgruppen, die weiter unten noch genauer beschrieben wird, erfolgt zweckmäßig durch Alkylierung mit Hilfe von Epoxiden, Halogenalkyl-Verbindungen oder dergleichen. Die Alkylierung zur Bildung der quaternären Ammoniumgruppen in den erfindungsgemäßen Verbindungen erfolgt im allgemeinen unter Kettenverlängerung beim Aufbau oligomerer oder polymerer erfindungsgemäßer Verbindungen unter Verwendung polyfunktioneller Alkylierungsmittel. Sie kann jedoch auch nach erfolgtem Aufbau des oligomeren oder polymeren Gerüstes der erfindungsgemäßen Verbindungen unter Einsatz monofunktioneller Alkylierungsmittel erfolgen. Die erfindungsgemäßen Verbindungen weisen bevorzugt mindestens zwei, bevorzugter mindestens vier noch bevorzugter mindestens sechs quaternäre Ammoniumgruppen auf.

Die Einheit V wird ausgewählt aus mindestens einem mehrwertigen, geradkettigen, cyclischen oder verzweigten, gesättigten, ungesättigten oder aromatischen Kohlenwasserstoffrest mit bis zu 1000 Kohlenstoffatomen (wobei die Kohlenstoffatome des gegebenenfalls vorhandenen Polyorganosiloxanrestes nicht mitgezählt werden), der gegebenenfalls eine oder mehrere Gruppen, ausgewählt aus

- -O-,
- -C(O)-,
- -C(S)-,
- $-NR^2-$, worin $R^2$ Wasserstoff, einen einwertigen, geradkettigen, cyclischen oder verzweigten, gesättigten, ungesättigten oder aromatischen Kohlenwasserstoffrest mit bis zu 300 Kohlenstoffatomen darstellt, der eine oder mehrere Gruppen ausgewählt aus -O-, -NH-, -C(O)- und -C(S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Polyetherresten, Polyetheresterresten, Polyorganosiloxanylresten und $-Si(OR)_{3-a}(R')_a$, worin a, R und R' wie oben definiert sind, substituiert sein kann, wobei wenn mehrere Gruppen $-NR^2-$ vorliegen, diese gleich oder verschieden sein können, und mit der Maßgabe, dass die Gruppe - $NR^2$- an ein Carbonyl- und/oder Thiocarbonylkohlenstoffatom bindet,

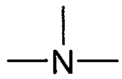

  und
- Polyorganosiloxanresten

enthalten kann, und der gegebenenfalls durch eine oder mehrere Hydroxylgruppen und/oder Gruppen der Formel (II)

$$-Si(OR)_{3-a}(R')_a \qquad (II)$$

worin a eine ganze Zahl von 0 bis 2 ist, und R und R' sind gleich oder verschieden voneinander und stellen jeweils einen organischen Rest dar, "a" ist 0, 1 oder 2, wobei 0 bevorzugt ist, R' wird bevorzugt aus der Gruppe ausgewählt, die aus $C_1$ bis $C_{22}$-Alkyl, Fluor-($C_3$-$C_{10}$)-alkyl und $C_6$-$C_{10}$-Aryl besteht, besonders bevorzugt ist R' Methyl, R wird bevorzugt aus der Gruppe ausgewählt, die aus $C_1$ bis $C_{22}$-Alkyl, $C_5$ bis $C_{10}$-Cycloalkyl, $C_7$ bis $C_{18}$-Alkylaryl, $C_7$ bis $C_{18}$-Arylalkyl und $C_6$-$C_{10}$-Aryl besteht, bevorzugt wird R ausgewählt aus sekundären oder tertiären Alkylgruppen oder sterisch anspruchsvollen Gruppen, wie Bis(tert.-butyl)phenyl, Cyclohexyl, besonders bevorzugt ist R gleich Isopropyl, sek.-Butyl, tert.-Butyl und sek.-Arnyl,

mit der Maßgabe, das mindestens ein Rest V mindestens einen Polyorganosiloxanrest enthält, und worin die terminale Absättigung der miteinander verbundenen mehrwertigen Gruppen Q und V über einwertige organische Reste erfolgt. Die Gruppe V enthält insbesondere die Einheiten die nachstehend definierten Einheiten U.

Die Nennung der Gruppe der Formel (II) sowohl als Substituent der Gruppe $R^2$ als auch der Gruppe V bedeutet, dass die genannte Gruppe sowohl Bestandteil einer terminalen Gruppe $R^2$ über Amidstickstoff mit der Hauptkette verbunden ist als auch über eine aliphatische Gruppe über ein Kohlenstoffatom mit der Hauptkette in den Gruppen V oder Q verbunden sein kann.

In den genannten Einheiten V ist der genannte Polyorganosiloxanrest zweckmäßig eine zweiwertige Gruppe der Formel (III):

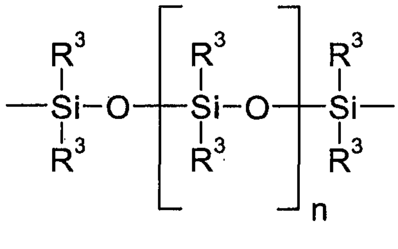

(III)

worin $R^3$ gleich oder verschieden sein kann und aus der Gruppe ausgewählt wird, die aus $C_1$ bis $C_{22}$ Alkyl, Fluor($C_3$-$C_{10}$)alkyl, $C_6$-$C_{10}$ Aryl und $-W-Si(OR)_{3-a}(R')_a$ besteht, worin R, R' und a wie oben definiert sind und W -O- oder einen zweiwertigen, geradkettigen, cyclischen oder verzweigten, gesättigten, ungesättigten oder aroma-tischen Kohlenwasserstoffrest mit bis zu 100 Kohlenstoffatomen darstellt, der eine oder mehrere Gruppen -C(O) -, -O-, -NH-, -S- enthalten kann, und gegebenenfalls durch Hydroxygruppen substituiert sein kann, und n = 0 bis 1000 bedeutet. Die erfindungsgemäßen Amino- und/oder Ammonium-Polysiloxan-verbindungen und Salze davon, enthalten zweckmäßig durchschnittlich mindestens einen, bevorzugt mindestens zwei, noch bevorzugter mehr als drei der genannten Einheiten V, die einen Polyorganosiloxanrest der Formel (III) enthalten.

Besonders bevorzugt enthalten die erfindungsgemäßen Verbindungen, die die funktionelle Gruppe der Formel (VIIIa), aber keine funktionelle Gruppe der Formel (I) aufweisen, mindestens drei Einheiten V, die einen Polyorganosiloxanrest der Formel (III) aufweisen. Diese Verbindungen weisen demzufolge mindestens zwei Gruppen Q auf, die zwischen den Einheiten V liegen.

Weisen die erfindungsgemäßen Verbindungen eine funktionelle Gruppe der Formel (I) auf, so enthalten sie mindestens eine, bevorzugt zwei, noch bevorzugter mindestens drei Einheiten V, die einen Polyorganosiloxanrest der Formel (III) aufweisen.

"n" in dem Polyorganosiloxanrest der Formel (III) ist bevorzugt 20 bis 200. Die Polyorganosiloxanreste in den Einheiten V können gleich oder verschieden von-einander sein.

Die erfindungsgemäßen Verbindungen enthalten bevorzugt mindestens eine, noch bevorzugter mindestens zwei Einheiten der Formel (IV):

-[Q-V]- (IV)

worin Q und V wie oben definiert sind, und die terminale Absättigung der Gruppen Q und V durch einwertige organische Gruppen erfolgt.

Bei den erfindungsgemäßen Amino- und/oder Ammonium-Polysiloxanverbindungen und Salzen davon, kann es sich beispielsweise um Polyamino- und/oder Polyammonium-Polysiloxan-Verbindungen handeln. Die Polyamino- und/oder Polyammonium-Polysiloxan-Verbindungen sind zweckmäßig Copolymer-Verbindungen, die in der Polymerhauptkette Amino- und/oder Ammonium-Wiederholungseinheiten und Polysiloxan-Wiederholungseinheiten aufweist. Die Amino-Einheiten enthalten zweckmäßig sekundäre und/oder tertiäre Stickstoffatome (2 oder 3 organische Reste am neutralen Stickstoffatom). Die Ammonium-Einheiten enthalten sekundäre, tertiäre und/oder quartäre positive geladene Stickstoffatome (2, 3, oder 4 organische Reste am Stickstoff). Als Amino- und/oder Ammonium-Wiederholungseinheiten können auch über zwei Stickstoffatome in die Polymerkette eingebundene heterocyclische Reste dienen.

Bei den erfindungsgemäßen Amino- und/oder Ammonium-Polysiloxanverbindungen und Salzen davon, kann es sich auch um Amino- und/oder Ammonium-Polysiloxan-Verbindungen handeln, die in den Seitengruppen einer Polyorganosiloxangruppe Amino- und/oder Ammoniumgruppen die bevorzugt an weitere Polyorganosiloxangruppen binden, enthalten. D.h., dass sich die Amino- und/oder Ammoniumgruppen nicht in der Hauptkette aus Polyorganosiloxan-Wiederholungseinheiten befinden.

Der Unterschied kann wie folgt veranschaulicht werden: Polyamino- und/oder Polyammonium-Polysiloxan-Verbindung mit $\alpha,\omega$-gebunden siloxanhaltigen Grupen V:

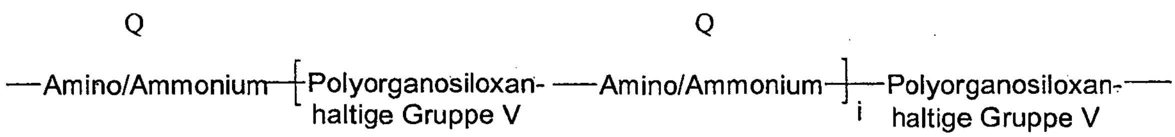


Amino- und/oder Ammonium-Polysiloxan-Verbindung:

Typ ( P1 )

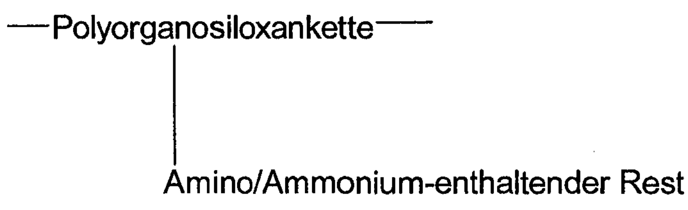


oder

Typ ( P2)

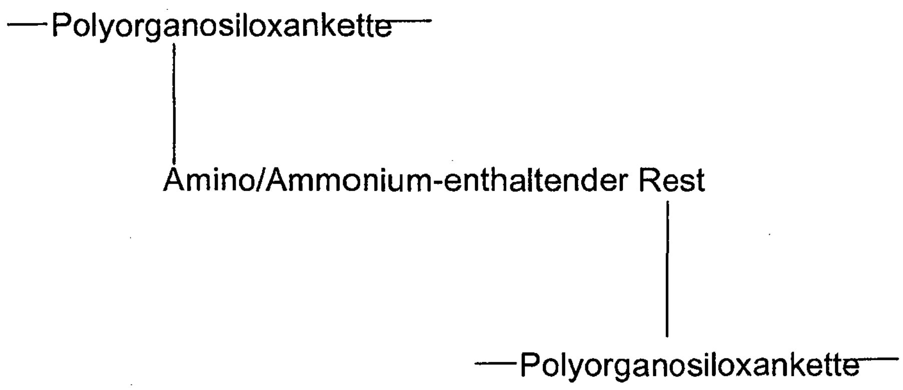


Typ (P3)

In den erfindungsgemäßen Polyamino- und/oder Polyammonium-Polysiloxan-Verbindung, die bevorzugt mindestens zwei Einheiten der Formel (IV):

-[Q-V]- (IV)

enthalten, worin Q und V wie oben definiert sind, und die terminale Absättigung der Gruppen Q und V durch einwertige organische Gruppen erfolgt, werden die Einheiten V beispielsweise aus der Gruppe ausgewählt, die aus $V^1$, $V^2$ und $V^3$ besteht. $V^2$ ist eine zweiwertige Gruppe V, die wie oben definiert ist, und $V^2$ enthält mindestens einen langkettigen Polysiloxanrest $-Z^2-$ der Formel (V)

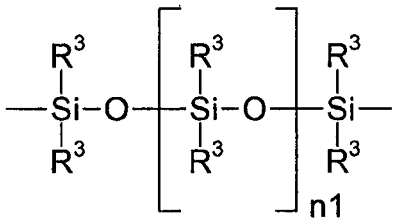

(V)

worin $R^3$ wie oben definiert ist und $n_1$ = 20 bis 1000 bedeutet.
$V^1$ ist eine zweiwertige Gruppe V, die wie oben definiert ist, die keinen langkettigen Polysiloxanrest $-Z^2-$ der Formel (V) enthält, und die gegebenenfalls einen kurzkettigen Polysiloxanrest $-Z^1-$ der Formel (VI) enthalten kann,

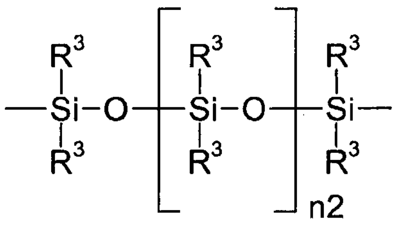

(VI)

worin $R^3$ wie oben definiert ist und $n_2$ = 0 bis 19 bedeutet.

$V^3$ ist ein drei- oder höherwertige Gruppe V, die wie oben definiert ist, und $V^3$ enthält mindestens eine drei- oder höherwertige Organopolysiloxaneinheit.

Die erfindungsgemäßen Polyamino- und/oder Polyammonium-Polysiloxan-Verbin-dung, die mindestens eine, bevorzugt mindestens zwei Einheiten der Formel (IV):

-[Q-V]- (IV)

enthalten, werden zweckmäßig durch monofunktionelle Gruppen $-Q-R^4$ und/oder $-V-R^4$ als Endgruppe terminiert, worin $R^4$ einen einwertigen organischen Rest, bevorzugt Methyl, Wasserstoff oder Hydroxyl darstellt.

Die Gruppe -V-$R^4$ kann in einer bevorzugten Ausführungsform auch eine Siloxangruppe darstellen, die nicht über eine $R^3{}_2$SiO-Gruppe mit $V^{2*}$ bzw. dem restlichen V verbunden ( Typ P1) ist, sondern über nur eine $R^3$SiO-Einheit,d.h. eine Seitengruppe, mit $V^{2*}$ bzw. V verbunden ist (Typ P2) und (P3)).

Dieses Copolymer weist demnach bevorzugt 2 Einheiten der Formel (IIIb) als Endgruppe auf, die die Wiederholungseinheiten V und Q begrenzen, wobei V und Q die genannten Zusammensetzungen aufweisen.

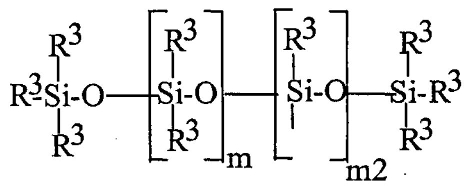

( IIIb) ,

worin $R^3$ m und $m_2$ wie oben definiert sind.

Die erfindungsgemäßen Polyamino- und/oder Polyammonium-Polysiloxane, die mindestens eine Einheit der Formel (IV) enthalten, sollen auch den Fall einschließen, in dem nur eine Einheit -[Q-V]- vorliegt, so dass auch Verbindungen der Formeln $R^4$-V-[Q-V]-$R^4$ bzw. $R^4$-[Q-V]-Q-$R^4$ umfasst sein können. Bevorzugt sind dann Polydialkylsiloxan terminierte Copolymere, in denen eine zweiwertige Einheit V zwischen zwei Q-Einheiten mindestens eine uretdionhaltige Einheit enthält.

Beispiele für derartige Polymere, aber ohne uretdionhaltige Struktureinheiten, finden sich in WO 02/10256.

Bevorzugt handelt es sich bei diesen Amino- und/oder Ammonium-Polysiloxan-Verbindungen mit 2 Einheiten der Formel (IIIb), die an unabhängige Reste V2* binden, um Strukturen vom Typ P1 und P2.

Bei den erfindungsgemäßen Polyamino- und/oder Polyammonium-Polysiloxan, die mindestens Einheit der Formel (IV) enthalten, handelt sich zum Beispiel um lineare Polysiloxancopolymere der allgemeinen Formel (IV'):

-[Q-V]- (IV')

worin Q wie oben definiert ist, V mindestens eine Gruppe $V^1$ und/oder mindestens eine Gruppe $V^2$ darstellt, worin $V^1$ und $V^2$ wie oben definiert sind.

In den allgemeinen Formeln (IV) bzw. (IV') kann das molare Verhältnis der Gruppen $V^1$ und $V^2$ in den Polysiloxan-Verbindungen $V^2/V^1$ an sich einen beliebigen Wert annehmen. Erfindungsgemäß ist somit auch der Fall eingeschlossen, bei dem die Polysiloxanverbindung der Formeln (IV) oder (IV') nur $V^2$-Einheiten enthält, die Polysiloxanverbindung also die Formel -[Q-$V^2$]- aufweist. Auch der Fall, bei dem die Polysiloxanverbindung nur $V^1$-Einheiten enthält, ist erfindungsgemäß umfasst. In diesem Fall müssen die $V^1$-Einheiten jedoch $Z^1$-Siloxaneinheiten enthalten.

In einer bevorzugten Ausführungsform der Erfindung enthält die Polysiloxanverbindung der Formeln (IV) oder (IV') jedoch sowohl $V^2$ als auch $V^1$-Einheiten.

Das molare Verhältnis der Gruppen $V^1$ und $V^2$ in den Polysiloxan-Verbindungen der allgemeinen Formeln (IV) bzw. (IV') kann je nach gewünschter Aufgabenstellung, wie Weichheit, Hydrophilie und Substantivität, der erfindungsgemäßen Verbindungen eingestellt werden. In einer bevorzugten Ausführungsform der Erfindung ist das Verhältnis der Gruppen $V^1$ zu $V^2$ ungefähr 1 : 1, wobei in einer besonders bevorzugten Ausführungsform $V^1$ kein Polysiloxan Z1 enthält und gleichzeitig zwei unterschiedliche V1-Reste, z.B. Hexamethylen und bis-alky-terminierter Polyether, anwesend sind, wie in Beispiel 3 dargestellt. Der Aufbau derartiger linearer Polyamino- bzw. Polytetraorgano-Ammonium-Verbindungen ist z.B. beschrieben worden in der WO 02/10257, WO 02/10259, EP 282720 oder US 5,981,681. Besonders bevorzugt sind die Polyamino- bzw. Polyammonium-Polysiloxan-Gerüste der WO 02/10259 und der WO 02/10257 auf deren in den Ansprüchen 1 definierte Polysiloxanpolymere hiermit ausdrücklich Bezug genommen wird, und die zum Offenbarungsgehalt der vorliegenden Anmeldung gehören. In einer weiteren Ausführungsform der linearen Polyamino- bzw. Polyammonium-Polysiloxanverbindungen der Formel (IV) bzw. (IV') ist $V^2/V^1$ ungleich 1, bevorzugt ist $V^2/V^1 < 1$, bevorzugter < 0,9, noch bevorzugter erfüllt $V^2/V^1$ die Beziehung

$$0{,}0005 < V^2/V^1 < 0{,}5.$$

Die Gruppe $R^4$ wird bevorzugt ausgewählt aus den Gruppen $R^2$.

In einer bevorzugten Ausführungsform der Erfindung wird der zweiwertige Rest Q in den Formeln (IV) oder

(IV') aus der Gruppe ausgewählt, die besteht aus:

einer quaternierten Imidazoleinheit der Struktur

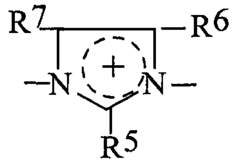

,

einer quaternierten Pyrazoleinheit der Struktur

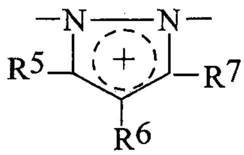

einer zweifach quaternierten Piperazineinheit der Struktur

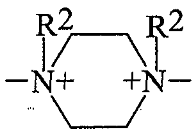

,

einer monoquaternierten Piperazineinheit der Struktur

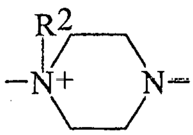

,

einer monoquaternierten Piperazineinheit der Struktur

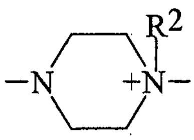

,

einer zweifach quaternierten Einheit der Struktur

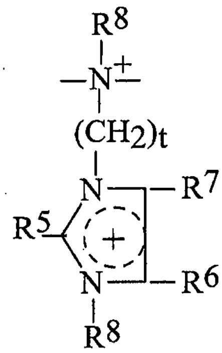

,

einer monoquaternierten Einheit der Struktur

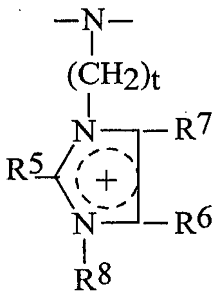

,

einer monoquaternierten Einheit der Struktur

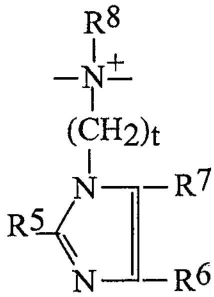

,

einer zweifach quaternierten Einheit der Struktur

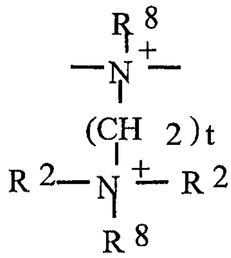

,

einer monoquaternierten Einheit der Struktur

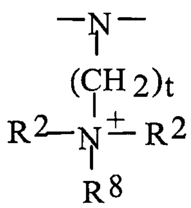

.

und einer monoquaternierten Einheit der Struktur

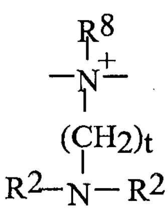

worin

t von 2 bis 10 ist,
$R^2$ wie oben definiert ist, und wenn mehrere Reste $R^2$ vorhanden sind, diese gleich oder verschieden sein können, und zwei Reste $R^2$ gemeinsam mit dem Stickstoffatom einen fünf- bis siebengliedrigen Heterocyclus bilden, der gegebenenfalls zusätzlich ein oder mehrere Stickstoff-, Sauerstoff- und/oder Schwefelatome aufweisen kann, $R^5$, $R^6$, $R^7$ gleich oder verschieden sein können und aus der Gruppe ausgewählt werden, die besteht aus: Wasserstoff, Halogen, Hydroxylgruppe, Nitrogruppe, Cyanogruppe, Thiolgruppe, Carboxylgruppe, Alkylgruppe, Monohydroxyalkylgruppe, Polyhydroxyalkylgruppe, Thioalkylgruppe, Cyanoalkylgruppe, Alkoxygruppe, Acylgruppe, Acetyloxygruppe, Cycloalkylgruppe, Arylgruppe, Alkylarylgruppe, und Gruppen des Typs -$NHR^W$, in denen $R^W$ H, Alkylgruppe, Monohydroxyalkylgruppe, Polyhydroxyalkylgruppe, Acetylgruppe, Ureidogruppe bedeuten, und jeweils zwei der benachbarten Reste $R^5$, $R^6$ und $R^7$ mit den sie an den Heterocyclus bindenden Kohlenstoffatomen aromatische Fünf- bis Siebenringe bilden können, und
$R^8$ die Bedeutung von $R^2$ aufweist, wobei $R^8$ und $R^2$ gleich oder verschieden sein können.

In einer bevorzugten Ausführungsform der Polysiloxanverbindungen der Formel (IV) bzw. (IV') als Komponente bl) stellt $V^2$ eine Gruppe der Formel

$$-V^{2*}-Z^2-V^{2*}-$$

dar, worin $Z^2$ wie oben definiert ist und $V^{2*}$ einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -CONH-,-$CONR^2$-, worin $R^2$ wie oben definiert ist, , -C(O)- und -C(S)- enthalten kann, und der Rest $V^{2*}$ gegebenenfalls durch eine oder mehrere Hydroxylgruppen substituiert sein kann.
In der vorstehend erwähnten Ausführungsform kann das erfindungsgemäße lineare Polysiloxancopolymer die folgenden Wiederholungseinheiten aufweisen:
$-[V^{2*}-Z^2-V^{2*}-Q]-$ bevorzugt zusammen mit $-[V^1-Q]-$.
Das molare Verhältnis der Wiederholungseinheiten $-[V^{2*}-Z^2-V^{2*}-Q]-$ zu $-[V^1-Q]-$ , also das Verhältnis $V^2/V^1$ kann, wie vorstehend erwähnt geeignet eingestellt werden, und z.B. 1 betragen, ist in einer der Ausführungsformen jedoch bevorzugt ungleich 1, bevorzugter < 0,5. Im letzteren Fall enthalten die genannten linearen Polyamino- bzw. Polyammonium-Polysiloxancopolymere -[Q-V]- zwingend Blöcke, die mehr als eine $-[V^1-Q]$-Einheit, miteinander verknüpft enthalten.
Wie weiter unten im Zusammenhang mit dem Verfahren zur Herstellung der vorstehend beschriebenen linearen Polysiloxancopolymere noch ausführlich erläutert wird, können die blockartigen Sequenzen, die mehr als eine $-[V^1-Q]$-Einheit miteinander verknüpft aufweisen, je nach Herstellweise regelmäßig mit den $V^2$-Q-Einheiten oder unregelmäßig mit den $V^2$-Q-Einheiten verbunden werden.
Dies meint folgendes:

Bei der regelmäßigen Verbindung, bei der beispielsweise ein der Gruppe $-Q-[V^1-Q]_x$-entsprechendes Präpolymer mit $V^2$ entsprechenden Monomer-Einheiten im molaren Verhältnis 1:1 umgesetzt wird, lassen sich die linearen Polysiloxancopolymere wie folgt darstellen:

$$-\{V^2-Q-[V^1-Q]_x-\}-.$$

x kann dabei 2 bis 2000 sein und ist der Mittelwert der Verteilung. Die durch die Formel $-\{V^2-Q-[V^1-Q]_x-\}-$ dargestellten linearen Polysiloxancopolymere sind dadurch gekennzeichnet, dass sie im wesentlichen keine miteinander verknüpften $-V^2$-Q-Einheiten aufweisen, oder mit anderen Worten, sind zwei $-V^2$-Q-Einheiten stets durch mindestens eine $-V^1$-Q-Einheit unterbrochen.
Bei der unregelmäßigen Verbindung, bei der beispielsweise Q-Einheiten entsprechende Monomere mit $V^1$ entsprechenden Monomer-Einheiten und $V^2$ entsprechenden Monomer-Einheiten im Verhältnis $Q/(V^1 + V^2)$, mit beispielsweise $V^2/V^1 < 1$, bevorzugt < 0,5, von 1:1 umgesetzt wird, lassen sich die linearen Polysiloxancopolymere wie folgt darstellen:

$$-Q-(V^1,V^2)-,$$

worin V das Verhältnis $V^2/V^1 < 1$ bzw. < 0,5 ist. Dabei sind die Gruppen $V^1$ und $V^2$ statistisch über die Copolymerkette verteilt. Im Unterschied zu dem durch die regelmäßige Verbindung hergestellten linearen Polysiloxancopolymere kann

dieses Copolymer auch benachbarte -Q-$V^2$-Einheiten aufweisen.

In einer bevorzugten Ausführungsform der erfindungsgemäß verwendeten Polysiloxanverbindung der Formel (IV) bzw. (IV') wird die Gruppe $V^1$ ausgewählt aus zweiwertigen, geradkettigen, cyclischen oder verzweigten, gesättigten, ungesättigten oder aromatischen Kohlenwasserstoffresten mit bis zu 400 Kohlenstoffatomen, die gegebenenfalls eine oder mehrere Gruppen, ausgewählt aus -O-, -CONH-, -CONR$^2$-, worin $R^2$ wie oben definiert ist, -C(O)-, -C(S)- und -$Z^1$-enthalten kann, worin -$Z^1$- eine Gruppe der Formel

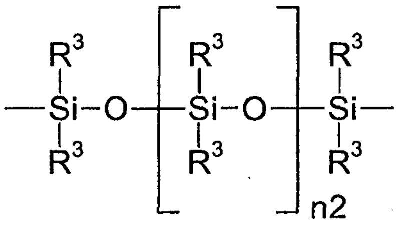

(VI)

ist, worin

$R^3$ bevorzugt $C_1$-$C_{18}$ Alkyl, das gegebenenfalls mit einem oder mehreren Fluoratomen substituiert sein kann, oder Phenyl ist, und $n_2$ wie oben definiert ist.

In einer weiteren bevorzugten Ausführungsform der Polysiloxan-Verbindungen der Formel (IV) bzw. (IV') wird die Gruppe Q ausgewählt aus:

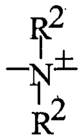

einer quaternierten Imidazoleinheit der Struktur

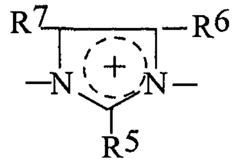

,

einer quaternierten Pyrazoleinheit der Struktur

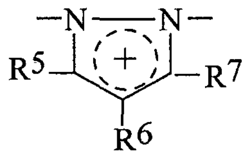

einer zweifach quaternierten Piperazineinheit der Struktur

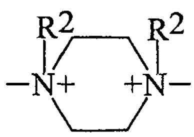

,

einer monoquaternierten Piperazineinheit der Struktur

22

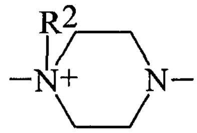

,

einer monoquaternierten Piperazineinheit der Struktur

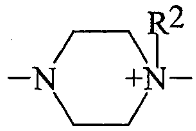

,

einer monoquaternierten Einheit der Struktur

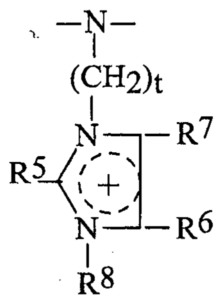

,

worin $R^2$, $R^4$, $R^5$, $R^6$, $R^7$ und $R^8$ wie oben definiert sind.

In einer weiteren bevorzugten Ausführungsform der linearen Polysiloxanverbindungen der Formel (IV') als der vorliegenden Erfindung erfüllt das molare Verhältnis $V^2/V^1$ die Beziehung

$$0{,}0005 < V^2/V^1 < 0{,}5, (= 2 < V^1/V^2 < 2000)$$

bevorzugter die Beziehung

$$0{,}005 < V^2/V^1 < 0{,}4, (= 2{,}5 < V^1/V^2 < 200)$$

noch bevorzugter die Beziehung

$$0{,}01 < V^2/V^1 < 0{,}3 (= 3{,}3 < V^1/V^2 < 100).$$

Bevorzugt sind in den Formeln (IV) und (IV'):
$R^3$ = $C_1$ bis $C_{18}$ Alkyl, insbesondere Methyl, Ethyl, Trifluorpropyl und Phenyl,

$n_1$ = 20 bis 400, besonders bevorzugt 20 bis 300, speziell 20 bis 200. In einer weiteren bevorzugten Ausführungsform ist $n_1$ zwischen 20 und 50 oder zwischen 80 und 200. Die Zahl $n_1$ ist die mittlere Polymerisationsgrad aus $M_n$ der Diorganosiloxy-Einheiten in der Gruppe $Z^2$.

$n_2$ = 0 bis 15, besonders bevorzugt 0 bis 10, speziell 0 bis 5, spezieller 0. Die Zahl $n_2$ ist die mittlere Polymerisationsgrad aus $M_n$ der Diorganosiloxy-Einheiten in der Gruppe $Z^1$.

$V^{2*}$ = ein zweiwertiger geradkettiger, cyclischer oder verzweigter, gesättigter, ungesättigter $C_3$ bis $C_{16}$ Kohlenwasserstoffrest oder aromatischer $C_8$ bis $C_{20}$ Kohlenwasserstoffrest, der gegebenenfalls eine oder mehrere Gruppen, ausgewählt aus -O-,-CONH-, -CONR$^2$-, -C(O)-, -C(S)- enthalten kann und durch eine oder mehrere OH-Gruppe substi-

tuiert sein kann, worin $R^2$ wie oben definiert ist.

Q =

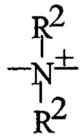

eine quaternierte Imidazoleinheit der Struktur

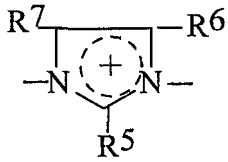

,

eine zweifach quaternierte Piperazineinheit der Struktur

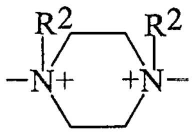

,

eine monoquaternierte Piperazineinheit der Struktur

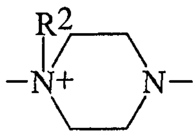

,

eine monoquaternierte Piperazineinheit der Struktur

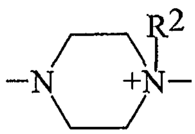

,

eine monoquaternierte Einheit der Struktur

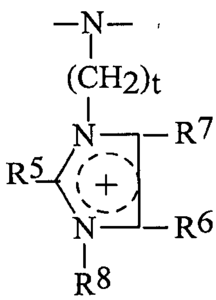

,

ist, worin $R^2$, $R^4$, $R^5$, $R^6$, $R^7$ und $R^8$ wie oben definiert sind.

Besonders bevorzugt steht

$V^{2*}$ für einen zweiwertigen geradkettigen, cyclischen oder verzweigten, gesättigten, ungesättigten oder aromatischen Kohlenwasserstoffrest mit bis zu 16 Kohlenstoffatomen, der eine oder mehrere Gruppen, ausgewählt aus -O-,-CONH-,

$-CONR^2-$, worin $R^2$ wie oben definiert ist, -C(O)-, -C(S)- enthalten kann und mit einer oder mehreren Hydroxylgruppen substituiert sein kann. Noch bevorzugter wird $-V^{2*}-$ ausgewählt aus Gruppen der Formeln:

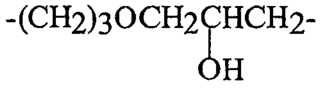

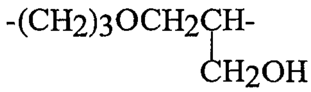

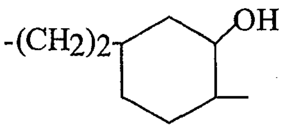

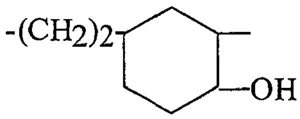

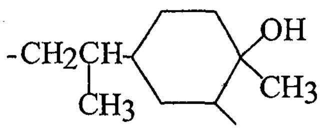

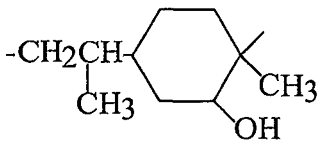

$-(CH_2)_2-$, $-(CH_2)_3-$ , $-(CH_2)_4-$, $-(CH_2)_5-$, $-(CH_2)_6-$,

$-CH=CHCH_2-$, $-CH=CHCH_2CH_2-$,

$-CH_2CH_2CH_2OC(O)CH_2-$, $-CH_2CH_2CH_2OC(O)CH_2CH_2-$,

$-CH=CHCH_2OC(O)CH_2-$, $-CH=CHCH_2OC(O)CH_2CH_2-$

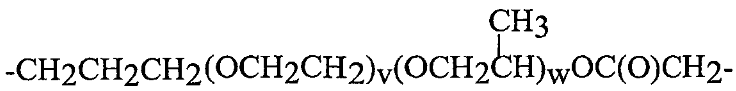

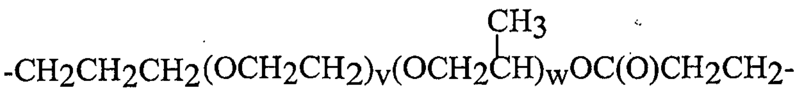

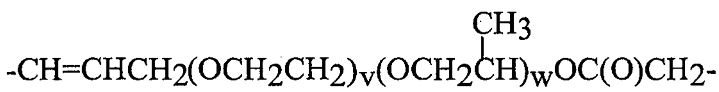

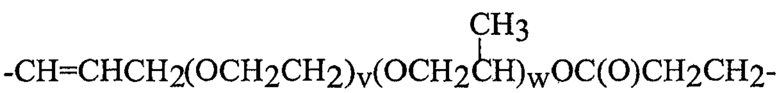

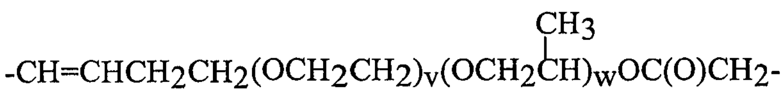

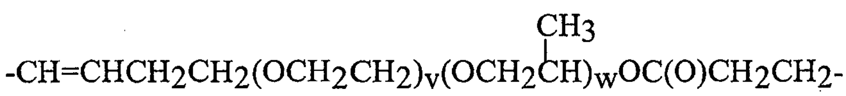

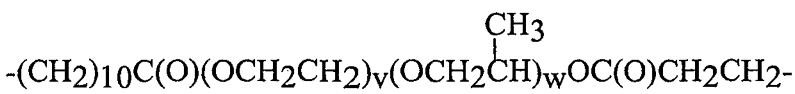

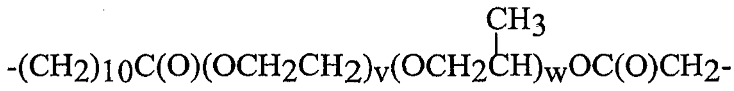

mit v+w ≥ 0,

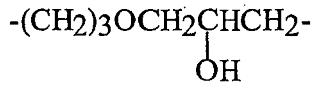 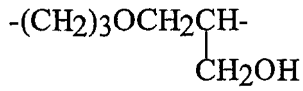

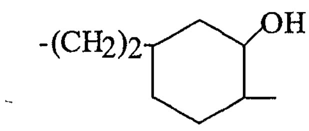 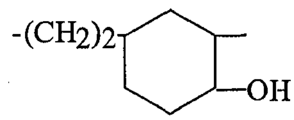

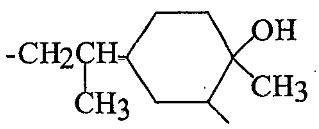 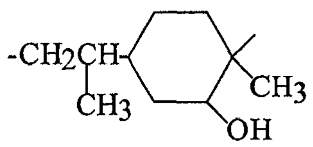

$-(CH_2)_3-$, $-(CH_2)_4-$, $-(CH_2)_5-$, $-(CH_2)_6-$,

$-CH{=}CHCH_2-$, $-CH{=}CHCH_2CH_2-$,

$-CH_2CH_2CH_2OC(O)CH_2-$, $-\ CH_2CH_2CH_2OC(O)CH_2CH_2-$,

$R^2$ steht bevorzugt für: H,

$-CH_3$, $-CH_2CH_3$, $-(CH_2)_2CH_3$, $-(CH_2)_3CH_3$, $-(CH_2)_5CH_3$, $-CH_2CH_2OH$,

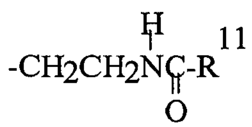 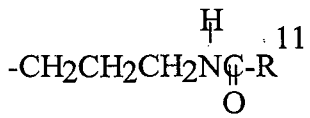

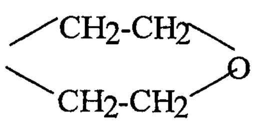 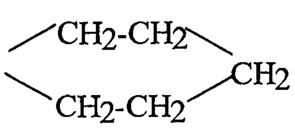

mit

$R^{11}$ = geradkettiger, cyclischer oder verzweigter $C_1$ bis $C_{18}$ Kohlenwasserstoffrest, der durch eine oder mehrere Gruppen, ausgewählt aus -O-, -NH-, -C(O)-, und -C(S)-enthalten kann und durch eine oder mehrere OH-Gruppen substituiert sein kann, speziell unsubstituierte $C_5$ bis $C_{17}$-Kohlenwasserstoffreste, die sich von den entsprechenden Fettäuren ableiten oder aber hydroxylierte $C_3$ bis $C_{17}$ Reste, die auf hydroxylierte Carbonsäuren, speziell Saccharidcarbonsäuren zurückgeführt werden können und ganz speziell

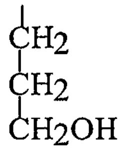
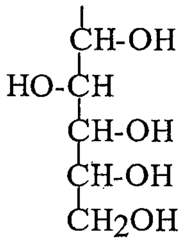
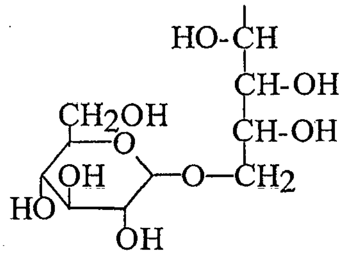

bedeuten.

Weiterhin steht $R^2$ bevorzugt für:

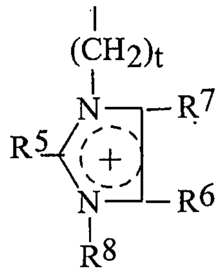

,

worin t, $R^5$ bis $R^8$ wie oben definiert sind,

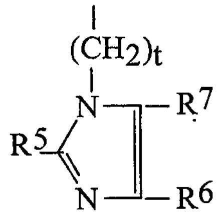

,

worin t, $R^5$ bis $R^7$ wie oben definiert sind,

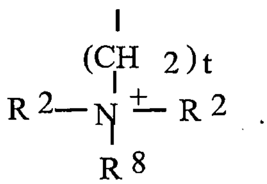

,

worin t, $R^2$ und $R^8$ wie oben definiert sind.

$V^1$ steht bevorzugt für

- $-R^9-$, worin $R^9$ einen zweiwertigen, gesättigten oder einfach oder mehrfach ungesättigten, geradkettigen oder verzweigten Kohlenwasserstoffrest mit zwei bis 25 Kohlenstoffatomen darstellt,
- $-(CH_2)_uC(O)O-[(CH_2CH_2O)_q-(CH_2CH(CH_3)O)_r]-C(O)(CH_2)_u-$
- $-(CH_2)_uC(O)O-R^9-O-C(O)(CH_2)_u-$, worin $R^9$ wie zuvor definiert ist,
- $-(CH_2)_u-R^{10}-(CH_2)_u-$, worin $R^{10}$ eine aromatische Gruppe ist,
- $-[CH_2CH_2O]_q-[CH_2CH(CH_3)O]_r-CH_2CH_2-$,
- $-CH(CH_3)CH_2O[CH_2CH_2O]_q-[CH_2CH(CH_3)O]_r-CH_2CH(CH_3)-$
- $-CH_2CH(OH)CH_2-$,
- $-CH_2CH(OH)(CH_2)_2CH(OH)CH_2-$,
- $-CH_2CH(OH)CH_2OCH_2CH(OH)CH_2OCH_2CH(OH)CH_2-$ und

- $-CH_2CH(OH)CH_2O-[CH_2CH_2O]_q-[CH_2CH(CH_3)O]_r-CH_2CH(OH)CH_2-$

worin
u von 1 bis 3 ist,
q und r von 0 bis 200, bevorzugt von 0 bis 100, bevorzugter von 0 bis 70 und besonders bevorzugt 0 bis 40 ist, und q+r>0 ist.

Bevorzugte Varianten von $V^1$ sind Strukturen der Formel:

$-CH_2C(O)O-[CH_2CH_2O]_q-[CH_2CH(CH_3)O]_r-C(O)CH_2-$,

$-CH_2CH_2C(O)O-[CH_2CH_2O]_q-[CH_2CH(CH_3)O]_r-C(O)CH_2CH_2-$,

$-CH_2CH_2CH_2C(O)O-[CH_2CH_2O]_q-[CH_2CH(CH_3)O]_r-C(O)CH_2CH_2CH_2-$,

veresterte Alkylen-, Alkenylen, Alkinyleneinheiten, speziell der Strukturen

$-CH_2C(O)O-[CH_2]_o-OC(O)CH_2-$,

$-CH_2CH_2C(O)O-[CH_2]_o-OC(O)CH_2CH_2-$,

$-CH_2CH_2CH_2C(O)O-[CH_2]_o-OC(O)CH_2CH_2CH_2-$

$-CH_2C(O)O-CH_2C{\equiv}CCH_2-OC(O)CH_2-$,

$-CH_2CH_2C(O)O-CH_2C{\equiv}CCH_2-OC(O)CH_2CH_2-$,

$-CH_2CH_2CH_2C(O)O-CH_2C{\equiv}CCH_2-OC(O)CH_2CH_2CH_2-$,

$-CH_2C(O)O-CH_2CH=CHCH_2-OC(O)CH_2-$,

$-CH_2CH_2C(O)O-CH_2CH=CHCH_2-OC(O)CH_2CH_2-$,

$-CH_2CH_2CH_2C(O)O-CH_2CH=CHCH_2-OC(O)CH_2CH_2CH_2-$,

Alkylen-, Alkenylen-, Alkinylen- und Aryleinheiten, speziell der Strukturen:

$-[CH_2]_o-$

mit o = 2 bis 6,

$-CH_2C{\equiv}CCH_2-$, $-CH_2CH=CHCH_2-$, $-CH(CH_3)CH_2CH_2-$,

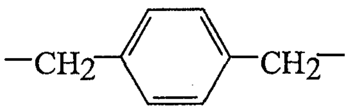

Polyalkylenoxideinheiten, speziell der Strukturen

$-[CH_2CH_2O]_q-[CH_2CH(CH_3)O]_r-CH_2CH_2-$,

$-CH(CH_3)CH_2O[CH_2CH_2O]_q-[CH_2CH(CH_3)O]_r-CH_2CH(CH_3)-$

mit
mono-, di- oder polyhydroxyfunktionelle Einheiten, speziell der Strukturen

$-CH_2CH(OH)CH_2-$, $-CH_2CH(OH)(CH_2)_2CH(OH)CH_2-$,

$-CH_2CH(OH)CH_2OCH_2CH(OH)CH_2OCH_2CH(OH)CH_2-$,

$-CH_2CH(OH)CH_2-[CH_2CH_2O]_q-[CH_2CH(CH_3)O]_r-CH_2CH(OH)CH_2-$

mit
q = 0 bis 200,
r = 0 bis 200

Bevorzugt sind q = 1 bis 50, insbesondere 2 bis 50, speziell 1 bis 20, ganz speziell 1 bis 10, sowie 1 oder 2, r = 0 bis 100, insbesondere 0 bis 50, speziell 0 bis 20, ganz speziell 0 bis 10, sowie 0 oder 1 oder 2.

Die Verzweigungseinheit $V^3$ kann silikonfrei sein. Beispiele hiervon schließen ein:

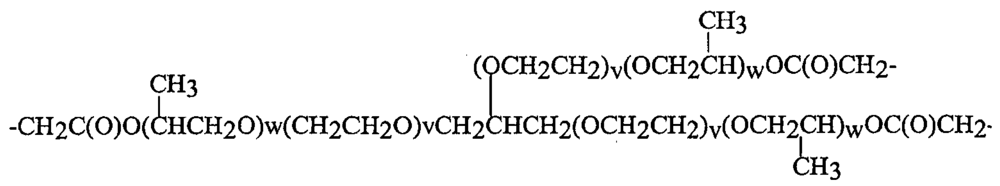

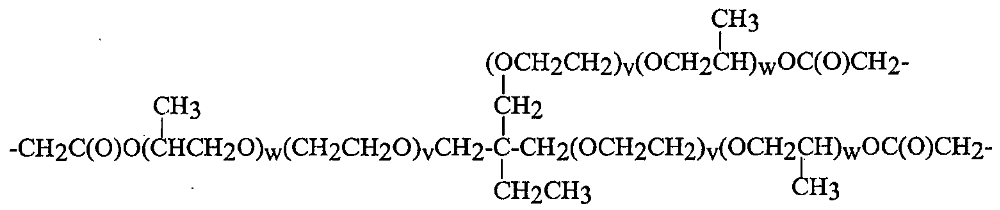

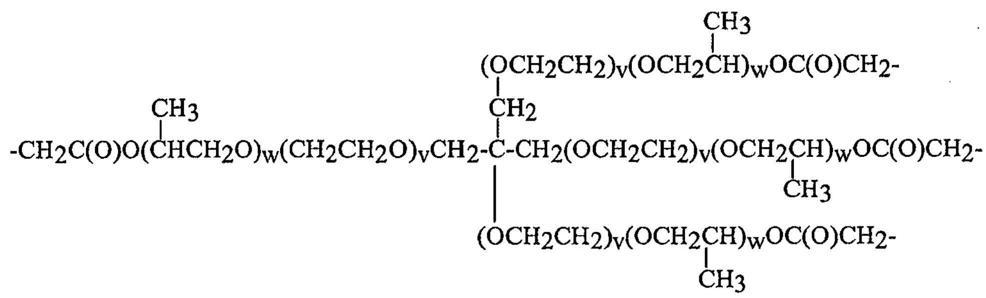

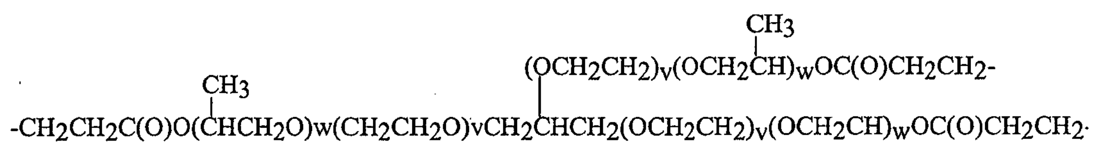

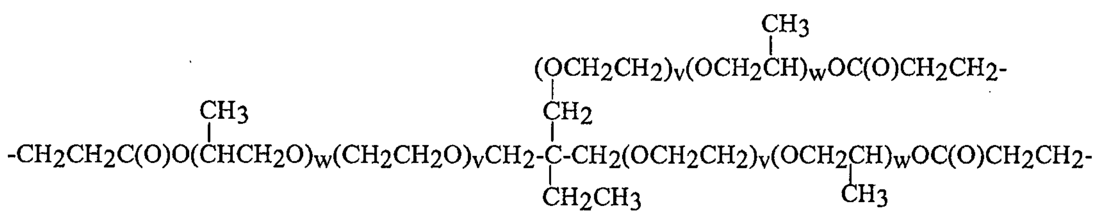

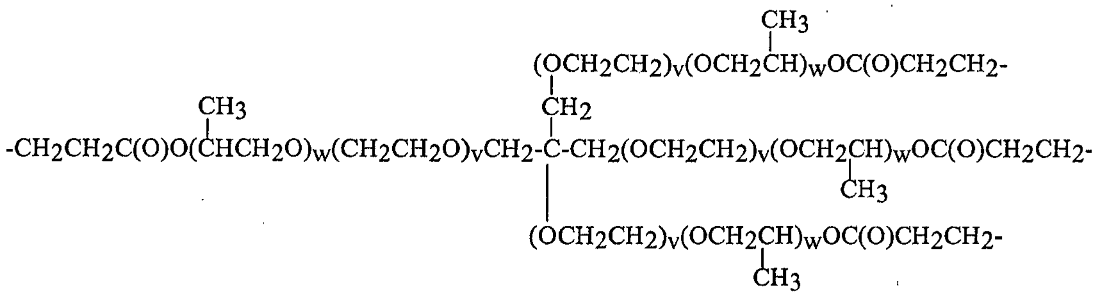

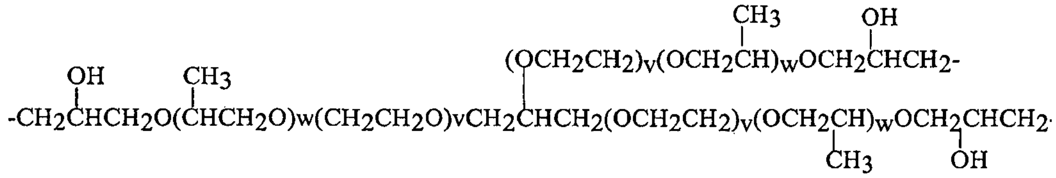

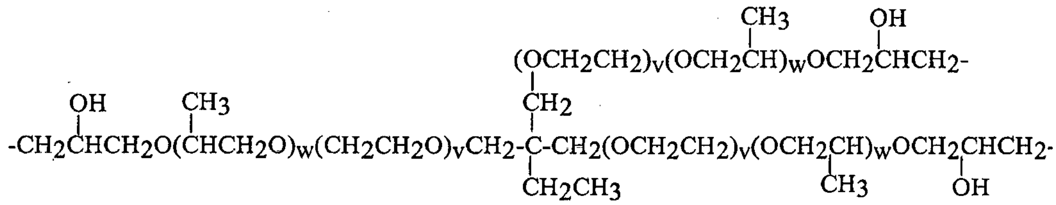

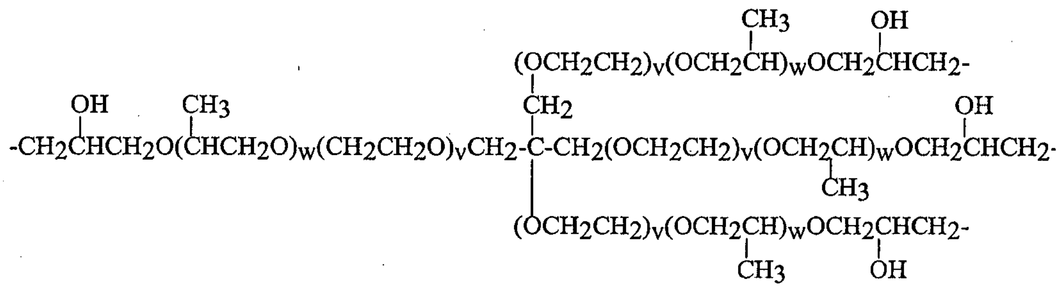

mit v+w ≥ 0.

Die Verzweigungseinheit $V^3$ kann eine drei- oder höherwertige Organopolysiloxan-einheit enthalten, wie zum Beispiel:

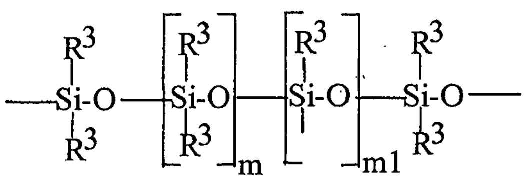

(VIIa)

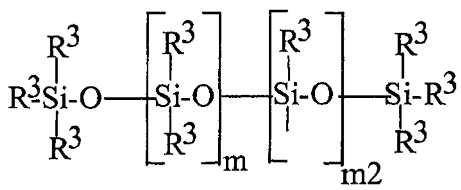

(VII b)

worin $R^3$ wie oben definiert ist, m = 0 bis 1000, und $m^1 \geq 1$ und $m^2 \geq 3$ ist,

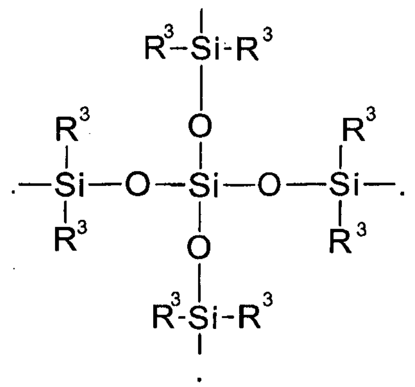

(VIIc)

und

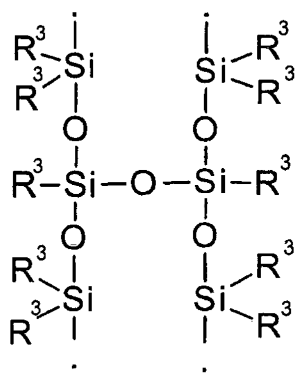

(VIId)

worin $R^3$ jeweils wie oben definiert ist.

Ein Beispiel einer $Z^3$-enthaltenden Verzweigungseinheit $V^3$ ist zum Beispiel:

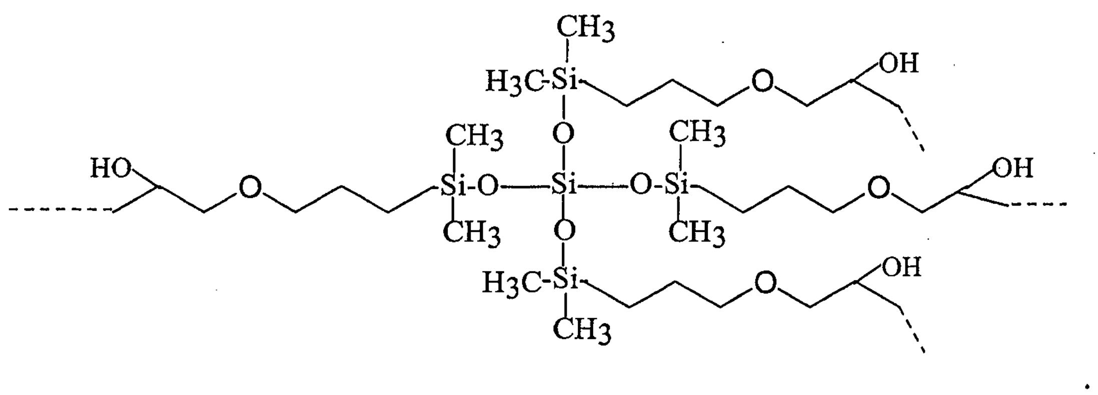

(VIIe)

Die erfindungsgemäßen Amino- und/oder Ammonium-Polysiloxanverbindung lassen sich zweckmäßig durch ein Verfahren herstellen, worin

a) mindestens eine Aminverbindung, ausgewählt aus einer Diamin-Verbindung und/oder einer primären oder sekundären Monoaminverbindung, mit einer zur Reaktion mit den Aminofunktionen der Amin-Verbindung befähigten multi-, bevorzugt difunktionellen organischen Verbindungen umgesetzt werden, wobei das molare Verhältnis der Aminofunktionen der genannten Aminverbindung zu den funktionellen Gruppen der genannten multi-, bevorzugt difunktionellen organischen Verbindung von etwa 0,5 bis 2 beträgt, oder

b) mindestens zwei Mol einer Aminverbindung (1), ausgewählt aus einer Diamin-Verbindung (1) und/oder einer

primären oder sekundären Monoaminverbindung (1), mit einem Mol einer multi-, bevorzugt difunktionellen, zur Reaktion mit den Aminofunktionen der Aminverbindung befähigten organischen Verbindung (1), unter Bildung einer Diaminverbindung (2) (Monomer) umgesetzt werden, und die Diamin-verbindung (2) (Monomer) anschließend mit mindestens einer weiteren multi-, bevorzugt difunktionellen, zur Reaktion mit den Amino-funktionen der Diaminverbindung (2) befähigten organischen Verbindung (2), gegebenenfalls in Anwesenheit weiterer Aminverbindungen (2) umgesetzt wird, wobei die Stöchiometrie der Amino-funktionen und der zur Reaktion mit Aminofunktionen befähigten funktionellen Gruppen in der letzten Stufe der Umsetzung etwa 1:1 beträgt, und die organischen Verbindungen (1) und (2) gleich oder verschieden von-einander sein können, oder

c) eine Aminverbindung, ausgewählt aus einer Diamin-Verbindung (1) und /oder einer primären oder sekundären Monoaminverbindung, mit einer multi-, bevorzugt difunktionellen, zur Reaktion mit den Aminofunktionen der Aminverbindungen befähigten organischen Verbindung (1) unter Bildung einer Diaminverbindung (2) (aminoterminiertes Oligomer) umgesetzt wird, wobei das molare Verhältnis der Aminofunktionen der genannten Aminverbindung zu den funktionellen Gruppen der genannten multi-, bevorzugt difunktionellen organischen Verbindung (1) etwa 1 bis 2 beträgt, anschließend die erhaltene Diaminverbindung (2) (aminoterminiertes Oligomer) mit mindestens einer multi-, bevorzugt difunktionellen zur Reaktion mit den Aminofunktionen der Diamin-Verbindungen befähigten organischen Verbindung (2) umgesetzt wird, wobei die Stöchiometrie derAminofunktionen und der zur Reaktion mit Aminofunktionen befähigten funktionellen Gruppen in der letzten Stufe der Umsetzung etwa 1:1 beträgt, und die organischen Verbindungen (1) und (2) gleich oder verschieden sein können, oder

d) eine Aminverbindung (1), ausgewählt aus einer Diamin-Verbindung und /oder einer primären oder sekundären Monoaminverbindung, mit einer multi-, bevorzugt difunktionellen, zur Reaktion mit den Aminofunktionen der Aminverbindung befähigten organischen Verbindung (1) unter Bildung einer multi-, bevorzugt difunktionellen, zur Reaktion mit Aminofunktionen befähigten organischen Verbindung (2) (difunktionelles Oligomer) umgesetzt wird, wobei das molare Verhältnis der Aminofunktionen der genannten Aminverbindung zu den funktionellen Gruppen der genannten multi-, bevorzugt difunktionellen organischen Verbindung (1) etwa 0,5 bis 1 beträgt, anschließend die organische Verbindung (2) (difunktionelles Oligomer) mit mindestens einer Aminverbindung (2), ausgewählt aus einer Diamin-Verbindung und/oder einer primären oder sekundären Monoamin-verbindung, gegebenenfalls in Anwesenheit einer oder mehrerer multi-, bevorzugt di-funktionellen, zur Reaktion mit Aminofunktionen befähigten organischen Verbindung (3) umgesetzt wird, wobei die Stöchiometrie der Aminofunktionen und der zur Reaktion mit Aminofunktionen befähigten funktionellen Gruppen in der letzten Stufe der Umsetzung etwa 1:1 beträgt,

wobei gegebenenfalls monofunktionelle, bevorzugt tertiäre Monoamine oder geeignete, zur Kettenfortpflanzung nicht befähigte Monoamine und/oder monofunktionelle, zur Reaktion mit Aminofunktionen befähigte Verbindungen als Kettenabbruchsmittel hinzugesetzt werden können, und wobei gegebenenfalls vorhandene Aminofunktionen in den erhaltenen Produkten anschließend protoniert oder quaterniert werden können.

Bezogen auf die Verbindungen der Formel (IV) mit den Einheiten Q und V lassen sich die genannten Verfahrensvarianten wie beispielsweise wie folgt veranschaulichen:

$$2\text{-[N-N]-} + \text{-[V}^1\text{]-} + \text{-[V}^2\text{]-} \rightarrow 2\text{-[Q-V]- oder}$$

$$2\text{-[N]-} + \text{-[V}^1\text{]-} + \text{-[V}^2\text{]-} \rightarrow 2\text{-[Q-V]-}$$

wobei -[N-N]- ein Diamin ist, das auch ein $V^1$-enthaltendes Diamin -[N-$V^1$-N]- oder ein $V^2$-enthaltendes Diamin -[N-$V^{2*}$-$Z^2$-$V^{2*}$-N]- einschließen kann, und -[$V^1$]- und - [$V^2$]- den Wiederholungseinheiten $V^1$ und $V^2$ entsprechende Monomere darstellen sollen,
und -[N]- ein primäres oder sekundäres zur Kettenfortpflanzung geeignetes Monoamin darstellt.

Aus den -[N-N]- und/oder -[N]-Einheiten wird dabei mindestens eine quaternäre Ammonium-Einheit Q gebildet, wobei die Quaternierung je nach Art der Verbindung zwischen -[N-N]- bzw. -[N]-Einheiten und -[V]-Einheiten gegebenenfalls auch nach der Polymerisation in einem separaten Schritt erfolgen kann.

Bevorzugte Beispiele von -[N-N]- sind wie unten noch ausführlicher beschrieben Piperazin, Imidazol, bevorzugte Diamin-Einheiten -[N-$V^1$-N]- schließen beispielsweise ein: Polymethylendiamine, wie Hexamethylendiamin, $\alpha,\omega$-diaminoterminierte Polyether, wie z.B. Jeffamine, etc.

Bevorzugte Diamin-Einheiten -[N-$V^{2*}$-$Z^2$-$V^{2*}$-N]- schließen beispielsweise Umsetzungprodukte von $\alpha,\omega$-Dihydrogenpolydialkylsiloxane mit Allylaminen ein. Bevorzugte Beispiele von -[N]- sind wie unten noch ausführlicher beschrieben z.B. Dimethylamin.

Die Verwendung von Diaminen -[N-N]- ist an sich bevorzugt.

Bevorzugte -[$V^1$]-Monomere schließen beispielweise Epichlorhydrin, Bisepoxide, Biscarbonsäurechloride, Diisocyanate oder Bisacrylate. Es können bevorzugt auch Mischungen der genannten -[$V^1$]-Monomere, wie z.B. Mischungen aus Epichlorhydrin, Bis-Chloralkylester oder Bisepoxiden umgesetzt werden.

Bevorzugte -[$V^2$]-Monomere sind Monomere der Formel -[$V^{2*}$-$Z^2$-$V^{2*}$]-, worin $Z^2$ wie oben definiert ist, und -[$V^{2*}$] funktionalisierte der Wiederholungseinheit $V^{2*}$ entsprechende Gruppe darstellt. Bevorzugte -[$V^2$]-Monomere zur Bildung der $V^2$-Wiederholungseinheiten sind insbesondere $\alpha,\omega$-diepoxyterminierte Polydialkylsiloxane.

Eine weitere Variante, die sich sowohl mit Diaminen, -[N-N]-, als auch geeigneten Monoaminen -[N]- durchführen lässt, ist wie folgt definiert:

Schritt 1): 2 -[N-N]- + -[$V^2$]- oder-[$V^1$]- $\rightarrow$ -[N-N-$V^1$-N-N]- oder -[N-N-$V^2$-N-N]-
Schritt 2.1): -[N-N-$V^2$-N-N]- + -[$V^1$]- + -[N-N]- $\rightarrow$Q$V^2$Q$V^1$Q bzw. QVQ$V^2$QVQ$V^1$QVQ,
Schritt 2.2): -[N-N-$V^1$-N-N]- + -[$V^2$]- + -[N-N]- $\rightarrow$ Q$V^1$Q$V^2$Q bzw. QVQ$V^1$QVQ$V^2$QVQ

Bezüglich der bevorzugt verwendeten Monomer-Einheiten -[N-N]-, -[$V^1$]- und -[$V^2$]-gilt das oben Gesagte.

Eine weitere Variante ist wie folgt:

Schritt 1): 2 -[N]- + -[$V^2$]- oder-[$V^1$]- $\rightarrow$ -[N-$V^1$-N]- oder -[N-$V^2$-N]-
Schritt 2.1): -[N-$V^2$-N]- +-[$V^1$]- +-[N]- $\rightarrow$ Q$V^2$Q$V^1$,
Schritt 2.2): -[N-$V^1$-N]- + -[$V^2$]- +-[N]- $\rightarrow$ Q$V^1$Q$V^2$Q,

wobei diese Variante wie oben erwähnt nur mit primären oder sekundären Monoaminen durchführbar ist und wobei bezüglich der bevorzugt verwendeten Monomer-Einheiten -[N]-, -[$V^1$]- und -[$V^2$]- das oben Gesagte gilt.

Eine weitere Variante lässt sich schematisch beispielsweise wie folgt darstellen:

Schritt 1): -[N-N]-+ -[$V^1$]- $\rightarrow$ -[N-N-($V^1$-N-N)$_x$]-
Schritt 2): -[N-N-($V^1$-N-N)$_x$]- + -[$V^2$]- $\rightarrow$

wobei bezüglich der bevorzugt verwendeten Monomer-Einheiten -[N-N]-, -[$V^1$]- und -[$V^2$]- das oben Gesagte gilt.

Eine weitere Variante lässt sich schematisch beispielsweise wie folgt darstellen:

Schritt 1): -[N]- + -[$V^1$]- $\rightarrow$ -[N-($V^1$-N)$_x$]-
Schritt 2): -[N-($V^1$-N)$_x$]- + -[$V^2$]- $\rightarrow$

wobei bezüglich der bevorzugt verwendeten Monomer-Einheiten -[N]-, -[$V^1$]- und - [$V^2$]- das oben Gesagte gilt.

Eine weitere Variante lässt sich schematisch beispielsweise wie folgt darstellen:

Schritt 1): x+1 -[$V^1$]- + x -[N-N]- $\rightarrow$ -[$V^1$-(N-N-$V^1$)$_x$]-
Schritt 2): -[$V^1$-(N-N-$V^1$)$_x$]- +-[$V^2$]- $\rightarrow$

wobei bezüglich der bevorzugt verwendeten Monomer-Einheiten -[N-N]-, -[$V^1$]- und -[$V^2$]- das oben Gesagte gilt.

Eine weitere Variante lässt sich schematisch beispielsweise wie folgt darstellen:

Schritt 1): x+1 -[$V^1$]- + x-[N]- $\rightarrow$ -[$V^1$-(N-$V^1$)$_x$]-
Schritt 2): -[$V^1$-(N-$V^1$)$_x$]- + -[$V^2$]- $\rightarrow$

wobei bezüglich der bevorzugt verwendeten Monomer-Einheiten -[N]-, -[$V^1$]- und - [$V^2$]- das oben Gesagte gilt.

Für alle oben schematisch dargestellten Varianten gilt, dass auch Mischungen von Monoaminen -[N]- und Diaminen -[N-N]- eingesetzt werden können.

Besonders bevorzugt werden die funktionellen Gruppen der difunktionellen, zur Reaktion mit Aminofunktionen befähigten Verbindungen ausgewählt aus der Gruppe die besteht aus Epoxygruppen und Halogenalkylgruppen.

Als Ausgangspunkt für die Synthesen der erfindungsgemäßen linearen Polysiloxancopolymere sind $\alpha,\omega$ Si-H funktionalisierte Siloxane der allgemeinen Struktur

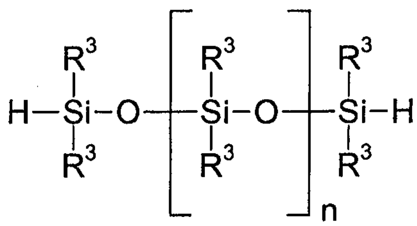

bevorzugt, wobei $R^3$ die oben angegebenen Bedeutung hat und n je nach gewünschter Wiederholungseinheit $V^1$ oder $V^2$, $n_2$ oder $n_1$ ist, die wie oben definiert sind. Sofern nicht kommerziell erhältlich, können diese Siloxane nach bekannten Verfahren, z.B. durch Äquilibrierung hergestellt werden (Silicone, Chemie und Technologie, Vulkan-Verlag, Essen 1989, S. 82-84).

Die einleitende Einführung der Strukturelemente $V^{2*}$ und Q kann z. B. auf zwei Wegen erfolgen.

Einerseits ist es möglich, zunächst tertiäre Aminofunktionen tragende ungesättigte Strukturen, beispielsweise N,N-Dimethylallylamin, durch Hydrosilylierung direkt an das Siloxan in $\alpha,\omega$-Stellung zu binden. Dieser Prozeß ist allgemein bekannt (B.Marciniec, Comprehensive Handbook on Hydrosilylation, Pergamon Press, Oxford 1992, S. 122-124).

Andererseits ist bevorzugt, durch Hydrosilylierung zunächst reaktive $\alpha,\omega$-funktionalisierte Zwischenprodukte zu erzeugen, welche nachfolgend in $\alpha,\omega$-ditertiäre Aminostrukturen oder direkt in die erfindungsgemäßen quartären Ammoniumstrukturen umgewandelt werden können. Geeignete Ausgangsstoffe zur Erzeugung reaktiver Zwischenstufen sind beispielsweise halogenierte Alkene oder Alkine, speziell Allylchlorid, Allylbromid, Chlorpropin und Chlorbutin, ungesättigte Halogencarbonsäureester, speziell Chloressigsäureallylester, Chloressig-säurepropargylester, 3-Chlorpropionsäureallylester und 3-Chlorpropionsäure-propargylester und epoxyfunktionelle Alkene, beispielsweise Vinylcyclohexenoxid und Allylglycidether. Die allgemeine Durchführung von Hydrosilylierungen mit Vertretern der genannten Stoffgruppen ist ebenfalls bekannt (B.Marciniec, Comprehensive Handbook on Hydrosilylation, Pergamon Press, Oxford 1992, S. 116-121, 127-130, 134-137, 151-155).

In einem nachfolgenden Schritt können die reaktiven Zwischenstufen dann mit sekundäre Aminofunktionen tragenden Verbindungen zur Reaktion gebracht werden. Geeignete Vertreter sind N,N-Dialkylamine, beispielsweise Dimethylamin, Diethylamin, Dibutylamin, Diethanolamin und N-Methylglucamin, cyclische sekundäre Amine, beispielsweise Morpholin und Piperidin, sekundäre Aminofunktionen tragende Aminoamide, beispielsweise die Umsetzungsprodukte von Diethylentriamin oder Dipropylentriamin mit Lactonen, wie y-Butyrolacton, Gluconsäure-$\delta$-lacton und Glucopyranosylarabonsäurelacton (DE-OS 4318536, Beispiele 11a, 12a, 13a), oder sekundär-tertiäre Diamine, wie beispielsweise N-Methylpiperazin. Es ist speziell bevorzugt, entsprechende Imidazol- oder Pyrazolderivate, speziell Imidazol und Pyrazol zur Einführung tertiärer Aminofunktionen zu nutzen.

Als Partner für die in einer Ausführungsform bevorzugt eingesetzten Epoxidderivate eignen sich besonders die genannten sekundär-tertiären Diamine, sowie auch Imidazol und Pyrazol. Auf diese Weise können die Alkylierungen regioselektiv und ohne zusätzlichen Aufwand an die Wasserstoffatome tragenden Stickstoffatome dirigiert werden.

Zur Absicherung einer quantitativen Umwandlung der reaktiven Gruppierungen in tertiäre Aminostrukturen werden die Amine in einem Verhältnis von $1 \leq \sum$ sekundäre Aminogruppen : reaktive Gruppen $\leq 10$, bevorzugt 1 bis 3, speziell 1 bis 2, ganz speziell 1 eingesetzt. Aminüberschüsse müssen ggf. entfernt werden.

Die Anbindung der vorstehend beschriebenen $\alpha,\omega$-ditertiären Aminosiloxane an $V^1$ entsprechende Monomer-Einheiten $-[V^1]-$ oder eine Präpolymereinheit $-[V^1-(Q-V^1)_x]$-führt zur Ausbildung von quarternären Ammoniumeinheiten und kann wieder-um auf zwei vorteilhaften Wegen erfolgen.

Einerseits ist es bevorzugt, separat ein stark hydrophiles, polyquaternäres, difunktionelles Vorkondensat $-[V^1-(Q-V^1)_x]-$ zu erzeugen, welches zu einem geeigneten Zeitpunkt mit den $\alpha,\omega$-ditertiären Aminosiloxanen vereinigt wird und zum poly-quaternären Siloxancopolymeren reagiert.

Die Herstellung hoch geladener, difunktioneller Präpolymere unterschiedlicher Kettenlänge $-[V^1-(Q-V^1)_x]-$ ist beispielhaft in WO 99/14300 (Beispiele 1 bis 7, Tabelle 11) beschrieben. In Abhängigkeit vom molaren Verhältnis von $V^1$ und dem Q zugrunde liegenden Amin kann entweder ein dem Wesen nach durch Aminogruppen terminiertes oder ein durch andere Reaktivgruppen terminiertes Präpolymer erzeugt werden.

Für den Fall der Anbindung eines durch Aminogruppen terminierten Präpolymer $-[N-(V-N)_x]-$ an die Aminfunktion einer $\alpha,\omega$-ditertiären Aminosiloxanstruktur kann beispielsweise ein der Wiederholungseinheit $V^1$ entsprechendes, quaternierendes, difunktionelles Monomer $-[V^1]-$, ausgewählt beispielsweise aus Bisepoxiden, Epichlorhydin, Bishalogenalkyl-Verbindungen, verwendet werden. Es braucht dabei nicht erwähnt zu werden, das unterschiedliche Gruppen $V^1$ im Präpolymer und im Verbindungsglied zwischen Präpolymer und $\alpha,\omega$-ditertiärer Aminosiloxanstruktur resultieren können.

Für den Fall eines gegenüber Aminogrupen mit Reaktivgruppen terminierten Präpolymers, wie-$[V^1-(Q-V^1)_x]$- kann eine direkte Anbindung an die Aminfunktion der $\alpha,\omega$-ditertiären Aminosiloxanstruktur ohne weiteren Linker, d.h. verbindende Übergangs- oder Zwischeneinheiten, erfolgen, da bei der Präpolymersynthese bereits ein Überschuß der $V^1$ erzeugenden Komponente eingesetzt wurde.

Alternativ zur separaten Herstellung eines Präpolymers $-[V^1-(Q-V^1)_x]-$ kann der Aufbau hoch geladener Blöcke parallel zum Einbau in das Copolymere erfolgen. Dies bedeutet, daß das $\alpha,\omega$-ditertiäre Aminosiloxan mit den Startkomponenten zum Aufbau von $-[V^1-(Q-V^1)_x]-$, d.h. beispielsweise $-[V^1]-$ und Mono oder Diamine der oben erwähnten Bedeutung -[N]- und/oder -[N-N-]- gemeinsam vorgelegt und zur Reaktion gebracht werden.

Schließlich ist es möglich, das $\alpha,\omega$-ditertiäre Aminosiloxan mit langkettiger Siloxaneinheit $Z^2$ oder kurzkettiger Siloxaneinheit $Z^1$ bzw. das $\alpha,\omega$-difunktionelle Siloxan - $[V^{2*}-Z^2-V^{2*}]$- oder $-[V^1]-$ in die vorgelegten Komponenten zum Aufbau von $-[V^1-(Q-V^1)_x]-$ über einen Zeitraum schrittweise zu dosieren oder aber umgekehrt diese Komponenten dem $\alpha,\omega$-ditertiären Aminosiloxan bzw. $\alpha,\omega$-difunktionellen Siloxan schrittweise hinzuzufügen.

Eine vorgelagerte Bereitstellung von durch Aminogruppen terminierten Präpolymeren, wie z.B. $-[N-(V^1-N)_x]-$ eröffnet die Möglichkeit, direkt mit geeigneten reaktiven Zwischenstufen, beispielsweise Epoxyderivaten, die Copolymerenbildung auszuführen.

Es ist ebenfalls bevorzugt, die reaktiven Zwischenstufen und die Startkomponenten für den Aufbau von $-[V^1-(Q-V^1)_x]-$ gemeinsam vorzulegen und anschließend zur Reaktion zu bringen.

Schließlich ist möglich, die reaktiven Zwischenstufen in die vorgelegten Komponenten zum Aufbau von $-[V^1-(Q-V^1)_x]-$ über einen Zeitraum schrittweise zu dosieren oder aber umgekehrt diese Komponenten der reaktiven Zwischenstufe schrittweise hinzuzufügen.

Unabhängig von der Wahl eines der vorstehend beschriebenen Reaktionswege und der damit eng verbundenen Frage, ob Aminoeinheiten zunächst das Siloxan oder aber das Präpolymer terminieren, wird die Gesamtstöchiometrie so gewählt, dass die Summe der Aminofunktionen und der mit ihnen reaktionsfähigen Gruppen etwa 1:1 beträgt.

Im Rahmen der Erfindung ist es möglich, von dieser bevorzugten Gesamtstöchiometrie abzuweichen. Es werden dann allerdings Produkte erhalten, die nicht mehr die in Abhängigkeit der Reaktionspartner maximal mögliche Länge des hoch geladenen, hydrophilen Blocks $-[V^1-(Q-V^1)_x]-$ aufweisen, sondern zusätzlich einen Überschuß einer nicht abreagierten Startkomponenten hinterlassen.

Neben der vorstehend behandelten Gesamtstöchiometrie der Reaktion ist für das Eigenschaftsbild der Produkte die Wahl der die Wiederholungseinheit $V^1$ bildenden Komponente(n) von großer Bedeutung.

Die Einführung von Alkylen-, Alkenylen-, Alkinylen- und Aryleinheiten erfolgt vorzugsweise ausgehend von den entsprechenden Halogeniden, speziell Chloriden und Bromiden. Beispielhafte Vertreter sind 1,6-Dichlorhexan, 1,4-Dichlorbut(2-)en, 1, 4-Dichlorbut(2-)in und 1,4-Bis(chlormethyl)benzol.

Polyalkylenoxideinheiten können ebenfalls über die $\alpha,\omega$-Dihalogenverbindungen eingeführt werden. Diese sind aus den oligomeren und polymeren Alkylenoxiden der allgemeinen Zusammensetzung

$$HO[CH_2CH_2O]_q-[CH_2CH(CH_3)O]_rH$$

wobei q und r die oben angegebenen Bedeutungen aufweisen, beispielsweise durch Chlorierung der Hydroxylgruppen mit $SOCl_2$ zugänglich (Organikum, Organischchemisches Grundpraktikum, 17. Auflage, VEB Deutscher Verlag der Wissenschaften, Berlin 1988, S. 189-190).

Mono-, di- oder polyhydroxyfunktionelle Einheiten als Gruppe $V^1$ können ausgehend von Epoxidderivaten eingeführt werden.

Kommerzielle Beispiele sind 1-Chlor- 2,3-Epoxypropan, der Glycerol-1,3-bis-glycidylether und Diethylenglykoldiglycidylether und Neopentylglykol-diglycidylether. Soweit nicht kommerziell verfügbar, können die gewünschten Diepoxide beispielsweise durch Reaktion der entsprechenden Diole mit 1-Chlor-2,3-Epoxypropan unter alkalischen Bedingungen synthetisiert werden.

Es liegt im Rahmen der Erfindung, in die Struktur von $V^1$ Siloxanketten $Z^1$ einzuführen. Hieraus ergibt sich u.a. die Möglichkeit, verschieden lange Siloxanketten für den Aufbau des Gesamtmoleküls zu verwenden. Es ist eine bevorzugte Variante, in $V^1$ Siloxanketten $Z^1$ des Kettenlängenbereichs $n_2$ = 0 bis 19, bevorzugt 0 bis 15, besonders bevorzugt 0 bis 10, speziell 0 bis 5, spezieller 0, einzubauen. Geeignete Startmaterialien zum Einbau sind z.B. die entsprechenden $\alpha,\omega$-Diepoxide.

Bei der Umsetzung von Epoxiden mit primären oder sekundären Aminen ist darauf zu achten, daß für Alkylierungen von tertiären Aminogruppen ein mol $H^+$ pro mol Epoxid/ tertiäres Amin zuzusetzen ist.

Die Wahl geeigneter Amine als Ausgangskomponenten für die Bildung von Q in der Wiederholungseinheit $-[V^1-(Q-V^1)_x]-$ bestimmt ebenfalls in hohem Maße die Molekülstruktur. Die Verwendung ditertiärer Amine, beispielsweise N,N,N',N'-Tetramethylethylendiamin, N,N,N',N'-Tetramethyltetramethylendiamin, N,N,N',N'-Tetra-methylhexamethylendiamin, N,N'-Dimethylpiperazin, führt zu Produkten, in denen jedes Stickstoffatom der Wiederholungseinheit quaterniert ist.

Die Verwendung von sekundär-tertiären Diaminen, beispielsweise N-Methylpiperazin, öffnet den Weg zu Wiederholungseinheiten $-[V^1-(Q-V^1)_x]-$, in denen tertiäre und quartäre Amin- bzw. Ammoniumstrukturen im Verhältnis 1 : 1 vorliegen. Eine teilweise oder vollständige nachträgliche Quaternierung verbliebener tertiärer Aminostrukturen stellt eine bevorzugte Variante zur Einstellung einer gewünschten hohen Dichte der quartären Ammoniumgruppen dar. Die entsprechenden aromatischen Amine Imidazol bzw. Pyrazol führen zu Produkten mit einer deloka-lisierten Ladung.

Bei Einsatz von primär-tertiären Diaminen, beispielsweise N,N-Dimethylpropylendiamin und 1-(3-Aminopropyl)imidazol, speziell in Kombination mit Diepoxiden, können kammartige Strukturen aufgebaut werden, für die der Quaternierungsgrad während einer abschließenden Alkylierung wählbar ist. Grundsätzlich können Quaternierungsgrade von durchschnittlich weniger als einer quartären Ammoniumgruppe pro Wiederholungseinheit $-[V^1-(Q-V^1)_x]-$ eingestellt werden. Es ist jedoch bevorzugt, mindestens ein Stickstoffatom pro Wiederholungseinheit zu quaternieren. Ausgehend von disekundären Aminen, beispielsweise Piperazin, N,N'-Bis(2-hydroxyethyl)-hexamethylendiamin, N,N'-Bis(2-hydroxypropyl)-hexamethylendiamin, können grundsätzlich auch Wiederholungseinheiten $-[V^1-(Q-V^1)_x]-$ mit einem durch-

schnittlichen Gehalt von weniger als einer quartären Ammoniumgruppe synthetisiert werden. Die disekundären Amine liefern hierbei zunächst polytertiär aminomodifizierte Siloxancopolymere oder aber Präpolymere, die in einer abschließenden Reaktion teilweise oder vollständig zu $-[V^1-(Q-V^1)_x]-$ quaterniert werden können. Es ist aber auch in dieser Variante bevorzugt, wenigstens ein Stickstoffatom pro Wiederholungseinheit zu quaternieren.

Als geeignete Quaternierungsagenzien kommen die allgemein bekannten Stoffgruppen wie Alkylhalogenide, Halogencarbonsäureester, Epoxidderivaten in Gegenwart von $H^+$ und Dialkylsulfate, speziell Dimethylsulfat, in Betracht.

Die Herstellung nicht kommerziell verfügbarer disekundärer Amine erfolgt in einer bevorzugten Ausführungsform ausgehend von den entsprechenden diprimären Aminen, beispielsweise Hexamethylendiamin durch Alkylierung mit Epoxiden, wie z.B. Ethylenoxid, Propylenoxid, Isopropylglycidether unter Ausnutzung der unterschiedlichen Reaktionsgeschwindigkeiten primärer und sekundärer Amine.

Es war bereits dargelegt worden, daß im Rahmen der Erfindung die Möglichkeit besteht, Siloxanketten $Z^1$ in die Struktur von $V^1$ einzuführen. Als geeignete Startmaterialien wurden exemplarisch die reaktiven Zwischenstufen $\alpha,\omega$-Diepoxide benannt.

Als die aus den Ammoniumgruppen resultierenden positiven Ladungen neutralisierende Anionen $A^-$ kommen bevorzugt die während der Quaternierung gebildeten Ionen, wie Halogenidionen, speziell Chlorid und Bromid, Alkylsulfate, speziell Methosulfat, Carboxylate, speziell Acetat, Propionat, Octanoat, Decanoat, Dodecanoat, Tetradecanoat, Hexadecanoat, Octadecanoat, Oleat, Sufonate, speziell Toluensulfonat in Betracht. Jedoch können durch Ionenaustausch auch andere Anionen eingeführt werden. Zu nennen sind beispielsweise organische Anionen, wie Polyethercarboxylate und Polyethersulfate.

Die Einführung der funktionellen Gruppe der Formel (I), die unten ausführlicher erläutert wird umfasst beispielsweise:

a) Die Umsetzung von Diisocyanaten, enthaltend die funktionelle Gruppe der Formel (I), mit mindestens einem Mol eines Diamins (1) unter Bildung eines monomeren, oligomeren oder polymeren Diamins (2), dass die funktionelle Gruppe der Formel (I) enthält,

b) die Umsetzung von einem Mol eines Diisocyanates, enthaltend die funktionelle Gruppe der Formel (I), mit mindestens einem Mol einer multi-, bevorzugt difunktionellen, zur Reaktion mit den Isocyanatgruppen und Aminogruppen befähigten organischen Verbindungen (1), unter Bildung einer multi-, bevorzugt difunktionellen, zur Reaktion mit Aminogruppen befähigten monomeren, oligomeren oder polymeren organischen Verbindung (2), enthaltend die Gruppe der Formel (I),

c) die Umsetzung von einem Mol eines Diisocyanates, enthaltend die funktionelle Gruppe der Formel (I), mit mindestens einem Mol einer multi-, bevorzugt difunktionellen, zur Reaktion mit den Isocyanatgruppen befähigten organischen Verbindungen (1) unter Bildung einer multi-, bevorzugt difunktionellen, organischen monomeren, oligomeren oder polymeren Verbindung (2), enthaltend die funktionelle Gruppe der Formel (I) und terminale zur Reaktion mit Isocyanatgruppen befähigte Gruppen, Überführung der genannten organischen Verbindung (2) in eine multi-, bevorzugt difunktionellen, zur Reaktion mit Aminogruppen befähigte monomere, oligomere oder polymere organischen Verbindung (3)

und Verwendung der erhaltenen, die Gruppe der Formel (I) enthaltenden Verbindungen in den Verfahren a) bis d) des oben beschriebenen Verfahrens.

Die Einführung der funktionellen Gruppe der Formel (VIIIa), die unten ausführlicher erläutert wird, umfasst beispielsweise die Umsetzung einer Aminverbindung, ausgewählt aus einer Diamin-Verbindung und/oder einer primären, sekundären oder tertiären Monoaminverbindung, enthaltend die Einheit der Formel (VIIIa), und/oder die Umsetzung einer multi-, bevorzugt difunktionellen organischen Verbindung, enthaltend die Einheit der Formel (VIIIa).

In dem Verfahren zur Herstellung der erfindungsgemäßen Verbindungen werden die funktionellen Gruppen der multi-, bevorzugt difunktionellen, zur Reaktion mit Aminofunktionen befähigten Verbindungen bevorzugt ausgewählt aus der Gruppe, die besteht aus Epoxygruppen und Halogenalkylgruppen.

Die erfindungsgemäßen Verbindungen, die eine funktionelle Gruppe der Formel (I)

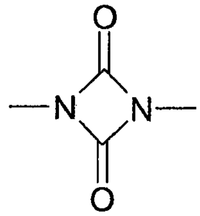

(I)

enthalten, weisen die genannte Gruppe der Formel (I) zweckmäßig in einer Gruppe V auf.

Die erfindungsgemäßen Verbindungen, die die funktionelle Gruppe der Formel (I) aufweisen, enthalten bevorzugt die Einheit der Formel (Ia)

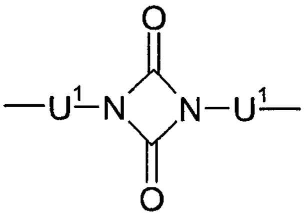

(Ia)

worin

$U^1$ ausgewählt wird aus der Gruppe, die besteht aus zweiwertigen Resten der Formeln:

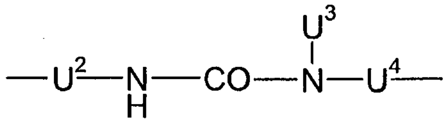

(Ib),

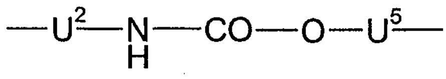

(Ic)

und

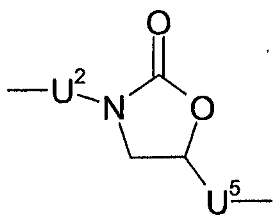

(Id)

wobei

$U^2$ mit dem Stickstoffatom der funktionellen Gruppe der Formel (I) verbunden ist, und

$U^2$ einen zweiwertigen, geradkettigen, cyclischen oder verzweigten, gesättigten, ungesättigten oder aromatischen Kohlenwasserstoffrest mit bis zu 100, bevorzugt bis zu 30 Kohlenstoffatomen darstellt, der eine oder mehrere Gruppen -O- enthalten kann,

$U^2$ ist bevorzugt ein zweiwertiger, geradkettiger Kohlenwasserstofftest mit bis zu 15 Kohlenstoffatomen, beispielsweise Hexamethylen,

zweiwertige, cyclische Kohlenwasserstoffteste mit bis zu 15 Kohlenstoffatomen, beispielsweise auf Basis von Bis-cyclohexyl-methanstrukturen

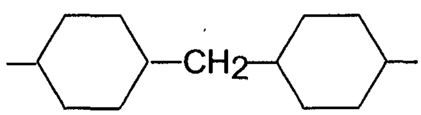

zweiwertige, verzweigte Kohlenwasserstoffteste mit bis zu 15 Kohlenstoffatomen, beispielsweise auf Basis von Methylcyclohexyl- oder Isophoronstrukturen

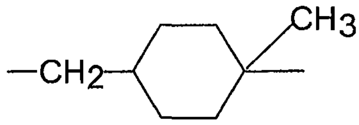 oder 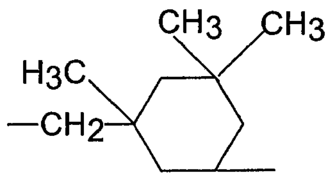

zweiwertige, aromatische Kohlenwasserstoffteste mit bis zu 15 Kohlenstoffatomen, beispielsweise auf Basis von 2,4-Toluyl, 2,6-Toluyl, Bis-phenyl-methan- und Naphthyl enstrukturen.

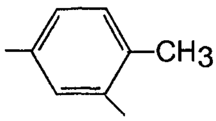 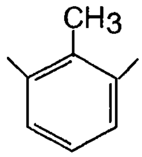 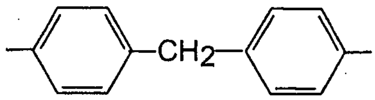 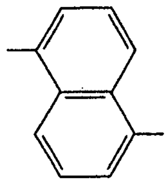

$U^3$ kann Wasserstoff oder ein einwertiger, geradkettiger, cyclischer oder verzweigter, gesättigter, ungesättigter oder aromatischer Kohlenwasserstoffrest mit bis zu 100, bevorzugt bis zu 30 Kohlenstoffatomen darstellen, der eine oder mehrere Gruppen -O- enthalten und durch OH substituiert sein kann,
$U^4$ und $U^5$ stellen zweiwertige geradkettige, cyclische oder verzweigte, gesättigte, ungesättigte oder aromatische Kohlenwasserstoffreste mit bis zu 1000, bevorzugt bis zu 200, noch bevorzugter bis 100 Kohlenstoffatomen dar, die gegebenenfalls eine oder mehrere Gruppen, ausgewählt aus -O-, -C(O)-,

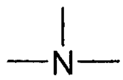

, $-NR^2-$, worin $R^2$ wie oben definiert ist, enthalten können und die gegebenenfalls durch eine oder mehrere Hydroxylgruppen substituiert sein können, mit der Maßgabe, dass die Gruppen

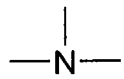

$-NR^2-$ an ein Carbonylkohlenstoffatom binden.
U3 kann darüber hinaus aus den Gruppen $-W-Si(OR)_{3-a}(R')_a$ bestehen, worin R, R' und a wie oben definiert sind und W einen zweiwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen -C(O) -, -O-, NH-, -S- enthalten kann, und gegebenenfalls durch Hydroxygruppen substituiert sein kann.
$U^3$ bedeutet bevorzugt einen einwertigen, geradkettigen, cyclischen oder verzweigten, gesättigten, ungesättigten Kohlenwasserstoffrest mit bis zu 15 Kohlenstoffatomen, der eine oder mehrere -O- Gruppierungen und Siliciumatome enthalten und durch OH substituiert sein kann, beispielsweise Methyl, Ethyl, Propyl, Butyl, Hexyl, sowie Reste der nachfolgenden Formeln

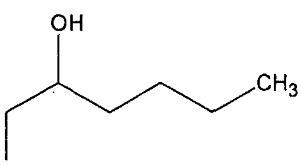 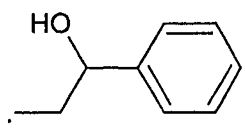

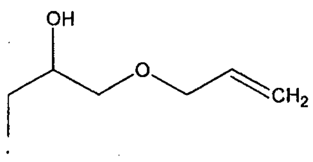

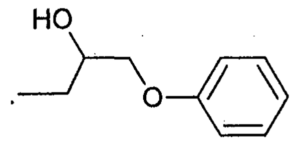

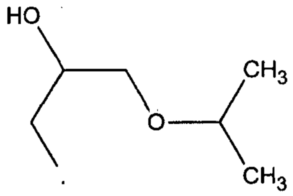

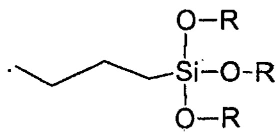

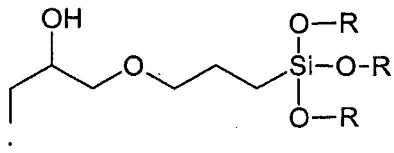

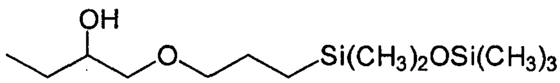
,

wobei die Reste $U^3$, die Trialkoxysilylgruppen enthalten, zu Verbindungen führen, die sowohl funktionelle Gruppen der Formel (I) als auch funktionelle Gruppen der Formel (VIIIa) aufweisen. $U^4$ und $U^5$ Gruppen, wie Alkyleneinheiten, beispielsweise Dimethylen, Trimethylen, Hexamethylen, oder Alkylenestereinheiten, speziell abgeleitet von veresterten Alkandiolen, Alkendiolen, Alkindiolen und Polyalkylenoxiden, beispielsweise

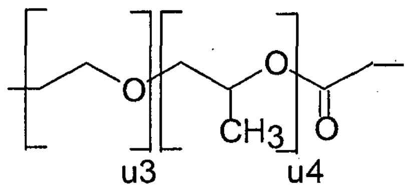

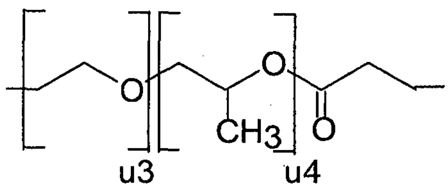

oder Oligoalkylenoxideinheiten, beispielsweise

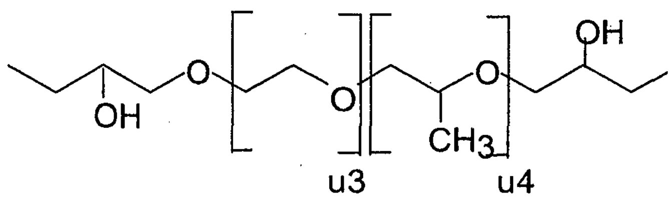

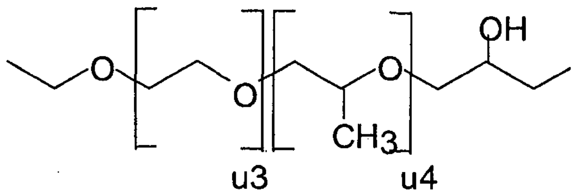

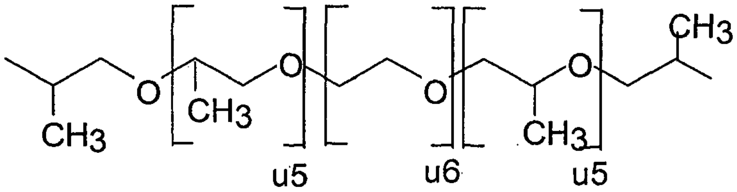

oder polysiloxanhaltige Einheiten, beispielsweise

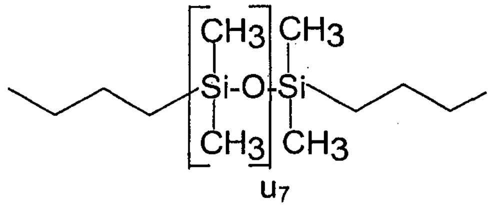

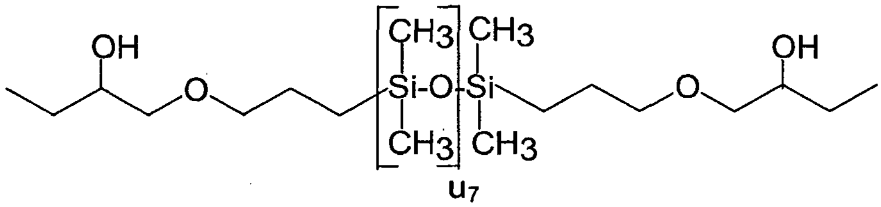

mit

$u_3$ 0 bis 100,
$u_4$ 0 bis 100,
$u_3 + u_4 \geq 1$,
$u_5$ 1 bis 100,
$u_6$ 1 bis 100,
$u_7$ 1 bis 300.

Zur Einführung der Einheiten gemäß Formel I in die erfindungsgemäßen polyquaternären Polysiloxancopolymere werden über Uretdionsubstrukturen verfügende Monomere bevorzugt des Typs

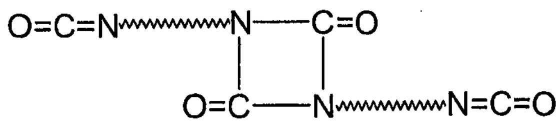

zunächst adäquat vorfunktionalisiert.

Die Wellenlinie deutet an, daß es sich grundsätzlich um frei wählbare Spacer, d.h. verknüpfende, mindestens zweiwertige Gruppen $U^2$, zwischen der Uretdionstruktur und den Isocyanatgruppen handelt. Dies schließt u.a. ein, daß sich weitere reaktive Isocyanatgruppen in den Startmolekülen befinden.

Diese Startmoleküle werden durch Dimerisierung entsprechender, bevorzugt Diisocyanate gewonnen (H.J. Laas, R.Halpaap, J. Pedain, Journal f. Prakt. Chemie 336[1994], 185-200; H.J. Laas, R.Halpaap, J. Pedain, Farbe+Lack 100[1994], 330-336) und sind kommerziell von der Bayer AG Leverkusen unter der Typenbezeichnung DESMODUR® erhältlich. Besonders bevorzugte Diisocyanate sind Hexamethylendiisocyanat, Isophorondiisocyanat, Bis-(4-isocyanatocyclohexyl)-methan, Toluylen -2,4-diisocyanat, Toluylen-2,6-diisocyanat, Bis-(4-isocyanatophenyl)methan, Naphthylen-1,5-diisocyanat, 1-Isocyanato-1-methyl-4(3)-isocyanato-methylcyclohexan, 5-Methyl-1,9-diisocyanatononan, 2,4-Dimethyl-1,8-diisocyanato-octan, 2-Methyl-1,5-diisoeyanatopentan und 2-Ethyl-1,4-diisocyanatobutan.

Gemäß Formeln I(b) bis I(d) erfolgt die Verknüpfung der Uretdionstrukturen mit dem Restmolekül über Harnstoff-, Urethan- und Oxazolidinongruppen, die an eine Gruppe $U^4$ bzw. $U^5$ binden bzw. überleiten.

$U^4$ bzw. $U^5$ wird durch Reaktion von Vorstufen von $U^4$ bzw. $U^5$ wie difunktionellen Verbindungen vom Aminoalkyl-, Hydroxyalkyl- und Epoxyalkyltyp mit Isocyanat gebildet, wobei definitionsgemäß gegebenenfalls nur ein Teil des

Moleküls der mit Isocynatgruppen reagierenden Vorstufen durch die Einheit $U^4$ bzw. $U^5$ bzw. V wiedergegeben wird und der restliche Teil, wenn er z.B. eine Aminogruppe darstellt, die mit Isocyanat nicht zur Reaktion gebracht wurde, d.h. nicht zu einer Ureidogruppe umgewandelten Einheit wurde, unter die Definition von Q fällt.

Bei den difunktionellen Aminoverbindungen, die mit den bevorzugten Uretdionhaltigen Diisocyanaten reagieren, handelt es sich beispielsweise um- diprimäre Diaminoalkylverbindungen, wie Ethylendiamin, 1,2-Propylendiamin, 1,3-Propylendiamin und 1,6-Hexandiamin,

- siloxanhaltige diprimäre Diaminoalkylverbindungen, wie $\alpha,\omega$-aminopropyl-substituierte geradkettige Siloxane,
    - diprimäre Diaminopolyether vom Ethylenoxid - und/oder Propylenoxidtyp, wie die Jeffamine® der ED-Reihe (Huntsman Corp.),
    - primär-sekundäre Diaminoalkylverbindungen, wie beispielsweise 2-Hydroxy-ethylethylendiamin, N(2-Aminoethyl)piperazin,
- siliciumhaltige primär-sekundäre Diaminoalkylverbindungen, wie beispielsweise aminoethylaminopropylsubstituierte Alkoxysilane der Dynasilan®-Reihe (Degussa),
- disekundäre Diaminoalkylverbindungen, wie beispielsweise Piperazin,
- siloxanhaltige disekundäre Diaminoalkylverbindungen, wie die Umsetzungsprodukte von $\alpha,\omega$-glycidylsubstituierten geradkettigen Siloxanen mit monofunktionellen primären Aminen,
- Silylgruppen-haltige disekundäre Diaminoalkylverbindungen, wie beispielsweise die Umsetzungsprodukte von $\alpha,\omega$-glycidylsubstituierten Polyethern (DER®-Typen Dow Chemicals) mit aminopropylsubstituierten bzw. aminoethylaminopropylsubstituierten Alkoxysilanen (Dynasilan®-Reihe Degussa) oder die Umsetzungsprodukte von diprimären Aminen mit epoxyfunktionalisierten Alkoxyssilanen (GLYMO Silan® Degussa),
- disekundäre Diaminopolyether vom Ethylenoxid - und/oder Propylenoxidtyp, wie die Umsetzungsprodukte von $\alpha,\omega$-glycidylsubstituierten Polyethern (DER®-Typen Dow Chemicals) mit monofunktionellen primären Aminen oder die Umsetzungsprodukte von diprimären Diaminopolyethern vom Ethylenoxid - und/oder Propylenoxidtyp, wie die Jeffamine® der ED-Reihe (Huntsman Corp.) mit monofunktionellen Epoxiden,
- primär-tertiäre Diaminoalkylverbindungen, wie N,N-Dimethylpropylendiamin und N-(3-Aminopropyl)imidazol,
- sekundär-tertiäre Diaminoalkylverbindungen, wie N-Methylpiperazin.

Bei den difunktionellen Hydroxyverbindungen, die mit den bevorzugten Uretdionhaltigen Diisocyanaten reagieren, handelt es sich beispielsweise um hydroxy-funk-tionalisierte tertiäre Amine, die durch Mono-/Oligoalkoxylierung entsprechender, sekundäre Aminofunktionen aufweisender Moleküle gewonnen werden, wie 2-Hydro-xyethyl-dimethylamin, N-(2-Hydroxyethyl)-N'-Methyl-piperazin und -hydroxyfunktionalisierte Ester von Halogencarbonsäuren, speziell Monoester von Monohalogencarbonsäuren mit Diolen, ganz speziell der Chloressigsäure und 3-Chlorpropionsäure, wie beispielsweise $HOCH_2CH_2OC(O)CH_2Cl$,

- hydroxyfunktionalisierte Epoxide, wie Glycidol,
  Bei den difunktionellen Epoxyverbindungen, die mit den bevorzugten Uretdion-haltigen Diisocyanaten reagieren, handelt es sich beispielsweise um
- Diepoxide aud Basis von Alkylenoxiden, speziell vom vom Ethylenoxid - und/oder Propylenoxidtyp, wie $\alpha,\omega$-Glycidyl-substituierte Polyether vom DER®-Typ (Dow Chemicals)-$\alpha,\omega$-Glycidyl- bzw. $\alpha,\omega$-Cyclohexyloxy- substituierte geradkettige Polydiorganosiloxane.

Die Gruppen $U^4$ und $U^5$ sind über funktionelle Strukturen, bevorzugt auf Basis von Estern, Hydroxyalkyleinheiten mit Amin- bzw. quaternären Ammoniumeinheiten Q verbunden und sind über Q- sowie weitere V-Einheiten mit dem Restmolekül verknüpft. Dies bedeutet, daß die Gruppen $U^4$ bzw. $U^5$ während der Verknüpfungsreaktion einerseits alkylierend wirken können, wie im Fall der Ester- und Hydroxyalkyleinheiten. Die Gruppen $U^2$, $U^3$ , $U^4$ und $U^5$ fallen somit unter die Definition von V, es sind Teilstrukturen von V.

Die erfindungsgemäßen Verbindungen enthalten eine Einheit Q mit mindestens einem Rest $R^1$ der Formel (VIIIa)

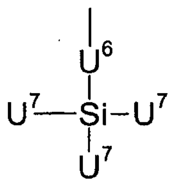

( VIIIa ),

worin

$U^6$ einen zweiwertigen geradkettigen, cyclischen oder verzweigten, gesättigten, ungesättigten oder aromatischen Kohlenwasserstoffreste mit bis zu 100 Kohlenstoffatomen darstellt, der gegebenenfalls eine oder mehrere Gruppen, ausgewählt aus -O-, -C(O)-, -NH- und $-NU^8-$ enthalten kann, oder gegebenenfalls durch eine oder mehrere Hydroxylgruppen substituiert sein kann, worin $U^8$ Wasserstoff oder einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen -O- enthalten und durch OH substituiert sein kann, mit der Maßgabe, dass -NH- und $-NU^8-$ an ein Carbonyl- und/oder Thiocarbonylkohlen-stoffatom gebunden ist, und

$U^7$ einen einwertigen, geradkettigen, cyclischen oder verzweigten, gesättigten, ungesättigten oder aromatischen Kohlenwasserstoffrest mit bis zu 20 Kohlenstoffatomen darstellt, der eine oder mehrere Gruppen -O- enthalten und durch OH substituiert sein kann, mit der Maßgabe, dass die Reste $U^7$ gleich oder verschieden sein können und mindestens ein Rest $U^7$ pro Siliciumatom über -O- an das Siliciumatom gebunden ist.

Die Gruppen, die die Formeln (VIIIa) aufweisen, können beispielsweise unter Verwendung von primäre, sekundäre tertiäre Aminogruppen enthaltenden Silane der Formel (IX) wie z.B.

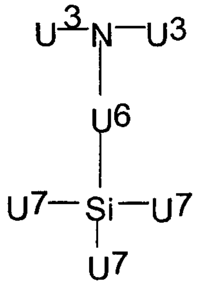

(IXa)

worin, $U^3$, $U^6$ und $U^7$ wie oben definiert sind, eingeführt werden. Sie können je nach Alkylierungsgrad bzw. Zeitpunkt der Zugabe bei der Co- bzw. Terpolymerisation als Polymerkettenstopper wirken, insbesondere wenn es sich um tertiäre Amine handelt.

Bei Verwendung von primäre Aminogruppen und/oder mehrere Aminogruppen enthaltenden Silanen für diesen Zweck ist die Stöchiometrie der Gesamtreaktion zu beachten. Beispiele bevorzugter Materialien sind

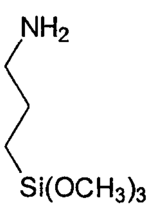 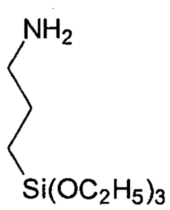 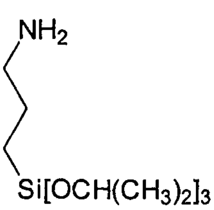 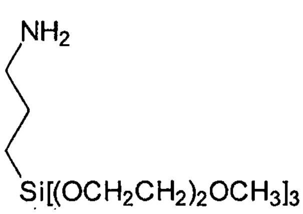

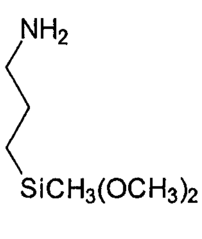 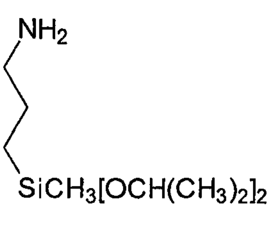 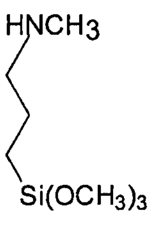 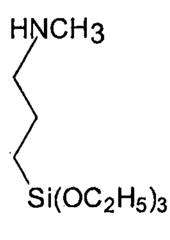 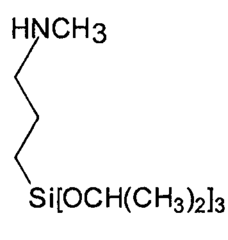

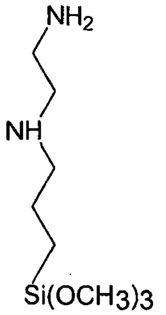
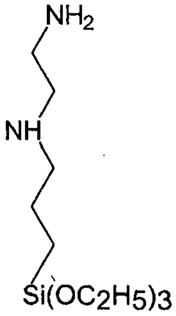
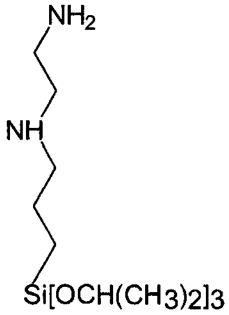
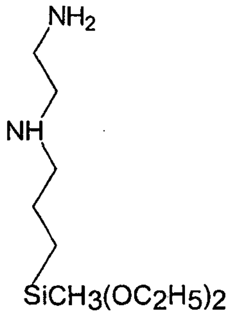
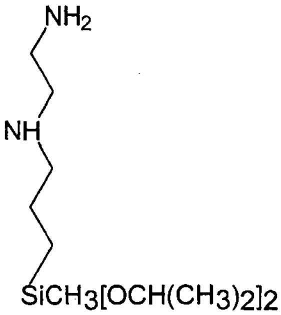

Die Struktur der Aminogruppe entscheidet über die Art der Anbindung an das Polymermolekül, während über das Substitutionsmuster am Siliciumatom die Geschwindigkeit der Hydrolyse in wässriger Umgebung und die finale Netzwerkdichte gesteuert werden können. Generell führen sperrigere Alkoxysubstituenten zu einer Verlangsamung der Hydrolyse in wässriger Umgebung. Die partielle Einführung nicht hydrolysierbarer Alkylsubstituenten reduziert die Funktionalität der Netzwerkdichte und damit den Umfang der Vernetzung.

Aminosilane der dargestellten Typen sind entweder kommerziell verfügbar (Dynasilane® Degussa) oder können z.B. durch basisch katalysierten Austausch von Alkoxygruppen am Siliciumatom in die gewünschten Materialien überführt werden. Es ist weiterhin möglich, spezielle Startsilane durch Alkylierung von Ammoniak oder primären Aminen mit z.B. epoxyfunktionalisierten Silanen wie

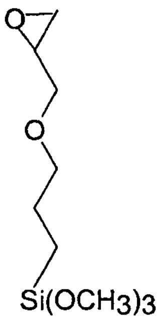

oder dem analogen Cyclohexyloxyderivat zu gewinnen.

Bevorzugte Ausgangsstoffe sind weiterhin monofunktionelle, primäre Aminogruppen enthaltende Silane (IXb):

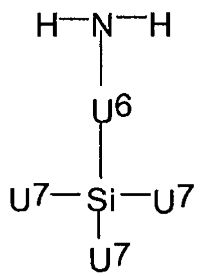

(IXb)

Sie wirken als Polymerkettenverlängerer, wenn die Polymerbildungsreaktion als zweifache Alkylierung des Stickstoffatomes zur Kettenfortpflanzung erfolgt. Der Alkylierungsgrad bzw. eine geeignete Einstellung der Gesamtstöchiometrie der Polymerbildungsreaktion ermöglicht dies. Beispiele sind :

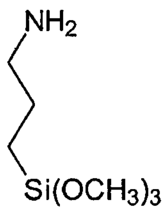 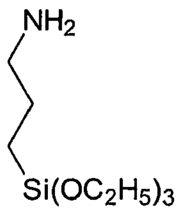 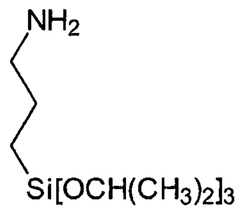 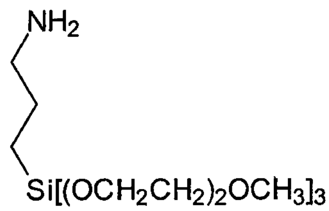

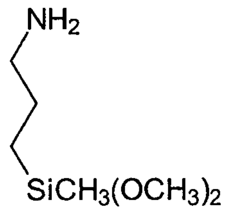 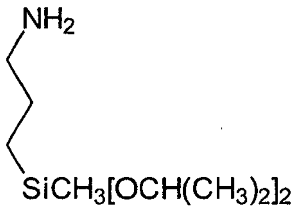

Bevorzugte Ausgangsstoffe sind weiterhin tertiäre Aminogruppen enthaltende Silane (IXa):

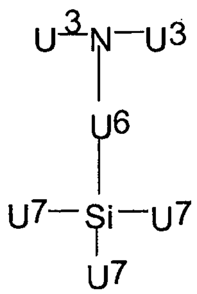

(IXa)

Sie wirken als Polymerkettenstopper, wenn im Verlauf der Polymerbildungsreaktion eine Quaternierung der tertiären Aminogruppe erfolgen kann. Geeignete Startsilane, besonders methylierte Derivate können durch Hydrosilylierung von z.B. N,N-Dimethylallylamin mit entsprechenden H-Silanen gewonnen werden. Alternativ ist es möglich, epoxyfunktionalisierte Silane wie

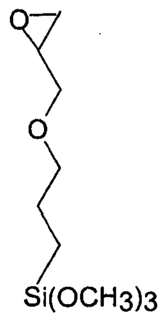

oder das analoge Cyclohexyloxyderivat als Reaktionsprodukt mit sekundären Aminen, wie Dimethylamin oder komplexeren methylierten Aminen, wie N-Methylpiperazin umzusetzen.

Die Reaktion von Isocyanatosilanen, wie 3-Isocyanatopropyltriethyoxysilan (ABCR GmbH), mit primär-tertiären Diaminen, beispielsweise N,N-Dimethylpropylendiamin und N-(3-Aminopropyl)imidazol, sekundär-tertiären Diaminen, beispielsweise N-Methylpiperazin, hydroxyfunktionellen Aminen, beispielsweise 2-Hydroxyethyldimethylamin und komplexeren Aminen, wie N-(2-Hydroxyethyl)-N'-Methyl-piperazin führt ebenfalls zu den gewünschten Startsilanen.

Es ist ebenfalls möglich, primäre oder sekundäre Aminofunktionen aufweisende Silane, wie

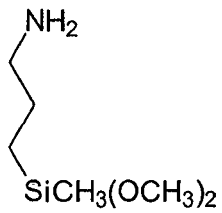 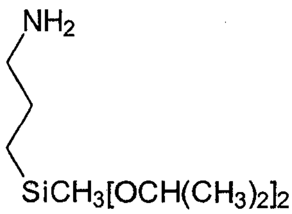

mit monofunktionellen Epoxiden in die gewünschten tertiären Strukturen zu überführen. Schließlich ist es möglich, nach Abschluß der Polymerbildungsreaktion der Formeln (IX) durch Alkylierung mit geeigneten Alkylierungsmitteln gegebenenfalls quaternisierbare Aminogruppen in quaternisierte Ammoniumgruppen zu überführen. Weitere Ausgangsstoffe sind tris-tertiäre Aminogruppen enthaltende Silane:

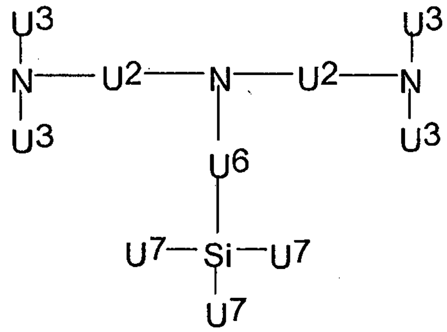

(IXb)

worin $U^2$, $U^3$, $U^6$ und $U^7$ wie oben definiert sind. Die Verbindungen (IX) können wie alle di- bzw. höherwertigen Amine als Polymerkettenverlängerer wirken, wenn sie mit einem di- oder höherwertigen Alkylierungsmittel umgesetzt werden, d.h. wenn im Verlauf der Polymerbildungsreaktion eine Quaternierung zweier tertiärer Aminogruppen erfolgen kann. Geeignete Startsilane, besonders N-methylierte Derivate, können bevorzugt durch Alkylierung von Triaminen, die über zwei tertiäre und eine sekundäre Aminofunktion verfügen, mit epoxyfunktionalisierten Silanen wie

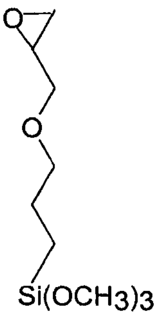

oder dem analogen Cyclohexyloxyderivat erhalten werden. Ein Beispiel für ein bevorzugtes Triamin ist N,N,N',N'-Tetramethyldipropylentriamin (Jeffcat®ZR50B, Huntsman Corp.). Die Verwendung N-methylierter Aminokomponenten, speziell von N,N-Dimethylstrukturen sichert ab, daß sich der nachfolgende Einbau dieser Monomeren in das Polymermolekül praktisch regioselektiv an den methylierten Stickstoffatomen vollzieht.

Es ist ebenfalls möglich, Triamine, wie N,N,N',N'-Tetramethyldipropylentriamin mit monofunktionellen Isocyanatosilanen, beispielsweise 3-Isocyanatopropyl-triethyoxysilan (ABCR GmbH) umzusetzen. In diesem Fall verfügt das dann gebildete Startsilan über zwei tertiäre Aminogruppen und eine Harnstoffgruppierung.

Derartige Isocyanatosilane eröffnen ebenfalls die Möglichkeit, ditertiäre Amine mit zusätzlicher Hydroxylfunktion als Aminbasis zu nutzen. Beispiele sind entsprechende Ethylenoxid- bzw. Propylenoxidderivate, wie N,N-Bis-(3-Dimethylaminopropyl-)N-isopropanolamin (Jeffcat®ZR50, Huntsman Corp.) und N,N,N'-Trimethyl-N'-hydro-xyethylbisaminoethylether (Jeffcat®ZF10, Huntsman Corp.). Im Ergebnis einer derartigen Vorfunktionalisierung werden ditertiäre Amine zugänglich, die die Silangruppierung über eine Urethanstruktur anbinden.

Zum Aufbau vornehmlich oligomerisierten Silanstrukturen sind $\alpha,\omega$-NH terminierte Oligostrukturen als Ausgangsstoff bevorzugt.

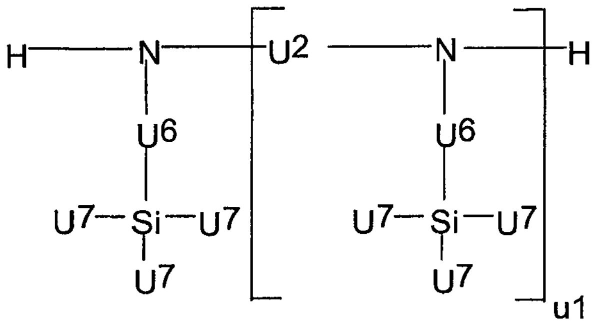

(IXc)

worin $U^2$, $U^6$, $U^7$ wie oben definiert sind, und $u_1$ 0 bis 10 ist.

Zu deren Synthese stellen monofunktionelle primäre Aminosilane, wie 3-Amino-propylsilane, und Diepoxyderivate, beispielsweise Diepoxide auf Basis von Alkylenoxiden, speziell vom vom Ethylenoxid - und/oder Propylenoxidtyp, wie $\alpha,\omega$-glycidylsubstituierte Polyether vom DER®-Typ (Dow Chemicals) und $\alpha,\omega$-glycidyl bzw. $\alpha,\omega$-cyclohexyloxy substituierte geradkettige Siloxane, die bevorzugten Startmaterialien dar. Durch gezielte Einstellung der Stöchiometrie zwischen difunk-tionell reagierender Aminosilankomponente und Diepoxid kann in einer Oligomerisierungsreaktion ein Präpolymert (IXc) erzeugt werden, welches $\alpha,\omega$-NH tenniniert ist und in den nachfolgenden Polymerisationsprozeß einbezogen werden kann. Zur Erreichung dieser $\alpha,\omega$-NH Terminierung ist ein Überschuß an Aminosilankomponente notwendig.

Zum Aufbau vornehmlich oligomerisierten Silanstrukturen (IXd) wird das zuvor dargelegte Konzept zur Erzeugung der Oligostrukturen dahingehend abgewandelt, daß in definierter Menge, bevorzugt zeitlich versetzt, sekundär-tertiäre Diaminosilane in den Oligomerisierungsprozeß eingespeist werden.

Hierbei handelt es sich um Silane, die bevorzugt durch Reaktion von primär-tertiären Diaminen, wie N,N-Dimethylpropylendiamin und N-(3-Aminopropyl)imidazol mit Epoxysilanen, beispielsweise von Glycidyl- und Cyclohexyloxytyp erhalten werden. Diese sekundär-tertiären Diaminosilane werden dem binären System, bestehend aus mono-primär-amino-funktionellem Silan und Diepoxyderivat, zugesetzt und führen bestimmt durch Reaktiviät und Zeitfolge, da monofunktionell reagierend, zu einem Präpolymer, welches in $\alpha,\omega$-Position durch tertiäre Aminogruppen, bevorzugt N,N-Dimethylgruppen terminiert und in der folgenden Formel dargestellt ist:

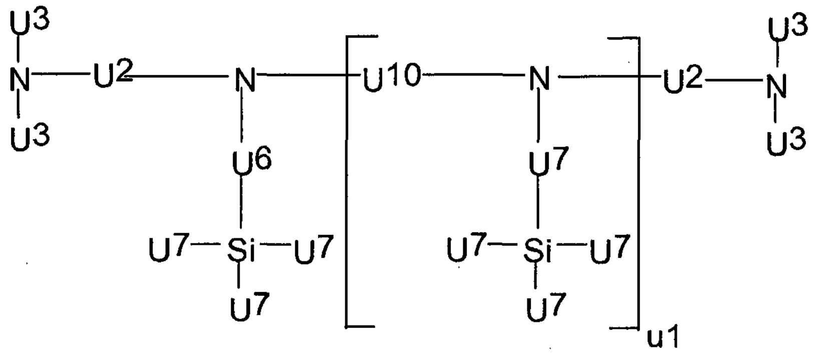

(IXd)

worin $U^3$, $U^6$ und $U^7$ und $u_1$ wie oben definiert sind und $U^{10}$ ein zweiwertiger organischer Rest ist.

Diese terminalen tertiären Aminogruppen können schließlich im Polymerisationsprozeß zur Kettenverlängerung genutzt werden.

Zum Ausbau dargestellten der vornehmlich oligomerisierten Silanstrukturen (Xa) sind $\alpha,\omega$-epoxyterminierte Oligostrukturen als Ausgangsstoff bevorzugt

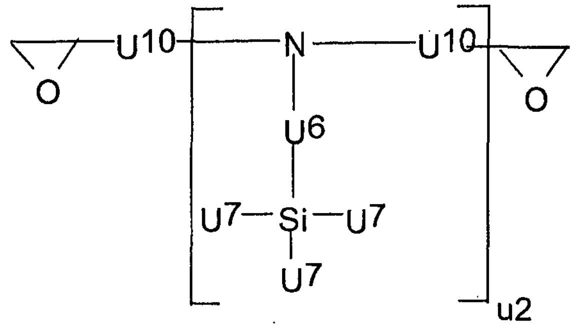

(Xa)

worin $U^3$, $U^6$, $U^7$, $U^{10}$ wie oben definiert sind und $u_2$ = 1 bis 10 ist.

Zur Synthese von (Xa) wird das oben dargestellte Konzept so modifiziert, daß durch gezielte Einstellung der Stöchiometrie zwischen difunktionell reagierender Aminosilankomponente und Diepoxid während der Oligomerisierungsreaktion ein Kondensat erzeugt werden, welches $\alpha,\omega$-epoxyterminiert ist und in den nachfolgenden Polymerisationsprozeß einbezogen werden kann. Zur Erreichung dieser $\alpha,\omega$-Epoxy-ter-minierung ist ein Überschuß an Diepoxid notwendig.

Bevorzugte Ausgangsmaterialien sind wiederum monofunktionelle primäre Aminosilane, wie 3-Aminopropylsilane, und Diepoxyderivate, beispielsweise Diepoxide auf Basis von Alkylenoxiden, speziell vom vom Ethylenoxid- und/oder Propylenoxidtyp, wie $\alpha,\omega$-glycidylsubstituierte Polyether vom DER®-Typ (Dow Chemicals) und $\alpha,\omega$-glycidyl bzw. $\alpha,\omega$-cyclohexyloxy substituierte geradkettige Siloxane.

Die Bereitstellung derartiger Silylalkoxy-haltiger Präpolymere führt nicht nur zu einer starken Erhöhung der Amin- bzw. Ammoniumeinheiten in diesem Segment, sondern auch zu einer hohen Konzentration von vernetzbaren Alkoxysilyleinheiten. Damit kann in besonderem Maße Substantivität und Hydrophilie durch diese Polymerblöcke vermittelt werden.

Die zur Einbindung der erfindungsgemäßen Uretdionsubstrukturen der Formel (I) und Silansubstrukturen der Formel (VIIIa) notwendigen Monomeren verfügen bevorzugt über alkylierbare Gruppen der Typen

- primäres Amin
- sekundäres Amin
- tertiäres Amin

und alkylierende Gruppen der Typen

- Halogencarbonsäureester
- Epoxid.

Zur erfolgreichen Einführung dieser Monomeren in die Polymermoleküle ist deren molarer Gehalt an alkylierbaren Gruppen bzw. alkylierenden Gruppen in die molare Gesamtbilanz der Polymerbildungsreaktion einzubeziehen. Einzelheiten zu diesen Bilanzen sind beispielsweise in WO 02/10256, WO 02/10257, WO 02/10259 und DE-100 36 533, DE 100 36 522, EP 282720, US 6240929, DE 33 40 708, DE 102 12 470.1, DE 102 51 525.5 und DE 102 51 526.3 dargelegt.

Bevorzugt gilt für die erfindungsgemäßen Gesamtreaktionen im wesentlichen

$$\Sigma\text{mol (primäres+sekundäres + tertiäres) Amin} = \Sigma\text{mol alkylierende Gruppen.}$$

Je nach Art der beabsichtigten Polymerbildungsreaktion kann hierbei eine primäre Amionogruppe mono- dioder trivalent eingerechnet werden. Sekundäre Amine können mono- oder divalent auftreten.

Abweichungen von dieser molaren Gesamtbilanz sind möglich. Vorteilhaft können Abweichungen von einer ausgeglichenen molaren Gesamtbilanz zur Erzeugung von Materialien mit spezifischen Endgruppen genutzt werden. So lassen sich beispielsweise Aminoendgruppen durch gezielten molaren Überschuß an Aminomonomeren erzeugen. Die erfindungsgemäßen Uretdionsubstrukturen der Formel (I) und Silansubstrukturen der Formel (VIIIa) sind am Polymergesamtaufbau zu 0.01 bis 50 mol-% beteiligt. Bevorzugt sind 0.01 bis 30 mol-%, ganz bevorzugt 1 bis 30 mol-%,

speziell 1 bis 10 mol-% und 10 bis 30 mol-%.

Der Einbau der erfindungsgemäßen reaktiven Substrukturen der Formeln (I) und/oder (VIIIa) ermöglicht eine kontrollierte Aktivierung der Polymermoleküle im Anwendungsfall. So ist es möglich, die Uretdionstrukturen thermisch und/oder katalytisch und in Gegenwart funktioneller Gruppen auf Substratoberflächen zu aktivieren. Zusätzlich sind die Dichte der Uretdionstrukturen im Polymermolekül und deren chemische Natur, d.h. beispielsweise deren Abstammung von aliphatischen oder aromatischen Diisocyanaten, Parameter, die zur Steuerung der Aktivierung genutzt werden können. Die erfindungsgemäßen Alkoxysilyl- bzw. Alkoxysiloxan-Strukturen lassen sich bei-spielsweise durch die Zugabe von Wasser, pH-Wert Änderungen, Temperaturer-höhungen und funktionelle Gruppen auf der Substratoberflächen aktivieren. Über die Dichte der Silylgruppierungen und besonders die chemische Konstitution der Alkoxygruppen können der Grad der Vernetzung und die Geschwindigkeit der Aktivierung, beispielsweise durch gezielte Hydrolyse, kontrolliert bzw. gesteuert werden.

Es ist somit ein wesentlicher Vorteil der durch die funktionellen, reaktiven Substrukturen der Formeln (I) und (VIIIa) modifizierten Polymermoleküle, daß sie vor dem zu erwartenden Anwendungsfall entsprechend vorformuliert und gelagert werden können. Erst nach Aktivierung in tatsächlichen Anwendungsfall wird durch Molekulargewichtserhöhung, Vernetzung, Fixierung bzw. Umklammerung des Substrates bzw. Reaktion mit funktionellen Gruppen auf der Substratoberfläche die sehr hohe Substantivität der erfindungsgemäß modifizierten Polymeren voll wirksam.

Die Polymerbildungsreaktionen werden bevorzugt in Wasser, polaren organischen Lösungsmitteln oder Mischungen beider genannter Komponenten ausgeführt. Geeignet sind z.B. Alkohole, speziell Methanol, Ethanol, i-Propanol und n-Butanol, Glykole, wie Ethylenglykol, Diethylenglykol, Triethylenglykol, die Methyl-, Ethyl-und Butylether der genannten Glykole, 1,2-Propylenglykol und 1,3-Propylenglykol, Ketone, wie Aceton und Methylethylketon, Ester, wie Ethylacetat, Butylacetat und 2-Ethylhexylacetat, Ether, wie Tetrahydrofuran und Nitroverbindungen, wie Nitromethan. Die Wahl des Lösungsmittels richtet sich wesentlich nach der Löslichkeit der Reaktionspartner, der angestrebten Reaktionstemperatur und einer gegebenfalls vorhandenen, die Umsetzung störenden Reaktivität.

Die Reaktionen werden im Bereich von 20 °C bis 130 °C, vorzugsweise 40 °C bis 100 °C ausgeführt.

Eine Begrenzung des Molekulargewichtes wird beispielsweise durch die sich bei der Reaktion zwischen Epoxiden und im Reaktionssystem gegebenenfalls vorhandenem Wasser bzw. Alkohol ergebende Endstoppung oder alternativ durch die zusätzliche Verwendung von tertiären Aminen, wie Trialkylaminen, oder die Zugabe monofunktioneller gegenüber Aminogruppen reaktiver Verbindungen bewirkt. Dies bedeutet, daß die Polyorganosiloxanpolymere neben den naturgemäß aus der Umsetzung der monomeren Ausgangsmaterialien resultierenden terminalen Gruppen auch solche aus monofunktionellen Kettenabbruchsmitteln, wie Trialkylaminen etc. und z.B. daraus resultierende Ammonium-, Amino-, Ether- oder Hydroxy-Endgruppen aufweisen.

Die erfindungsgemäßen Amino- und/oder Ammonium-Polysiloxan-Verbindungen erlauben außerdem die Herstellung von Formulierungen, die mindestens eine dieser Verbindung enthalten.

Dabei können auch Formulierungen hergestellt werden, die mindestens ein Lösemittel enthalten. Diese Lösemittel werden ausgewählt aus Wasser und organischen Lösemitteln, wie $C_1$-$C_{22}$-Aliphaten oder $C_6$-$C_8$-Aromaten, bevorzugt $C_1$-$C_{22}$-Alkohole, Estern und/oder Ether.

Die Formulierungen liegen insbesondere in Form einer wässrigen Emulsion vor, bevorzugt in Form einer wässrigen Mikroemulsion. Mikroemulsionen sind Emulsionen, in denen die dispergierte Phase Teilchen mit einer mittleren Größe von 10 bis 250 nm aufweist. Dabei können die erfindungsgemäßen Polymere selbst als Emulgator dienen.

Waschmittelformulierung, enthaltend mindestens eine der erfindungsgemäßen Verbindungen, insbesondere diejenigen mit nichtionogenen und/oder anionischen Tensiden und kosmetische Formulierungen.

Die erfindungsgemäßen Verbindungen oder Formulierungen können wiederum selbst zur Herstellung von weiteren Folgeformulierungen zur Ausrüstung oder Behand-lung von natürlichen oder synthetischen Fasern oder faserartigen Substraten und für kosmetische Anwendung genutzt werden, die für die Behandlung oder Anwendung von natürlichen oder synthetischen Fasern oder faserartigen Substraten einschließlich Papier und in kosmetischen Anwendungen geeignet sind.

Die Erfindung schließt damit auch Verfahren zur Behandlung und/oder Ausrüstung von natürlichen oder synthetischen Fasern oder faserartigen Substraten ein, die die benetzende Behandlung von natürlichen oder synthetischen Fasern oder faserartigern Substrate und gegebenenfalls die Aktivierung mit mindestens einer der erfindungsgemäßen Verbindungen nutzen. Der Begriff Papier schließt Vliese, Pulpen, Schichten oder Beschichtungen mit ein, die anschließend Anwendung in Wisch- oder Taschen- und Reinigungstüchern finden, um die Gebrauchs-eigenschaften, wie Griff, Hydrophilie oder Festigkeit und Steifigkeit zu verbessern.

Diese Verfahren umfassen das Inkontaktbringen, wie Tauchen, Spülen, Besprühen und Transferübertragen (Drucken oder Pressen), Extrudieren oder Kalandrieren.

Die Erfindung umfaßt somit darüber hinaus auch natürliche oder synthetische Fasern oder faserartige Substrate einschließlich Papier, die mit mindestens mit einer der erfindungsgemäßen Verbindungen behandelt wurden und daraus hergestellte Produkte, wie Textilen, Papiere, Vliese sowie beschichtete Formteil mit metallischer, Lack- oder Kunststoffoberflächen. Die Oberflächen werden hydrophiler, benetzbarer oder antistatisch und haben trotzdem einen samtartigen weichen, siliconartigen Griff.

Die erfindungsgemäß durch die Substrukturen (I) und (VIIIa) modifizierten quaternierten Polysiloxancopolymeren können deshalb auch vorteilhaft in kosmetischen Formulierungen für die Haut- und Haarpflege, wie "Rinse-off" Produkten, z.B. 2-in-1 Shampoos, Body Wash und Haarspülungen zur Nachbehandlung von Haaren nach der Reinigung oder dem Färben oder der Vorbehandlung von Haaren vor der Bleichung der Lockung oder Entkräuselung, sowie sogenannten "Leave-in" Produkte wie Haarkuren, Pflegecremes, Frisiercremes, Haargelen, Haarstylingprodukten, Haarfestigern, Haarsprays, Pumpsprays, Fönwellmitteln und Fönfestigern eingesetzt werden. Die erfindungsgemäßen Materialien bewirken eine Verbesserung der Naßkämmkräfte und Trockenkämmkräfte, eine Erhöhung des Haarvolumens und des Glanzes, sowie eine Verringerung des Auswaschens von Farbstoffen von bzw. aus getönten bzw. gefärbten Haaren. Bevorzugt setzt man für kosmetische Anwendungen Polymere mit den Struktureinheiten der Formel (VIIIa) ein.

Die erfindungsgemäß durch die reaktiven Substrukturen der Formel (I) und (VIIIa) modifizierten quaternierten Polysiloxancopolymeren können weiterhin vorteilhaft in Polituren für die Behandlung und Ausrüstung harter Oberflächen, in Formulierungen zum Trocknen von Automobilen und anderen harten Oberflächen nach maschinellen Wäschen, zur Ausrüstung von Textilen und Textilfasern, als separate Weichmacher nach dem Waschen von Textilien mit anionischen/nichtionogen Detergenzienformulierungen, als Weichmacher in auf anionischen/nichtionogenen Tensiden beruhenden Formulierungen zur Textilwäsche, als Bügelhilfe, Mittel zur Verhinderung bzw. Rückgängigmachung von Textilverknitterungen und Mittel zur Papierbehandlung vor und nach der Entwässerung eingesetzt werden. Die erfindungsgemäßen quaternären Copolymere erlauben es in weiten Grenzen das Benetzungsverhalten gegenüber Wasser und Schmutz, die elektrostatischen Eigenschaften und das Reinigungsverhalten zu beinflussen. Bei Fasern erhöhen sie z.B. kosmetische wichtige Eigenschaften wie Glanz, Fülle und Kämmbarkeit oder Weichheit, Steifigkeit und Festigkeit, wobei sie über lange Zeit auf dem Substrat fixiert werden können.

**Beispiele**

Die nachfolgenden Beispiele sollen die Erfindung näher erläutern, ohne sie jedoch einzuschränken.

Beispiel 1

1a) In einem 500ml Dreihalskolben werden bei Raumtemperatur 0,705 g (6,9 mmol) N,N-Dimethyl-1,3-propandiamin in 50ml Isopropanol gelöst. Anschließend werden 1,81 g (16 % NCO Gehalt; 6,9 mmol -NCO) des Isophorondiisocyanat-Dimeren der Struktur

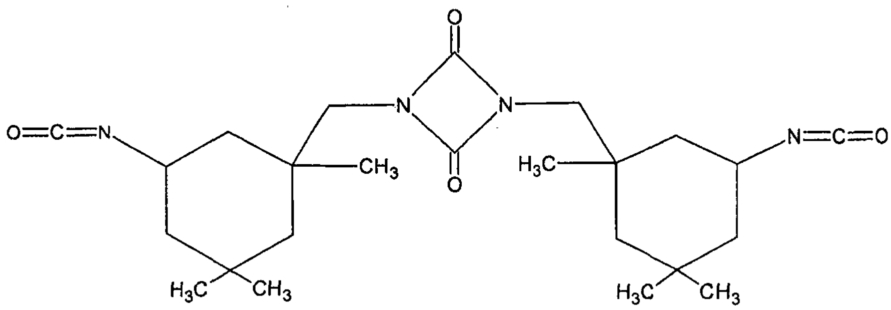

innerhalb weniger Minuten so zugetropft, daß stets eine klare Lösung erhalten bleibt. Nach Beendigung des Zutropfens wird die Lösung wird für 1 Stunde auf 60 °C erhitzt. Es wird ein ditertiäres Amin der Struktur

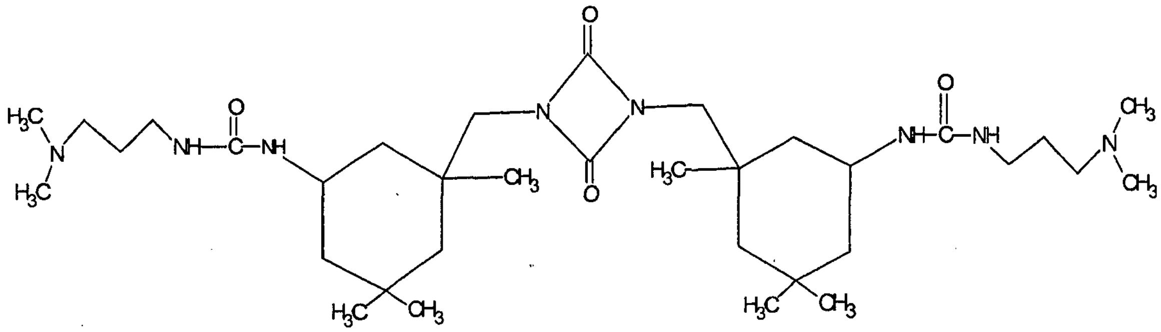

gebildet.

1b) 4 g deionisiertes Wasser, 1,04 g (17,27 mmol) Essigsäure, 3,45 g (17,27 mmol) Dodecansäure, 1,78 g (20,72 mmol tertiäre Aminogruppen) N,N,N',N'-Tetramethyl-1,6-hexandiamin und 2,19 g (6,9 mmol primäre Aminogruppen) eines unter dem Handelsnamen Jeffamin® ED 600 erhältlichen Alkylenoxidderivates der Struktur

$$H_2NCH(CH_3)CH_2[OCH_2CH(CH_3)]_a(OCH_2CH_2)_9[OCH_2CH(CH_3)]_bNH_2$$

mit a+b = 3,6
werden in 50 g Isopropanol gelöst.

1c) 100 g (34,54 mmol Epoxidgruppen) eines Diepoxids der Struktur

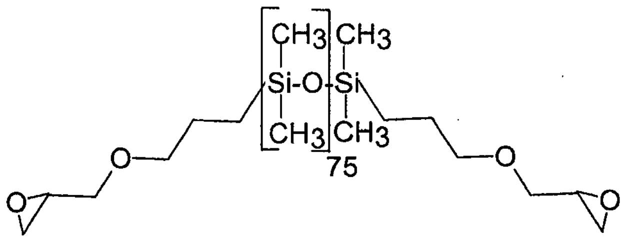

werden in einem Dreihalskolben vorgelegt. Anschließend werden unter Rührung die Lösungen 1a) und 1b) vollständig zugetropft. Nach Beendigung der Zugabe wird der Gesamtansatz für 10 Stunden auf 80-82 °C erhitzt. Es werden 204,6 g einer gelblichtrüben Lösung erhalten (Festkörpergehalt 52,5 %), die ein Polymer, das u.a. die mit den Struktureinheiten

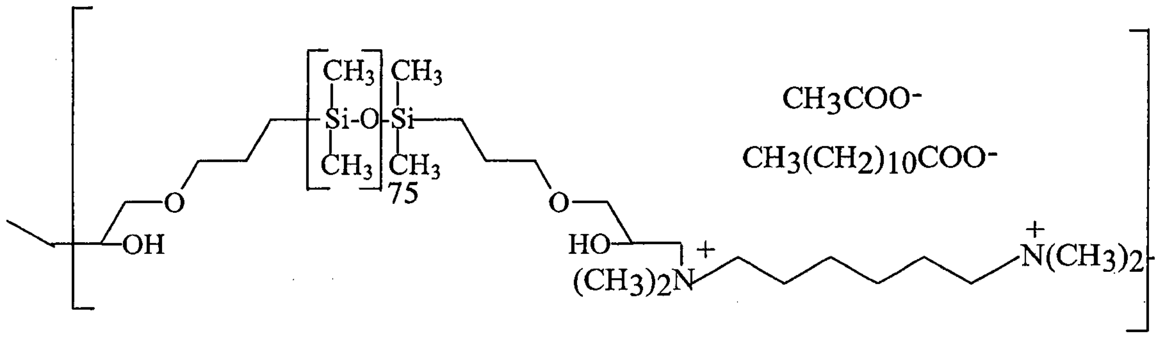

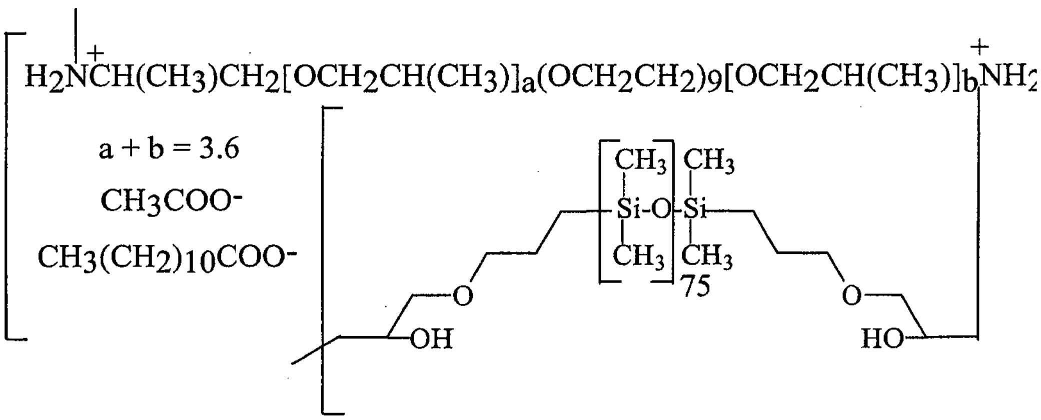

$CH_3COO^-$ $CH_3(CH_2)_{10}COO^-$

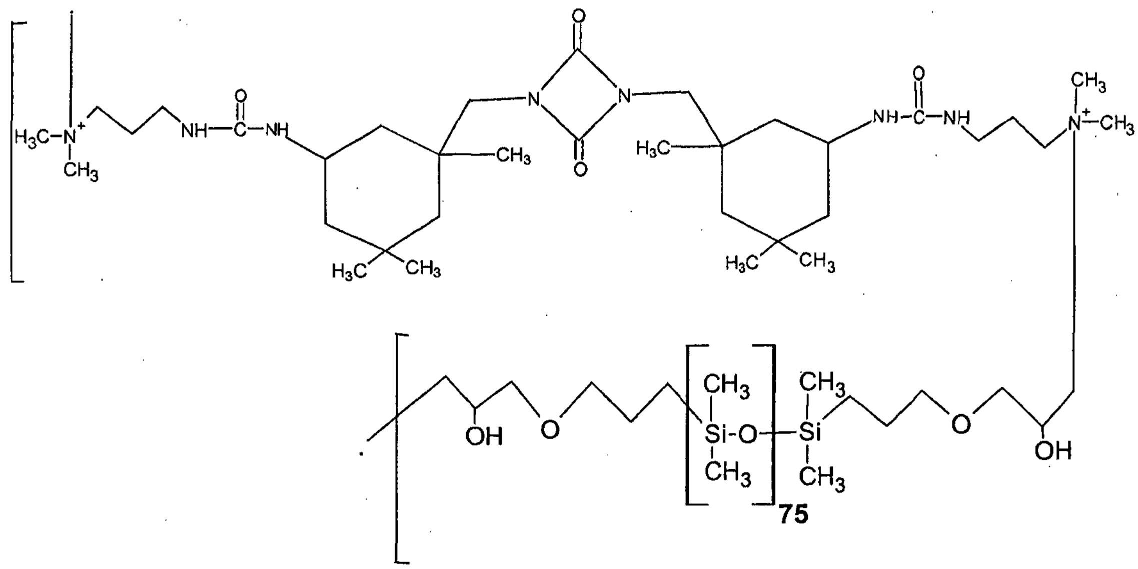

enthält.

Beispiel 2

2a) 4 g deionisiertes Wasser, 1,04 g (17,27 mmol) Essigsäure, 3,45 g (17,27 mmol) Dodecansäure, 2,38 g (27,64 mmol tertiäre Aminogruppen) N,N,N',N'-Tetramethyl-1,6-hexandiamin und 1,09 g (3,46 mmol primäre Aminogruppen) eines unter dem Handelsnamen Jeffamin® ED 600 erhältlichen Alkylenoxidderivates der Struktur

$H_2NCH(CH_3)CH_2[OCH_2CH(CH_3)]_a(OCH_2CH_2)_9[OCH_2CH(CH_3)]_bNH_2$

mit a+b = 3,6
werden in 50ml Isopropanol gelöst.

2b) 100 g (34,54mmol Epoxidgruppen) eines Diepoxids der Struktur

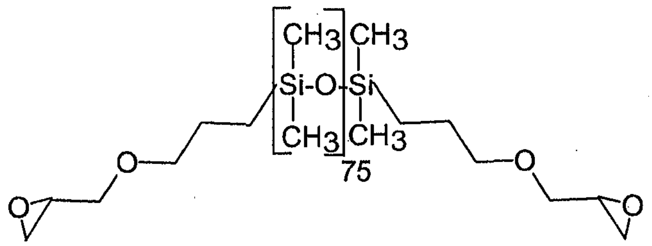

werden in einem 500 ml Dreihalskolben in 100 ml Isopropanol gelöst. 1,61 g (59,5 % Aktivgehalt, 3,45 mmol-$NHCH_3$ Gruppen) einer isopropanolischen Lösung eines Aminosilans der Struktur

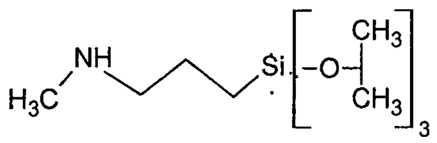

werden zugetropft und der Ansatz anschließend für 8 Stunden auf 80 °C erhitzt. Anschließend wird die Lösung 2a) vollständig zugetropft und der Gesamtansatz für 10 Stunden auf 82-84 °C erhitzt. Es werden 204,7 g einer klaren Lösung (Festkörpergehalt 51,3 %) erhalten, die ein Polymer mit den Struktureinheiten

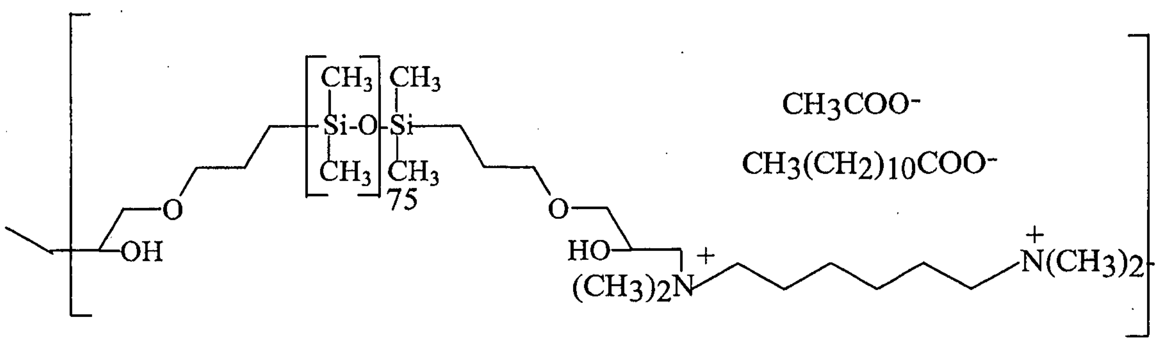

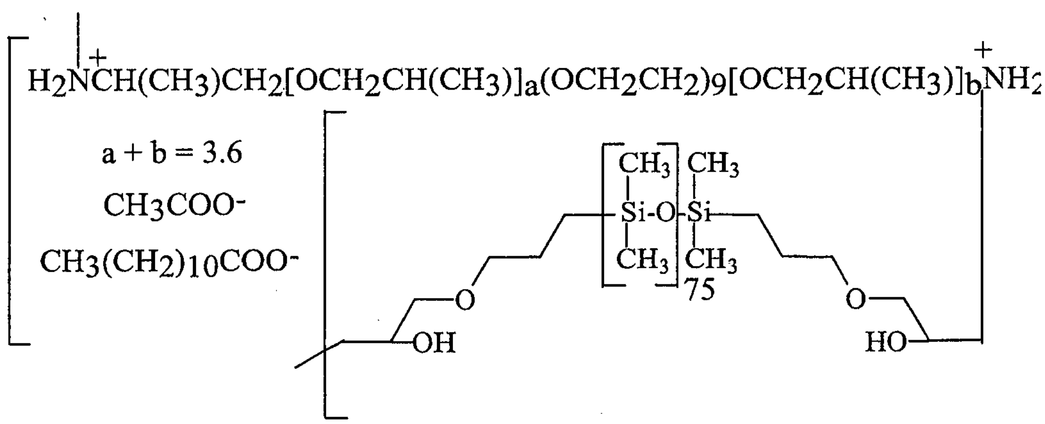

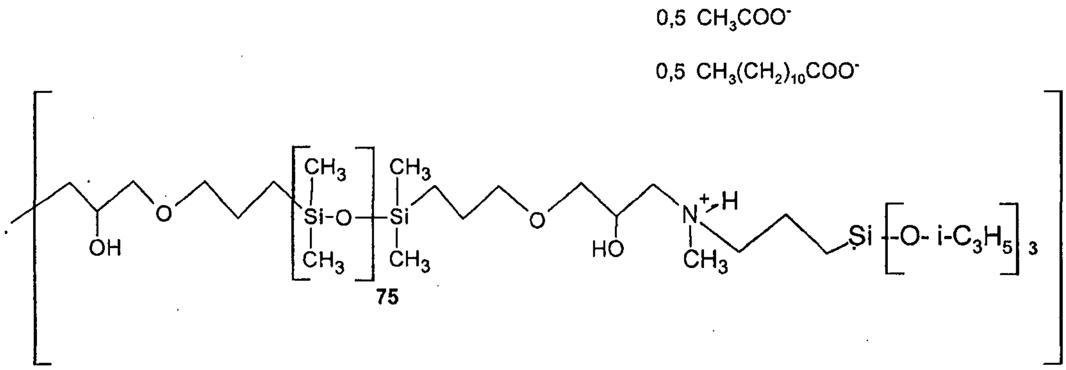

enthält.

Beispiel 3

In Analogie zu Beispiel 1 und 2 wird ein Polymer mit 82,7 % Aktivgehalt in Lösung synthetisiert, welches die erfindungsgemäßen Reaktivgruppen (I) und (VIIIa) nicht enthält und u.a. folgende Strukturelemente aufweist.

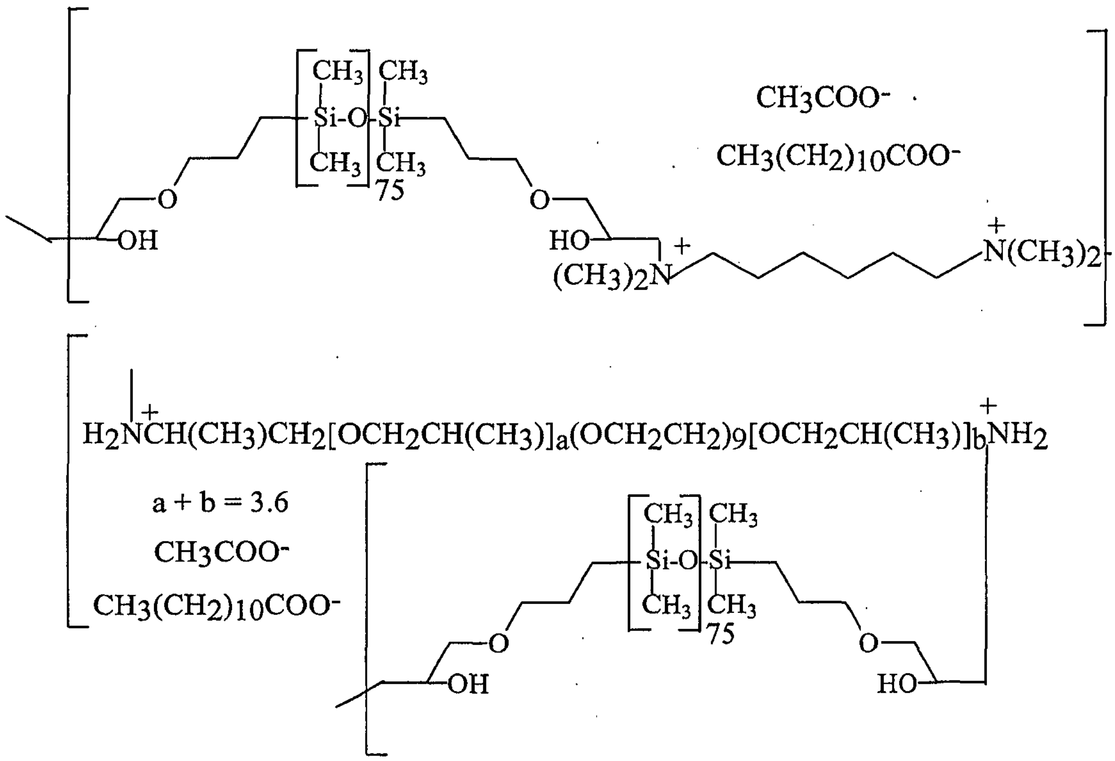

enthält.

Beispiel 4

4a) In einem 500 ml Dreihalskolben werden bei Raumtemperatur 1,76 g (17,26 mmol) N,N-Dimethyl-1,3-propandiamin in 50 ml Isopropanol gelöst. Anschließend werden 4,53 g (16 % -NCO Gehalt; 17,26 mmol -NCO) des Isophorondiisocyanat-Dimeren der Struktur

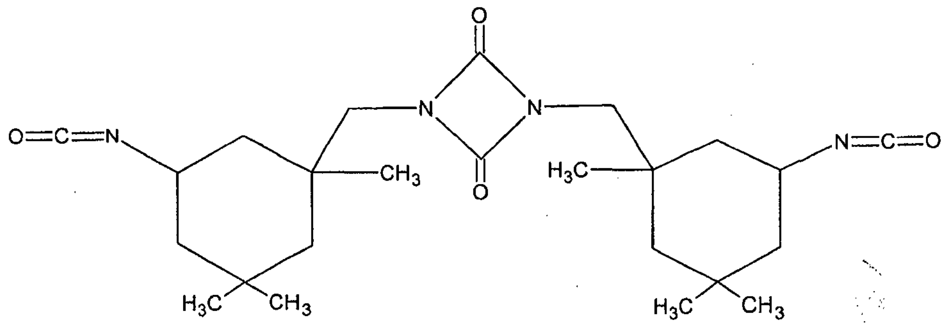

innerhalb weniger Minuten so zugetropft, daß stets eine klare Lösung erhalten bleibt. Nach Beendigung des Zutropfens wird die Lösung wird für 1 Stunde auf 60 °C erhitzt. Es wird ein ditertiäres Amin der Struktur

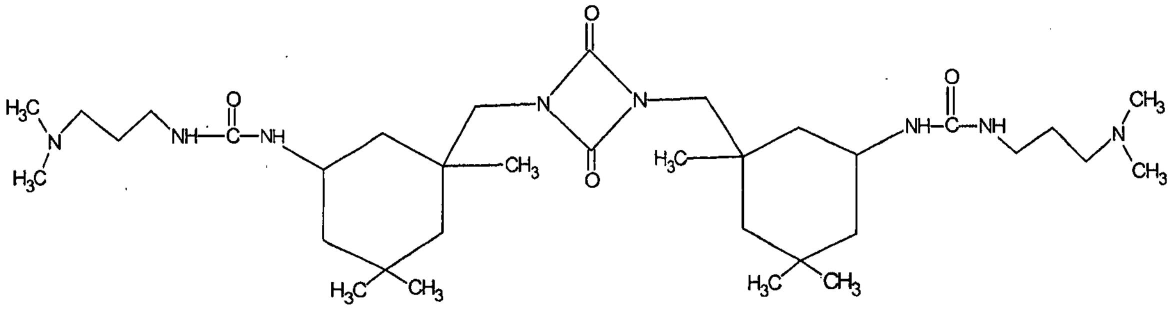

gebildet.

4b) 6 g deionisiertes Wasser und 3,45 g (17,26 mmol) Dodecansäure werden zur Lösung 1a) gegeben und die Mischung 5 Minuten gerührt.

4c) 50 g (17,26 mmol Epoxidgruppen) eines Diepoxids der Struktur

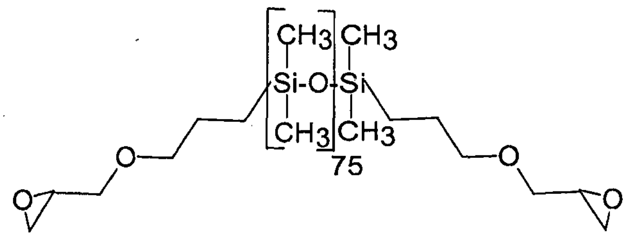

werden in einem Dreihalskolben vorgelegt. Anschließend werden unter Rührung die Mischung 1b) vollständig zugetropft. Nach Beendigung der Zugabe wird der Gesamtansatz für 11 Stunden auf 80-82 °C erhitzt. Nach 6 Stunden Reaktionszeit wird die anfangs trübe Lösung klar. Es werden 99 g einer hellgelben, klaren Lösung erhalten (Festkörpergehalt 56,5 %), die ein Polymer u.a mit den Struktureinheiten

$CH_3COO^-$ $CH_3(CH_2)_{10}COO^-$

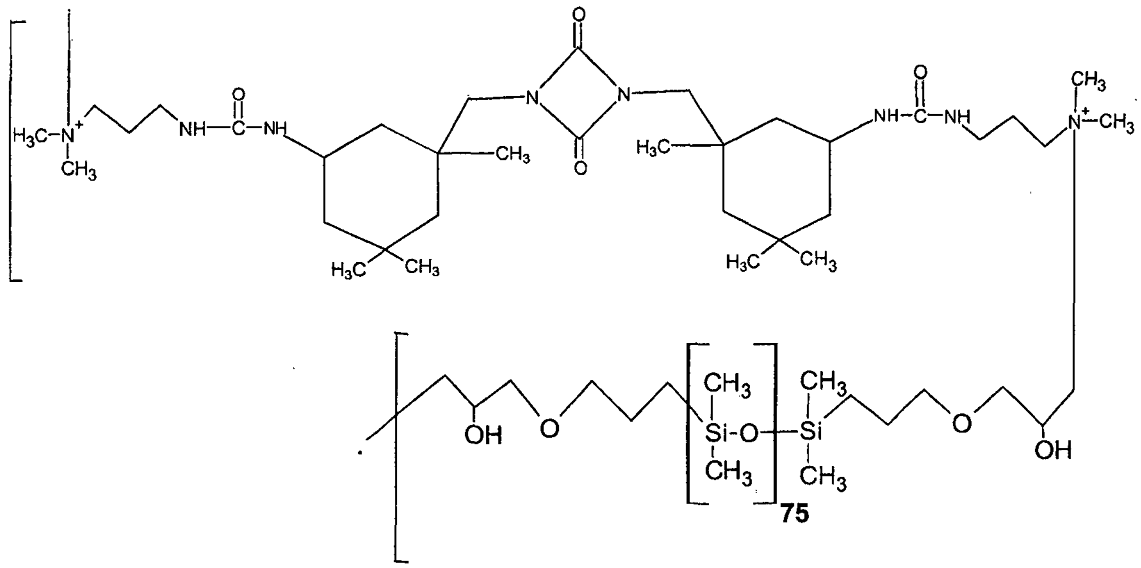

enthält.

Beispiel 5

Ausgehend von den quaternären Polysiloxanen gemäß Beispielen 1 bis 3 wurden drei Microemulsionskonzentrate folgender Zusammensetzung hergestellt:

| **Formulierung 1 (F1) Uretdion erfindungsgemäß** | **Formulierung 2 (F2) Silan erfindungsgemäß** | **Formulierung 3 (F3) nicht erfindungsgemäß** |
|---|---|---|
| 61,9 g Quat Bsp. 1 | 54,4 g Quat Bsp. 2 | 52,0 g Quat Bsp. 3 |
| 11,6 g Renex® 36 | 10,0 g Renex® 36 | 15,48 g Renex® 36 |
| 3,3 g Renex® 30 | 2,9 g Renex® 30 | 4,45 g Renex® 30 |
| 5,4 g Crodet® S40 | 4,7 g Crodet® S40 | 7,25 g Crodet® S40 |
| 0,75g Essigsäure | 0,64 g Essigsäure | 1,0 g Essigsäure |
| 0,56 g Natriumacetat | 0,48 g Natriumacetat | 0,75 g Natriumacetat |
| 20,6 g dest. Wasser | 17,7 g dest. Wasser | 19,07g dest. Wasser |

Renex® 36 Handelsname der ICI Surfactants; Tridecylalkohol-$EO_{12}$-OH
Renex® 30 Handelsname der ICI Surfactants; Tridecylalkohol-$EO_6$-OH
Crodet® S40 Handelsname der Croda GmbH; Stearinsäure-$EO_{40}$-OH

Diese drei etwa 40 %-igen Microemulsionskonzentrate wurden mit Wasser einheitlich auf jeweils 11 % Siliconquatgehalt verdünnt. Von diesen 11 %-igen transparenten Microemulsionen wurden jeweils 6g (absolute Siliconquatmenge 0.66g) abgenommen, mit 6000 ml Wasser und gegebenenfalls Zusätzen intensiv vermischt und unter folgenden Randbedingungen zur Textilausrüstung nach dem Jet-Verfahren benutzt:

Jettyp: Mathis Labor-Jumbo-Jet
Jetpumpe: Stufe 6 (höchstmögliche Scherung)
Wassermenge im Jet: 6000 ml
Ausrüstung: 20 Minuten bei 40°C
Textil: 300g gebleichtes und mit optischem Aufheller behandeltes Baumwoll-Jersey

Die mit den Formulierungen F1, F2 bzw. F3 ausgerüsteten Baumwoll-Jersey-Streifen wurden 15 Minuten bei 100 °C im Umlufttrockenschrank getrocknet.

Nachfolgend wurden die Baumwoll-Jersey-Streifen geteilt und einzelne Stücke den in der folgenden Tabelle aufgeführten zusätzlichen Temperungen unterworfen.

| keine zusätzliche Temperung | 45 sek./ 120 °C | 120 sek./ 120 °C | 45 sek./ 150 °C | 120sek./ 150 °C |
|---|---|---|---|---|
| F1-1 | F1-2 | F1-3 | F1-4 | F1-5 |
| F2-1 | F2-2 | F2-3 | F2-4 | F2-5 |
| F3-1 | F3-2 | F3-3 | F3-4 | F3-5 |

Die einzelnen Baumwoll-Jersey-Stücken wurden dann 5x für 20 Minuten bei 40 °C mit einem silicatfreien Feinwaschmittel gewaschen (1,7 g Waschmittel/ Liter Waschflotte).

Anschließend ermittelten 5 Testpersonen unabhängig voneinander die Reihung der Baumwoll-Jersey-Stücken hinsichtlich Weichheit.

Innerhalb der drei Gruppen F1-1 bis F1-5, F2-1 bis F2-5 und F3-1 bis F3-5 wurden zunächst als weicheste Jersey-Streifen F1-5, F2-1 und F3-4 ermittelt.

Diese selektierten drei Streifen wurden von den 5 Testpersonen in einem direkten Vergleich einer Bewertung unterzogen, wobei das weicheste Textilstück die Note 1 und das härteste Textilstück die Note 3 erhielt.

Parallel hierzu wird als Maß für die Hydrophilie die Tropfen-Einsink-Zeit ermittelt. In der nachfolgenden Tabelle sind die Ergebnisse zusammengefaßt.

| Textilstück | Ø Note Weichheit (5 Testpersonen) | Tropfen-Einsink-Zeit (Sekunden) |
|---|---|---|
| F1-5 | 1.2 | 2 |
| F2-1 | 1.8 | 2 |
| F3-4 | 3.0 | 1 |
| *Tropfen-Einsink-Zeit | | |

Die Daten zu den Weichheiten der Textilstücken F1-5 und F2-1 nach 5 Waschzyklen belegen, daß der Einbau erfindungsgemäßer Strukturelemente die Permanenz der Textilausrüstung und damit die Weichheit weiter verbessert. Zusätzlich kann der stark hydrophile Charakter der Ausrüstung aufrecht erhalten werden.

## Patentansprüche

1. Amino- und/oder Ammonium-Polysiloxanverbindungen und Salze davon, **dadurch gekennzeichnet, dass** sie mindestens eine funktionelle Gruppe, ausgewählt aus Gruppen der Formeln (I) und (VIIIa) aufweisen:

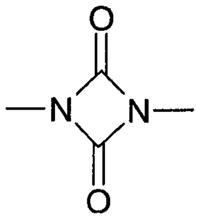

(I),

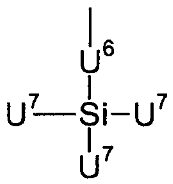

(VIIIa),

worin

$U^6$ einen zweiwertigen geradkettigen, cyclischen oder verzweigten, gesättigten, ungesättigten oder aromatischen Kohlenwasserstoffrest mit bis zu 100 Kohlenstoffatomen darstellt, der gegebenenfalls eine oder mehrere Gruppen, ausgewählt aus -O-, -C(O)-, -NH- und -$NU^8$- enthalten kann, oder gegebenenfalls durch eine oder mehrere Hydroxylgruppen substituiert sein kann, worin

$U^8$ Wasserstoff oder einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen -O- enthalten und durch OH substituiert sein kann, mit der Maßgabe, dass -NH- und -$NU^8$- an ein Carbonyl- und/oder Thiocarbonylkohlen-stoffatom gebunden ist, und

$U^7$ einen einwertigen, geradkettigen, cyclischen oder verzweigten, gesättigten, ungesättigten oder aromatischen Kohlenwasserstoffrest mit bis zu 20 Kohlenstoffatomen darstellt, der eine oder mehrere Gruppen -O- enthalten und durch OH substituiert sein kann,

mit der Maßgabe, dass die Reste $U^7$ gleich oder verschieden sein können und mindestens ein Rest $U^7$ pro Siliciumatom über -O- an das Siliciumatom gebunden ist,

worin die Gruppe der Formel (VIIIa) als Rest $R^1$ der Einheiten Q, wie im folgenden definiert, enthalten ist,

und dass es sich um Verbindungen handelt, die mindestens drei Einheiten ausgewählt aus den Einheiten Q und V aufweisen,

worin Q mindestens eine zwei-, drei- und/oder vierwertige Amino- und/oder Ammoniumgruppe ist, die nicht über ein Carbonylkohlenstoffatom an V gebunden ist, die ausgewählt wird aus der Gruppe, die besteht aus:

$-NR^1-$,

$-N^+R^1{}_2-$,

einem gesättigten oder ungesättigten, gegebenenfalls mit weiteren Substituenten substituierten diaminofunktionellen Heterocyclus der Formeln:

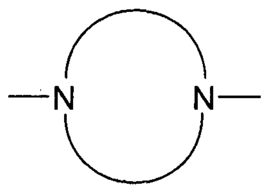

,

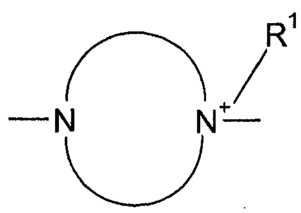

und

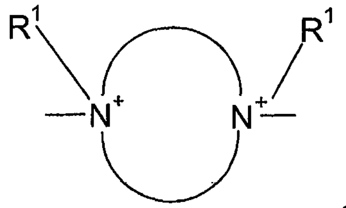

,

sowie

einem aromatischen, gegebenenfalls substituierten diaminofunktionellen Heterocyclus der Formel:

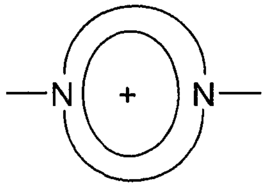

einem dreiwertigen Rest der Formel:

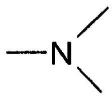

,

einem dreiwertigen Rest der Formel:

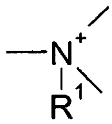

,

oder
einem vierwertigen Rest der Formel,

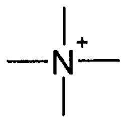

worin $R^1$ jeweils Wasserstoff oder einen einwertigen organischen Rest darstellt, wobei Q nicht an ein Carbonylkohlenstoffatpm bindet.
und
V mindestens eine organische Einheit ist, die über Kohlenstoff mit den Q-Einheiten verbunden ist,
mit der Maßgabe, dass mindestens eine der Einheiten V einen Polyorganosiloxanrest enthält, und worin
die Einheit V ausgewählt wird aus mindestens einem mehrwertigen, geradkettigen, cyclischen oder verzweigten, gesättigten, ungesättigten oder aromatischen Kohlenwasserstoffrest mit bis zu 1000 Kohlenstoffatomen wobei die Kohlenstoffatome des gegebenenfalls vorhandenen Polyorganosiloxanrestes nicht mitgezählt werden, der gegebenenfalls eine oder mehrere Gruppen, ausgewählt aus

-O-, -C(O)-, -C(S)-,

$-NR^2-$, worin $R^2$ Wasserstoff, einen einwertigen, geradkettigen, cyclischen oder verzweigten, gesättigten, ungesättigten oder aromatischen Kohlenwasserstoffrest mit bis zu 300 Kohlenstoffatomen darstellt, der eine oder mehrere Gruppen ausgewählt aus -O-, -NH-, -C(O)- und -C(S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Polyetherresten, Polyetheresterresten, Polyorganosiloxanylresten und

$-Si(OR)_{3-a}(R')_a$,

worin a eine ganze Zahl von 0 bis 2 ist und R und R' gleich oder verschieden voneinander sein können und jeweils einen organischen Rest darstellen,
wobei wenn mehrere Gruppen $-NR^2-$ vorliegen, diese gleich oder verschieden sein können, und mit der Maßgabe, dass die Gruppe $-NR^2-$ an ein Carbonyl-und/oder Thiocarbonylkohlenstoffatom bindet,

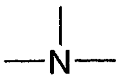

und Polyorganosiloxanresten enthalten kann, und der gegebenenfalls durch eine oder mehrere Hydroxyl-gruppen und/oder Gruppen der Formel (II)

$$-Si(OR)_{3-a}(R')_a$$

worin a, R und R' wie oben definiert sind, substituiert sein kann, und worin die terminale Absättigung der miteinander verbundenen mehrwertigen Gruppen Q und V über einwertige organische Reste erfolgt.

2. Verbindung nach Anspruch 1, **dadurch gekennzeichnet, dass** sie mindestens eine funktionelle Gruppe der Formel (I) aufweist:

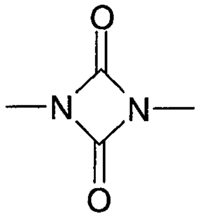

(I).

3. Verbindung nach Anspruch 1, die mindestens zwei Einheiten V, die einen Polyorganosiloxanrest enthalten, aufweist.

4. Verbindung nach Anspruch 1, **dadurch gekennzeichnet, dass** sie mindestens zwei Einheiten Q aufweist.

5. Verbindung nach Anspruch 1, **dadurch gekennzeichnet, dass** sie mindestens eine quaternäre Ammoniumgruppe aufweist.

6. Verbindung nach Anspruch 1, **dadurch gekennzeichnet, dass** sie mindestens zwei quaternäre Ammoniumgruppen aufweist.

7. Verbindung nach Anspruch 1, **dadurch gekennzeichnet, dass** der Polyorganosiloxanrest eine zweiwertige Gruppe der Formel (III)

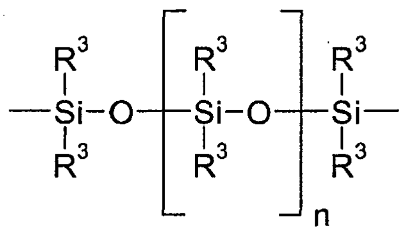

(III)

ist, worin $R^3$ gleich oder verschieden sein kann und aus der Gruppe ausgewählt wird, die aus $C_1$ bis $C_{22}$-Alkyl, Fluor($C_3$-$C_{10}$)-alkyl, $C_6$-$C_{10}$-Aryl und $-W-Si(OR)_{3-a}(R')_a$ besteht, worin R, R' und a wie oben definiert sind und W -O- oder einen zweiwertigen, geradkettigen, cyclischen oder verzweigten, gesättig-ten, ungesättigten oder aromatischen Kohlenwasserstoffrest mit bis zu 100 Koh-lenstoffatomen darstellt, der eine oder mehrere Gruppen -C(O)-, -O-, NH-, -S- enthalten kann, und gegebenenfalls durch Hydroxy substituiert sein kann, und n = 0 bis 1000 bedeutet.

8. Verbindung nach einem der Ansprüche 1 bis 7, **dadurch gekennzeichnet, dass** sie mindestens eine Einheit der Formel (IV) enthalten:

-[Q-V]- (IV)

worin Q und V wie oben definiert sind, und die terminale Absättigung der Gruppen Q und V durch einwertige organische Gruppen erfolgt.

**9.** Verbindung nach Anspruch 8, **dadurch gekennzeichnet, dass** sie mindestens zwei Wiederholungseinheiten der Formel (IV) aufweist.

**10.** Verbindung nach einem der Ansprüche 1 bis 9, **dadurch gekennzeichnet, dass** sie mindestens eine funktionelle Gruppe (I) der Formel (Ia)

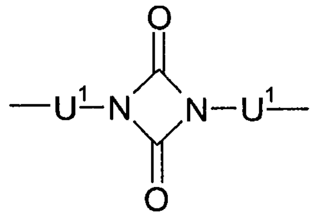

(Ia)

enthält, worin
$U^1$ ausgewählt wird aus der Gruppe, die besteht aus zweiwertigen Resten der Formeln:

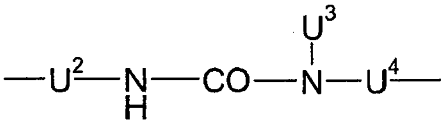

(Ib),

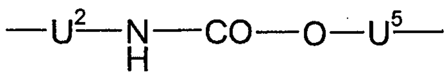

(Ic)

und

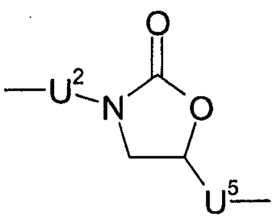

(Id),

wobei
$U^2$ mit dem Stickstoffatom der funktionellen Gruppe der Formel (I) verbunden ist, und
$U^2$ einen zweiwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen -O- enthalten kann,
$U^3$ Wasserstoff oder einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen -O- enthalten und durch OH substituiert sein kann, aus $-W-Si(OR)_{3-a}(R')_a$ bestehen, worin R, R' wie oben definiert und a = 0 bis 2 sind und W einen zweiwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen

-C(O) -, -O-, NH-, -S- enthalten kann, und gegebenenfalls durch Hydroxygruppen substituiert sein kann.
$U^4$ und $U^5$ zweiwertige geradkettige, cyclische oder verzweigte, gesättigte, ungesättigte oder aromatische Kohlenwasserstoffreste mit bis zu 1000 Kohlenstoffatomen darstellen, die gegebenenfalls eine oder mehrere Gruppen, ausgewählt aus -O-, -C(O)-,

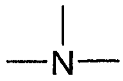,

$-NR^2-$, worin $R^2$ wie oben definiert ist, enthalten können und die gegebenenfalls durch eine oder mehrere Hydroxylgruppen substituiert sein können, mit der Maßgabe, dass die Gruppen

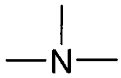

und $-NR^2-$ an ein Carbonylkohlenstoffatom binden.

**11.** Verfahren zur Herstellung der Amino- und/oder Ammonium-Polysiloxanverbindung nach einem der Ansprüche 1 bis 10, worin

a) mindestens eine Aminverbindung, ausgewählt aus einer Diamin-Verbindung und/oder einer primären oder sekundären Monoaminverbindung, mit einer zur Reaktion mit den Aminofunktionen der Amin-Verbindung befähigten multi-, bevorzugt difunktionellen organischen Verbindungen umgesetzt werden, wobei das molare Verhältnis der Aminofunktionen der genannten Aminverbindung zu den funktionellen Gruppen der genannten multi-, bevorzugt difunktionellen organischen Verbindung von etwa 0,5 bis 2 beträgt, oder
b) mindestens zwei Mol einer Aminverbindung (1), ausgewählt aus einer Diamin-Verbindung (1) und/oder einer primären oder sekundären Monoaminverbindung (1), mit einem Mol einer multi-, bevorzugt difunktionellen, zur Reaktion mit den Aminofunktionen der Aminverbindung befähigten organischen Verbindung (1), unter Bildung einer Diaminverbindung (2) (Monomer) umgesetzt werden, und die Diaminverbindung (2) (Monomer) anschließend mit mindestens einer weiteren multi-, bevorzugt difunktionellen, zur Reaktion mit den Aminofunktionen der Diaminverbindung (2) befähigten organischen Verbindung (2), gegebenenfalls in Anwesenheit weiterer Aminverbindungen (2) umgesetzt wird, wobei die Stöchiometrie der Aminofunktionen und der zur Reaktion mit Aminofunktionen befähigten funktionellen Gruppen in der letzten Stufe der Umsetzung etwa 1:1 beträgt, und die organischen Verbindungen (1) und (2) gleich oder verschieden voneinander sein können, oder
c) eine Aminverbindung, ausgewählt aus einer Diamin-Verbindung (1) und /oder einer primären oder sekundären Monoaminverbindung, mit einer multi-, bevorzugt difunktionellen, zur Reaktion mit den Aminofunktionen der Aminverbindungen befähigten organischen Verbindung (1) unter Bildung einer Diaminverbindung (2) (aminoferminiertes Oligomer) umgesetzt wird, wobei das molare Verhältnis der Aminofunktionen der genannten Aminverbindung zu den funktionellen Gruppen der genannten multi-, bevorzugt difunktionellen organischen Verbindung (1) etwa 1 bis 2 beträgt, anschließend die erhaltene Diaminverbindung (2) (aminoterminiertes Oligomer) mit mindestens einer multi-, bevorzugt difunktionellen zur Reaktion mit den Aminofunktionen der Diamin-Verbindungen befähigten organischen Verbindung (2) umgesetzt wird, wobei die Stöchiometrie der Aminofunktionen und der zur Reaktion mit Aminofunktionen befähigten funk-tionellen Gruppen in der letzten Stufe der Umsetzung etwa 1:1 beträgt, und die organischen Verbindungen (1) und (2) gleich oder verschieden sein können, oder
d) eine Aminverbindung (1), ausgewählt aus einer Diamin-Verbindung und /oder einer primären oder sekundären Monoaminverbindung, mit einer multi-, bevorzugt difunktionellen, zur Reaktion mit den Aminofunktionen der Aminverbindung befähigten organischen Verbindung (1) unter Bildung einer multi-, bevorzugt difunktionellen, zur Reaktion mit Aminofunktionen befähigten organischen Verbindung (2) (difunktionelles Oligomer) umgesetzt wird, wobei das molare Verhältnis der Aminofunktionen der genannten Aminverbindung zu den funktionellen Gruppen der genannten multi-, bevorzugt difunktionellen organischen Verbindung (1) etwa 0,5 bis 1 beträgt, anschließend die organische Verbindung (2) (difunktionelles Oligomer) mit mindestens einer Aminverbindung (2), ausgewählt aus einer Diamin-Verbindung und/oder einer primären oder sekundären Monoaminverbindung, gegebenenfalls in Anwesenheit einer oder mehrerer multi-, bevorzugt difunktionellen, zur Reaktion mit Aminofunktionen befähigten organischen Verbindung (3) umgesetzt wird, wobei die Stöchiometrie der Aminofunktionen und der zur Reaktion mit Aminofunktionen befähigten funktionellen Gruppen in der letzten Stufe der Umsetzung etwa 1:1 beträgt,

wobei gegebenenfalls monofunktionelle, bevorzugt tertiäre Monoamine oder geeignete, zur Kettenfortpflanzung nicht befähigte Monoamine und/oder mono-funktionelle, zur Reaktion mit Aminofunktionen befähigte Verbindungen als Kettenabbruchsmittel hinzugesetzt werden können, und wobei gegebenenfalls vorhandene Aminofunktionen in den erhaltenen Produkten anschließend protoniert oder quaterniert werden können.

**12.** Verfahren nach Anspruch 11, worin die Einführung der funktionellen Gruppe der Formel (I) umfasst:

a) Die Umsetzung von Diisocyanaten, enthaltend die funktionelle Gruppe der Formel (I), mit mindestens einem Mol eines Diamins (1) unter Bildung eines monomeren, oligomeren oder polymeren Diamins (2), dass die funktionelle Gruppe der Formel (I) enthält, oder
b) die Umsetzung von einem Mol eines Diisocyanates, enthaltend die funktionelle Gruppe der Formel (I), mit mindestens einem Mol einer multi-, bevorzugt difunktionellen, zur Reaktion mit den Isocyanatgruppen und Aminogruppen befähigten organischen Verbindungen (1), unter Bildung einer multi-, bevorzugt difunktionellen, zur Reaktion mit Aminogruppen befähigten monomeren, oligomeren oder polymeren organischen Verbindung (2), enthaltend die Gruppe der Formel (I), oder
c) die Umsetzung von einem Mol eines Diisocyanates, enthaltend die funktionelle Gruppe der Formel (I), mit mindestens einem Mol einer multi-, bevorzugt difunktionellen, zur Reaktion mit den Isocyanatgruppen befähigten organischen Verbindungen (1) unter Bildung einer multi-, bevorzugt difunktionellen, organischen monomeren, oligomeren oder polymeren Verbindung (2), enthaltend die funktionelle Gruppe der Formel (I) und terminale zur Reaktion mit Isocyanatgruppen befähigte Gruppen, Überführung der genannten organischen Verbindung (2) in eine multi-, bevorzugt difunktionellen, zur Reaktion mit Aminogruppen befähigte monomere, oligomere oder polymere organischen Verbindung (3)

und Verwendung der erhaltenen, die Gruppe der Formel (I) enthaltenden Verbindungen in den Verfahren a) bis d) des Anspruchs 22.

**13.** Verfahren nach Anspruch 11 oder 12, worin die Einführung der funktionellen Gruppe der Formel (VIIIa) die Umsetzung einer Aminverbindung, ausgewählt aus einer Diamin-Verbindung und/oder einer primären, sekundären oder tertiären Monoaminverbindung, enthaltend die Einheit der Formel (VIIIa), und/oder die Umsetzung einer multi-, bevorzugt difunktionellen organischen Verbindung, enthaltend die Einheit der Formel (VIIIa) umfasst.

**14.** Verfahren nach einem der Ansprüche 11 bis 13, worin die funktionellen Gruppen der multi-, bevorzugt difunktionellen, zur Reaktion mit Aminofunktionen befähigten Verbindungen ausgewählt werden aus der Gruppe, die besteht aus Epoxygruppen und Halogenalkylgruppen.

**15.** Formulierungen, enthaltend mindestens eine Verbindung nach einem der Ansprüche 1 bis 10.

**16.** Formulierung nach Anspruch 15, enthaltend mindestens ein Lösungsmittel, ausgewählt aus Wasser und organischen Lösungsmitteln.

**17.** Formulierung nach Anspruch 15 oder 16 in Form einer wässrigen Emulsion.

**18.** Formulierung nach Anspruch 17 in Form einer wässrigen Mikroemulsion.

**19.** Waschmittelformulierung, enthaltend mindestens eine Verbindung nach einem der Ansprüche 1 bis 10.

**20.** Waschmittelformulierung nach Anspruch 19, enthaltend nichtionogene und/oder anionischen Tenside.

**21.** Kosmetische Formulierung enthaltend mindestens eine Verbindung nach einem der Ansprüche 1 bis 10.

**22.** Verwendung der Verbindungen nach einem der Ansprüche 1 bis 10 zur Herstellung von Formulierungen zur Ausrüstung oder Behandlung von natürlichen oder synthetischen Fasern oder faserartigen Substraten, in kosmetischen Anwendungen.

**23.** Verfahren zur Behandlung und/oder Ausrüstung von natürlichen oder synthetischen Fasern oder faserartigen Substraten, dass die benetzende Behandlung von natürlichen oder synthetischen Fasern oder faserartigern Substrate

und gegebenenfalls Aktivierung mit mindestens einer der Verbindungen nach einem der Ansprüche 1 bis 10 oder mit umdestens einer der Formulierungen gemäß Anspruch 16 bis 18 umfasst.

**24.** Natürliche oder synthetische Fasern oder faserartige Substrate, behandelt mit mindestens einer der Verbindungen nach einem der Ansprüche 1 bis 10.

**25.** Produkte hergestellt aus den Fasern oder faserartigen Substraten des Anspruchs 24.

**26.** Verwendung der Verbindungen nach einem der Ansprüche 1 bis 10 und Formulierungen gemäß Anspruch 16-18 in kosmetischen Formulierungen, in Waschmitteln oder zur Oberflächenbehandlung von Substraten.

## Claims

**1.** Amino and/or ammonium polysiloxane compounds and salts thereof, **characterized in that** they have at least one functional group, selected from group of the formulas (I) and (VIIIa)

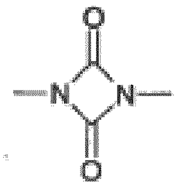

(I),

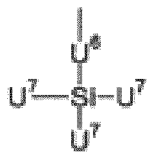

(VIIIa),

wherein
$U^6$ is a divalent linear, cyclic or branched, saturated, unsaturated or aromatic hydrocarbon radical having up to 100 carbon atoms, which optionally contains one or more groups, selected from -O-, -C(O)-, -NH- and -$NU^8$- or which is optionally substituted by one or more hydroxyl groups, wherein
$U^8$ is hydrogen or a univalent, linear, cyclic or branched, saturated, unsaturated or aromatic hydrocarbon radical having up to 100 carbon atoms, which may contain one or more -O- groups and may be substituted by OH, with the proviso that -NH- and - $NU^8$- is bonded to a carbonyl and/or thiocarbonyl carbon atom, and
$U^7$ is a univalent, linear, cyclic or branched, saturated, unsaturated or aromatic hydrocarbon radical having up to 20 carbon atoms, which may contain one or more -O-groups and may be substituted by OH,
with the proviso that the radicals $U^7$ may be the same or different from each other and at least one radical $U^7$ per silicon atom is bonded via -O- to the silicon atom,
wherein the group of the formula (VIIIa) is contained in the units Q as radical $R^1$ as defined below,
and that it concerns compounds having at least three units selected from the units Q and V,
wherein Q is at least a di-, tri- and/or tetravalent amino and/or ammonium group, which is not bonded via a carbonyl carbon atom to V, which is selected from the group consisting of:

-$NR^1$-,

$N^+R^1_2$-,

a saturated or unsaturated, optionally substituted with further substituents, diamino functional heterocycle of the formulas:

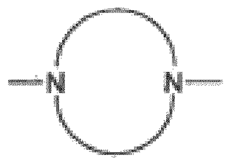

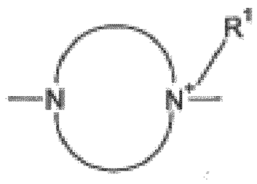

and

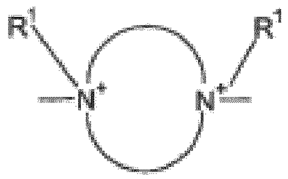

,

as well as
an aromatic, optionally substituted diamino functional heterocycle of the formula:

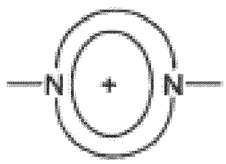

a trivalent radical of the formula:

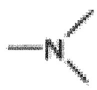

,

a trivalent radical of the formula:

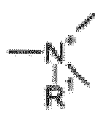

,

or
a tetravalent radical of the formula:

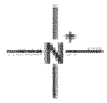

wherein $R^1$ each is hydrogen or a univalent organic radical, wherein Q is not bonded to a carbonyl carbon atom, and

V is at least an organic unit, which is bonded to the Q units via a carbon atom,

with the proviso that at least one of the units V contains a polyorganosiloxane radical and wherein the unit V is selected from at least one multivalent, linear, cyclic or branched, saturated, unsaturated or aromatic hydrocarbon radical having up to 1000 carbon atoms, wherein the carbon atoms of the optionally present polyorganosiloxane radical are not included, which optionally contains one or more groups, selected from

-O-, -C(O)-, -C(S)-,

$-NR^2-$, wherein $R^2$ is hydrogen, a univalent, linear cyclic or branched, saturated, unsaturated or aromatic hydrocarbon radical having up to 300 carbon atoms, which may contain one or more groups selected from -O-, -NH-, -C(O)- and -C(S)- and which is optionally substituted by one or more substituents selected from the group consisting of a hydroxyl group, a optionally substituted, preferably one or more nitrogen atoms containing heterocyclic group, polyether radicals, polyetherester radicals, polyorganosiloxanyl radicals and $-Si(OR)_{3-a}(R')_a$,

wherein a is an integer of 0 to 2 and R and R' may be the same or different and are each an organic radical, wherein if multiple groups $-NR^2-$ are present, these may be the same or different and with the proviso that the group $-NR^2-$ is bonded to a carbonyl carbon atom and/or thiocarbonyl carbon atom,

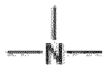

and

polyorganosiloxane radicals and which optionally may be substituted by one or more hydroxyl groups and/or groups of the formula (II)

$-Si(OR)_{3-a}(R')_a$

wherein a, R and R' are as defined above, and

wherein the terminal saturation of the groups Q and V which are connected to each other is done via monovalent organic radicals.

2. Compound according to claim 1, **characterized in that** it at least has a functional group of the formula (I):

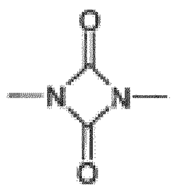

(I).

3. Compound according to claim 1, which at least has to units V, which contain a polyorganosiloxane radical.

4. Compound according to claim 1, **characterized in that** it contains at least two units Q.

5. Compound according to claim 1, **characterized in that** it at least has one quaternary ammonium group.

6. Compound according to claim 1, **characterized in that** that it at least has two quaternary ammonium groups.

7. Compound according to claim 1, **characterized in that** the polyorganosiloxane radical is a divalent group of the formula (III)

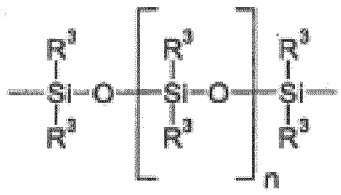

(III)

wherein $R^3$ may be the same or different and is selected from the group consisting of $C_1$ to $C_{22}$ alkyl, fluoro($C_3$-$C_{10}$) alkyl, $C_6$-$C_{10}$aryl and -W-Si(OR)$_{3-a}$(R')$_a$, wherein r, R' and a are as defined above and W is -O- or a divalent, linear, cyclic or branched, saturated, unsaturated or aromatic hydrocarbon radical having up to 100 carbon atoms, which optionally may contain one or more groups -C(O)-, -O-, -NH-, -S- and which may optionally be substituted by hydroxyl and n = 0 to 1000.

**8.** Compound according to one of the claims 1 to 7, **characterized in that** it at least contains a group of the formula (IV)

-[Q-V]- (IV)

wherein Q and V are as defined above and the terminal saturation of the groups Q and V is done via a univalent organic group.

**9.** Compound according to claim 8, **characterized in that** it has at least two repeating units of the formula (IV).

**10.** Compound according to one of the claims 1 to 9, **characterized in that** it at least contains a functional group (I) of the formula (Ia)

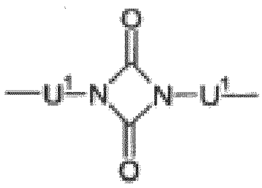

(Ia)

wherein
$U^1$ is selected from the group consisting of divalent radicals of the formulas:

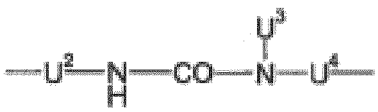

(Ib),

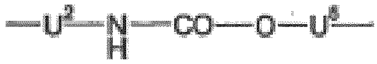

(Ic)

and

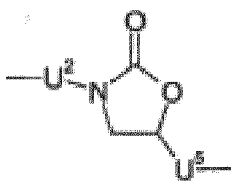

(Id),

wherein
$U^2$ is bonded to the nitrogen atom of the functional group of the formula (I), and
$U^2$ is a divalent, linear, cyclic or branched, saturated, unsaturated or aromatic hydrocarbon radical having up to 100 carbon atoms, which may contain one or more groups -O-,
$U^3$ is hydrogen or a univalent linear, cyclic or branched, saturated, unsaturated or aromatic hydrocarbon radical having up to 100 carbon atoms, which may contain one or more groups -O- and may be substituted by OH, may consist of $-W-Si(OR)_{3-a}(R')_a$, wherein R, R' are as defined above and a = 0 to 2 and W is a divalent, linear, cyclic or branched, saturated, unsaturated or aromatic hydrocarbon radical having up to 100 carbon atoms which may contain one or more groups -C(O)-, -O-, -NH-, -S- and may optionally be substituted by hydroxyl,
$U^4$ and $U^5$ are divalent linear, cyclic or branched, saturated, unsaturated or aromatic hydrocarbon radical having up to 1000 carbon atoms, which may optionally contain one or more groups selected from-O-, -C(O)-,

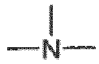 ,

$-NR^2-$, wherein $R^2$ is as defined above, and which may optionally be substituted by one or more hydroxyl groups, with the proviso that the groups

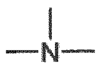

and $-NR^2-$ are bonded to a carbonyl carbon atom.

**11.** Process for the production of the amino and/or ammonium polysiloxane compounds according to any one of the claims 1 to 10, wherein

a) at least one amine compound selected from a diamine compound and/or a primary or secondary monoamine compound is reacted with a multi-, preferably difunctional organic compound capable of reacting with the amino functions of the amine compound, wherein the molar ratio of the amino functions of said amine compounds to the functional groups of said multi-, preferably difunctional organic compound is from about 0.5 to 2, or
b) at least two mole of an amine compound (1) selected from a diamine compound (1) and/or a primary or secondary monoamine compound (1) are reacted with one mole of a multi-, preferably difunctional organic compound (1) capable of reacting with the amino functions of the amine compound to form a diamine compound (2) (monomer), and the diamine compound (2) (monomer) is subsequently reacted with at least a further multi-, preferably difunctional organic compound (2) capable of reacting with the amino function of the diamine compound (2), optionally in the presence of further amine compounds (2), wherein the stoichiometry of the amino functions and the functional groups capable of reacting with amino functions is 1:1 in the last step of the reaction and the organic compounds (1) and (2) are the same or different from each other, or
c) a amine compound selected from a diamine compound (1) and/or a primary or secondary monoamine compound is reacted with a multi-, preferably difunctional organic compound (1) capable of reacting with the amino functions of the amine compound to form a diamine compound (2) (amino terminated oligomer), wherein the molar ratio of the amino functions of said amine compounds to the functional groups of said multi-, preferably difunctional organic compound (1) is from about 1 to 2,
the diamine compound (2) (amino terminated oligomer) is subsequently reacted with at least a further multi-, preferably difunctional organic compound (2) capable of reacting with the amino functions of the diamine compound, wherein the stoichiometry of the amino functions and the functional groups capable of reacting with amino functions is 1:1 in the last step of the reaction and the organic compounds (1) and (2) are the same or different from each other, or
d) an amine compound (1) selected from a diamine compound and/or a primary or secondary monoamine compound is reacted with a multi-, preferably difunctional organic compound (1) capable of reacting with the amino functions of the amine compound to form a multi-, preferably difunctional organic compound (2) (difunctional oligomer) capable of reacting with amino functions, wherein the molar ratio of the amino functions of said amine compounds to the functional groups of said multi-, preferably difunctional organic compound (1) is from about 0.5 to 1, the organic group (2) (difunctional oligomer) is subsequently reacted with at least one further amine compound (2) selected from a diamine compound and/or a primary or secondary monoamine compound, optionally in the presence of one or more multi-, preferably difunctional organic compound (3) capable of reacting

with amino functions, wherein the stoichiometry of the amino functions and the functional groups capable of reacting with amino functions is 1:1 in the last step of the reaction,

wherein optionally monofunctional, preferably tertiary monoamines or suitable monoamines not capable of chain propagation and/or monofunctional compounds capable of reacting with amino functions may be added as chain termination agents and wherein optionally present amino functions in the resulting product are subsequently protonated or quaternated.

**12.** Process according to claim 11, wherein the introduction of the functional group of the formula (I) comprises:

a) The reaction of diisocyanates containing the functional group of the formula (I) with at least one mole of a diamine (1) to from a monomeric, oligomeric or polymeric diamine (2), which contains the functional group of formula (I), or
b) the reaction of one mole of a diisocyanate containing the functional group of the formula (I) with at least one mole of a multi-, preferably difunctional organic compound (1) capable of reacting with isocyanate groups and amino groups to form a multi-, preferably difunctional monomeric, oligomeric or polymeric organic compound (2) capable of reacting with amino groups containing the functional group of formula (I), or
c) the reaction of one mole of a diisocyanate containing the functional group of the formula (I) with at least mole of a multi-, preferably difunctional organic compound (1) capable of reacting with isocyanate groups to form a multi-, preferably difunctional monomeric, oligomeric or polymeric organic compound (2) containing the functional group of the formula (I) and terminal groups capable of reacting with isocyanate groups, transfer of said organic compounds (2) to a multi-, preferably difunctional, monomeric, oligomeric or polymeric organic compound (3) capable of reacting with amino groups

and use of the obtained compounds containing the group of the formula (I) in the processes a) to d) of claim 22.

**13.** Process according to claim 11 or 12, wherein the introduction of the functional group of the formula (VIIIa) comprises the reaction of a amine compound selected from a diamine compound and/or a primary, secondary or tertiary monoamine compound containing the unit of the formula (VIIIa) and/or the reaction of a multi-, preferably difunctional organic compound containing the unit of the formula (VIIIa).

**14.** Process according to any one of the claims 11 to 13, wherein the functional groups of the multi-, preferably difunctional organic compounds capable of reacting with amino functions are selected from the group consisting of epoxy groups and haloalkyl groups.

**15.** Formulations containing at least one compound according to one of the claims 1 to 10.

**16.** Formulations according to claim 15 containing at least one solvent selected from water and organic solvents.

**17.** Formulations according to claims 15 or 16 in the form of an aqueous emulsion.

**18.** Formulations according to claim 17 in the form of a aqueous microemulsion.

**19.** Detergent formulation containing at least one compound according to one of the claims 1 to 10.

**20.** Detergent formulation according to claim 19 containing nonionic and/or anionic surfactants.

**21.** Cosmetic formulations containing at least one compound according to one of the claims 1 to 10.

**22.** Use of the compounds according to one of the claims 1 to 10 for the production of formulations for the equipment or treatment of natural or synthetic fibers or fibrous substrates in cosmetic applications.

**23.** Process for the treatment and/or equipment of natural or synthetic fibers or fibrous substrates comprising the wetting treatment of natural or synthetic fibers or fibrous substrates und optionally the activation with at least one of the compounds according to one of the claims 1 to 10 or with at least one of the formulations according to claim 16 to 18.

**24.** Natural or synthetic fibers or fibrous substrates treated with at least one of the compunds according to one of the claims 1 to 10.

**25.** Products produced from the fibers or fibrous substrates of claim 24.

**26.** Use of the compounds according to one of the claims 1 to 10 and formulations according to claims 16 - 18 in cosmetic formulations, in detergents or for the surface treatment of substrates.

## Revendications

**1.** Composés de polysiloxane amino et/ou ammonium réactifs et les sels de ceux-ci, **caractérisés par le fait qu'**ils ont au moins un groupe fonctionnel, sélectionné parmi les groupes des formules (I) et (VIIIa) :

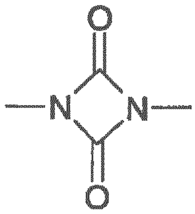

(I),

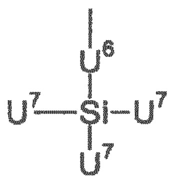

(VIIIa),

dans lesquels
$U^6$ représente un résidu d'hydrocarbure divalent à chaine droite, cyclique ou ramifié, saturé, insaturé ou aromatique ayant jusqu'à 100 atomes de carbone, qui, le cas échéant, peut contenir un ou plusieurs groupes, sélectionnés parmi -O-, -C(O)- et $-NU^8-$, ou, le cas échéant, peut être substitué par un ou plusieurs groupes hydroxyles, dans lequel $U^8$ représente l'hydrogène ou un résidu d'hydrocarbure monovalent à chaine droite, cyclique ou ramifié, saturé, insaturé ou aromatique ayant jusqu'à 100 atomes de carbone, qui peut contenir un ou plusieurs groupes -O- et peut être substitué par OH, à condition que -NH- et $NU^8$ sont liés à un atome de carbone carbonyle et/ou thiocarbonyle, et
$U^7$ représente un résidu d'hydrocarbure monovalent à chaine droite, cyclique ou ramifié, saturé, insaturé ou aromatique ayant jusqu'à 20 atomes de carbone, qui peut contenir un ou plusieurs groupes -O- et peut être substitué par OH,
à condition que les résidus $U^7$ peuvent être égaux ou différents et
au moins un résidu $U^7$ par atome de silice est lié à l'atome de silice par l'intermédiaire de -O-,
dans lequel le groupe de la formule (VIIIa) est contenu en tant que résidu $R^1$ de l'unité Q, tel que défini en ce qui suit, et qu'il s'agit des composés qui ont au moins trois unités sélectionnés des unités Q et V,
dans lequel Q est au moins un groupe amino et/ou ammonium di-, tri- et/ou tétravalent, qui n'est pas lié à V par l'intermédiaire d'un atome de carbone carbonylique, qui est sélectionné parmi le groupe consistant en :

$-NR^1-$,

$-N^+R^1_2-$,

un hétérocycle diaminofonctionnel, le cas échéant, substitué avec des substituants supplémentaires, saturé ou insaturé, des formules :

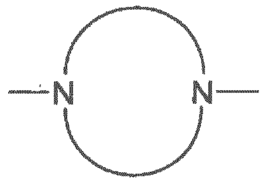

,

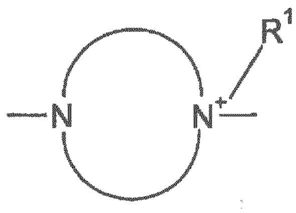

et

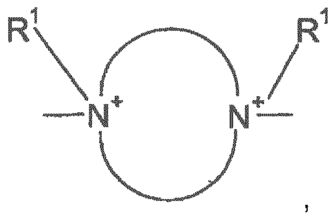

,

ainsi que un hétérocycle diaminofonctionnel, le cas échéant, substitué, aromatique, de la formul

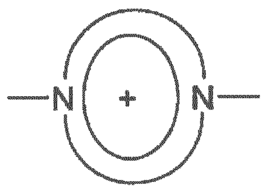

un résidu trivalent de la formule :

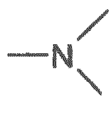

,

un résidu trivalent de la formule :

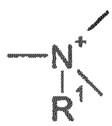

,

ou
un résidu tétravalent de la formule,

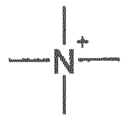

dans lequel $R^1$ est l'hydrogène ou un résidu organique monovalent, respectivement, dans lequel Q ne se lie pas à un atome de carbone carbonylique,

et

V est au moins une unité organique, qui est lié avec les unités Q par l'intermédiaire de carbone,

à condition qu'au moins l'une des unités V contient un résidu de polyorganosiloxane, et dans lequel

l'unité V est sélectionnée parmi au moins un résidu d'hydrocarbure polyvalent à chaine droite, cyclique ou ramifié, saturé, insaturé ou aromatique ayant jusqu'à 1000 atomes de carbone, les atomes de carbones du résidu polyorganosiloxane présent le cas échéant n'étant pas pris en considération dans le comptage, qui, le cas èchent, peut contenir un ou plusieurs groupes, sélectionnés parmi

-O-, -C(O)-, -C(S)-,

$-NR^2-$, dans lequel $R^2$ représente l'hydrogène, un résidu d'hydrocarbure monovalent à chaine droite, cyclique ou ramifié, saturé, insaturé ou aromatique ayant jusqu'à 300 atomes de carbone, qui peut contenir un ou plusieurs groupes, sélectionnés parmi -O-, -NH-, -C(O)- et -C(S)- et qui, le cas échéant, peut être substitué par un ou plusieurs substituants, sélectionné parmi le groupe consistant en un groupe hydroxyle, un groupe hétérocyclique, le cas échéant substitué, préférablement contenant un ou plusieurs atomes d'azote, les résidus polyéther, les résidus polyétheréther, les résidus polyorganosiloxanyle et

$-Si(OR)_{3-a}(R')_a$

dans lequel a est un nombre entier de 0 à 2 et R et R' peuvent être égaux ou différents l'un de l'autre et chacun repesent un résidu organique,

dans lequel lorsque il y a plusieurs groupes $-NR^2-$, ceux-ci peuvent être égaux ou différents, et à condition que le groupe $-NR^2-$ se lie à un atome de carbone carbonylique et/ou thiocarbonylique, peut contenir

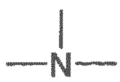

et

des résidus polyorganosiloxane, et qui, le cas échéant, peut être substitué par un ou plusieurs groupes hydroxyle et/ou groups de la formule (II)

$-Si(OR)_{3-a}(R')_a$

dans laquelle a, R et R' sont tels que définis là-dessus, et

dans lequel la saturation terminale des groupes polyvalents Q et V liés l'un à l'autre est mis en oeuvre par l'intermédiaire de résidus organiques monovalents.

2. Composé selon la revendication 1, **caractérisés par le fait qu'**il a au moins un groupe fonctionelle de la formule (I) :

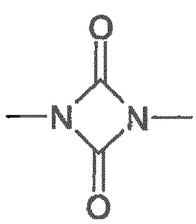

(I).

3. Composé selon la revendication 1, qui a au moins deux unités V, qui contiennent un résidu polyorganosiloxane.

**4.** Composé selon la revendication 1, **caractérisés par le fait qu'**il a au moins deux unités Q.

**5.** Composé selon la revendication 1, **caractérisés par le fait qu'**il a au moins un groupe ammonium quaternaire.

**6.** Composé selon la revendication 1, **caractérisés par le fait qu'**il a au moins deux groupes d'ammonium quaternaire.

**7.** Composé selon la revendication 1, **caractérisés par le fait que** le résidu Polyorganosiloxan est un groupe divalent de la formule (III)

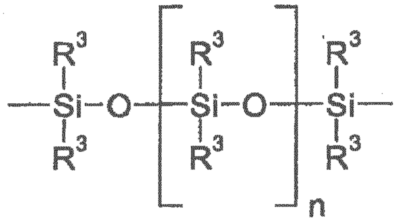

(III)

dans lequel $R^3$ peut être égal ou différent et est sélectionné parmi le groupe qui consiste en l'alkyle en $C_1$ à $C_{22}$, le flouroalkyle en ($C_3$-$C_{10}$), l'aryle en $C_6$-$C_{10}$ et $-W-Si(OR)_{3-a}(R')_a$, dans lequel R, R' et a sont tels que définis là-dessus et W représente -O- ou un résidu d'hydrocarbure divalent à chaine droite, cyclique ou ramifié, saturé, insaturé ou aromatique ayant jusqu'à 100 atomes de carbone, qui peut contenir un ou plusieurs groupes -C(O)-, -O-, -NH-, -S- et, le cas échéant, peut être substitué par hydroxy, et n = 0 à 1000.

**8.** Composé selon l'une des revendications 1 à 7, **caractérisés par le fait qu'**il contient au moins une unité de la formule (IV) :

-[Q-V]- (IV)

dans laquelle Q et V sont tels que définis là-dessus, et la saturation terminale des groupes Q et V est mise en oeuvre par des groupes monovalents organiques.

**9.** Composé selon la revendication 8, **caractérisés par le fait qu'**il a au moins deux unités répétitives de la formule (IV).

**10.** Composé selon l'une des revendications 1 à 9, **caractérisés par le fait qu'**il contient au moins un groupe fonctionnel (I) de la formule (la)

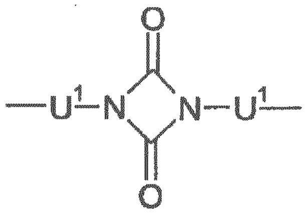

(Ia)

dans laquelle
$U^1$ est sélectionné parmi le groupe qui consiste en deux groupes des formules :

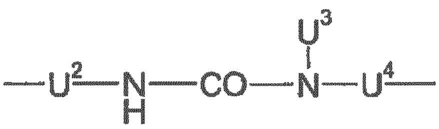

(Ib),

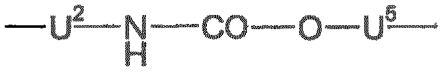

(Ic)

et

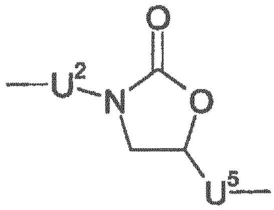

(Id),

dans lesquelles
$U^2$ est lié à l'atome d'azote du groupe fonctionnel de la formule (I), et
$U^2$ représente un résidu d'hydrocarbure divalent à chaine droite, cyclique ou ramifié, saturé, insaturé ou aromatique ayant jusqu'à 100 atomes de carbone, qui peut contenir un ou plusieurs groupes -O-,
$U^3$ représente l'hydrogène ou un résidu d'hydrocarbure monovalent à chaine droite, cyclique ou ramifié, saturé, insaturé ou aromatique ayant jusqu'à 100 atomes de carbone, qui peut contenir un ou plusieurs groupes -O- et peut être substitué par OH, consiste en $-W-Si(OR)_{3-a}(R')_a$, dans lequel R, R' sont tels que définis là-dessus et a = 0 à 2 et W représente un résidu d'hydrocarbure monovalent à chaine droite, cyclique ou ramifié, saturé, insaturé ou aromatique ayant jusqu'à 100 atomes de carbone, qui peut contenir un ou plusieurs groupes -C(O)-, -O-, -NH-, -S-, et, le cas échéant, peut être substitué par des groupes hydroxy,
$U^3$ et $U^4$ représentent des résidus d'hydrocarbure bivalents à chaine droite, cycliques ou ramifiés, saturés, insaturés ou aromatiques ayant jusqu'à 1000 atomes de carbone, qui, le cas échéant, peuvent contenir un ou plusieurs groupes sélectionnés parmi -C-, -C(O)-,

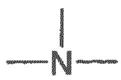,

$-NR^2-$, dans lequel $R^2$ est tel que défini là-dessus, et qui, le cas échéant peuvent être substitués par un ou plusieurs groupes hydroxyle, à condition que les groupes

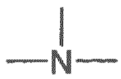

et $-NR^2-$ se lient à un atome de carbone carbonylique.

**11.** Procédé pour la production du composé de polysiloxane amino et/ou ammonium selon l'une des revendications 1 à 10, dans lequel

a) au moins composé amine, sélectionné parmi un composé diamine et/ou un composé monoamine primaire ou secondaire, est soumis à une réaction avec un composé organique multifonctionnel, préférablement bifonctionnel, capable de réagir avec les fonctions amino du composé amine, le rapport molaire des fonctions amine dudit composé amine aux groupes fonctionnels dudit composé organique multifonctionnel, préférablement bifonctionnel, allant d'environ 0,5 jusqu'à 2,
ou
b) au moins deux moles d'un composé amine (1), sélectionné parmi un composé diamine (1) et/ou un composé monoamine primaire ou secondaire (1) est soumis à une réaction avec une mole d'un composé organique (1) multifonctionnel, préférablement bifonctionnel, capable de réagir avec les fonctions amino du composé amine en formant un composé diamine (2) (monomère), et le composé diamine (2) (monomère) est ensuite soumis

à une réaction avec au moins un autre composé organique (2) multifonctionnel, préférablement bifonctionnel, capable de réagir avec les fonctions amino du composé amine (2), le cas échéant, en présence d'autres composés amine (2), la stoechiométrie des fonctions amino et des groupes fonctionnels capables de réagir avec des fonctions amino étant 1:1 dans la dernière étape de la réaction, et les composés organiques (1) et (2) pouvant être égaux ou différents l'un de l'autre, ou

c) un composé amine sélectionné parmi un composé diamine (1) et/ou un composé monoamine primaire ou secondaire est soumis à une réaction avec un composé organique (1) multifonctionnel, préférablement bifonctionnel, capable de réagir avec les fonctions amino des composés amine en formant un composé diamine (2) (oligomère aminoterminé), le rapport molaire des fonctions amine dudit composé amine aux groupes fonctionnels dudit composé organique multifonctionnel, préférablement bifonctionnel (1) étant environ 1 à 2, le composé diamine (2) (oligomère aminoterminé) obtenu est ensuite soumis à une réaction avec au moins un composé organique (2) multifonctionnel, préférablement bifonctionnel, capable de réagir avec les fonctions amino des composés diamine, la stoechiométrie des fonctions amino et des groupes fonctionnels capables de réagir avec des fonctions amino étant 1:1 dans la dernière étape de la réaction, et les composés organiques (1) et (2) pouvant être égaux ou différents,

ou

d) un composé amine (1) sélectionné parmi un composé diamine (1) et/ou un composé monoamine primaire ou secondaire est soumis à une réaction avec un composé organique (1) multifonctionnel, préférablement bifonctionnel, capable de réagir avec les fonctions amino du composé amine en formant un composé organique (2) multifonctionnel, préférablement bifonctionnel, capable de réagir avec des fonctions amino (oligomère bifonctionnel), le rapport molaire des fonctions amino dudit composé amine aux groupes fonctionnels dudit composé organique (1) multifonctionnel, préférablement bifonctionnel, étant environ 0,5 à 1,

le composé organique (2) (oligomère bifonctionnel) obtenu est ensuite soumis à une réaction avec au moins un composé amine (2) sélectionné parmi un composé diamine et/ou un composé monoamine primaire ou secondaire est soumis à une réaction, le cas échéant, en présence d'un ou plusieurs composés organiques (3) multifonctionnels, préférablement bifonctionnels, capables de réagir avec des fonctions amino, la stoechiométrie des fonctions amino et des groupes fonctionnels capables de réagir avec des fonctions amino étant 1:1 dans la dernière étape de la réaction;

dans lequel, le cas échéant, des monoamines monofonctionnels, préférablement des monoamines tertiaires, ou des monoamines n'étant pas apte à la propagation de chaine et/ou des composés monofonctionnels apte à la réaction avec des fonctions amino, le cas échéant, peuvent être ajoutées en tant qu'agent limiteur de chaîne, et dans lequel des fonctions amine présentes le cas échéant peuvent ensuite être protonées ou quaternées.

**12.** Procédé selon la revendication 11, dans lequel l'insertion du groupe fonctionnel de la formule (I) comprend :

a) la réaction de diisocyanates contenant le groupe fonctionnel de la formule (I) avec au moins une mole d'une diamine tout en formant une diamine (2) monomère, oligomère ou polymère, qui contient le groupe fonctionnel de la formule (I), ou

b) la réaction d'une mole d'un diisocyanate contenant le groupe fonctionnel de la formule (I) avec au moins une mole d'un composé organique (1) multifonctionnel, préférablement bifonctionnel, capable de réagir avec les groupes isocyanate et les groupes amino tout en formant un composé organique (2) monomère, oligomère ou polymère multifonctionnel, préférablement bifonctionnel, capable de réagir avec des groupes amino contenant le groupe de la formule (I), ou

c) la réaction d'une mole d'un diisocyanate contenant le groupe fonctionnel de la formule (I) avec au moins une mole d'un composé organique (1) multifonctionnel, préférablement bifonctionnel, capable de réagir avec les groupes isocyanate tout en formant un composé organique (2) monomère, oligomère ou polymère multifonctionnel, préférablement bifonctionnel, contenant le groupe fonctionnel de la formule (I) et des groupes terminales capable de réagir avec des groupes isocyanant, conversion dudit composé organique (2) en un composé organique (3) monomère, oligomère ou polymère multifonctionnel, préférablement bifonctionnel, capable de réagir avec des groupes amino

et utilisation des composés obtenus contenant le groupe de la formule (I) dans les procédés a) à d) de la revendication 11).

**13.** Procédé selon la revendication 11 ou 12, dans lequel l'insertion du groupe fonctionnel de la formule (VIIIa) comprend la réaction d'un composé amine sélectionné parmi un composé diamine et/ou un composé monoamine primaire, secondaire ou tertiaire contenant l'unité de la formule (VIIIa), et/ou la réaction d'un composé organique multifonctionnel, préférablement bifonctionnel, contenant l'unité de la formule (VIIIa).

**14.** Procédé selon l'une des revendications 11 à 13, dans lequel les groupes fonctionnels des composés multifonctionnels, préférablement bifonctionnels, capables de réagir avec des fonctions amino sont sélectionnés parmi le groupe consistant en les groupes époxy et les groupes alkyle halogéné.

**15.** Formulations contenant au moins un composé selon l'une des revendications 1 à 10.

**16.** Formulation selon la revendication 15, contenant au moins un solvant, sélectionné parmi l'eau et les solvants organiques.

**17.** Formulation selon les revendications 15 ou 16 en forme d'une émulsion aqueuse.

**18.** Formulation selon la revendication 17 en forme d'une microémulsion aqueuse.

**19.** Formulation de lessive contenant au moins un composé selon l'une des revendications 1 à 10.

**20.** Formulation selon la revendication 19 contenant les tensides non ionogènes et/ou anioniques.

**21.** Formulation cosmétique contenant au moins un composé selon l'une des revendications 1 à 10.

**22.** Utilisation des composés selon l'une des revendications 1 à 10 pour la production de formulations pour l'ennoblissement ou traitement de fibres naturelles ou synthétiques ou de substrats fibreux dans des applications cosmétiques.

**23.** Procédé pour le traitement et/ou l'ennoblissement qui comprend le traitement de mouillage de fibres naturelles ou synthétiques ou de substrats fibreux et, le cas échéant, l'activation avec au moins l'un des composés selon l'une des revendications 1 à 10 ou avec au moins l'une des formulations selon la revendication 16 à 18.

**24.** Fibres naturelles ou synthétiques ou substrats fibreux, traités avec au moins l'une des composés selon l'une des revendications 1 à 10.

**25.** Produits fabriqués des fibres naturelles ou synthétiques ou des substrats fibreux selon la revendication 24.

**26.** Utilisation des composés selon l'une des revendications 1 à 10 et des formulations selon la revendication 16 à 18 dans les formulations cosmétiques, les lessives ou pour le traitement de surface de substrats.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 441530 A
- US 5591880 A
- US 5650529 A
- US 5807956 A
- US 5981681 A
- US 6475568 B
- DE PS3719086 C
- US 6242554 B
- US 5098979 A
- US 5153294 A
- US 5166297 A
- EP 282720 A
- DE 3340708
- US 6240929 B
- DE 19817776
- DE 10051258
- DE 10036533
- WO 0210256 A
- WO 0210257 A
- WO 0210259 A
- DE 10251525
- DE 10251526
- US 6177511 B
- US 5602224 A
- DE 10212470
- DE 4211256
- WO 0127232 A
- US 4661577 A
- US 20020028900 A
- DE 3236466
- DE 10036522
- DE OS4318536 A
- WO 9914300 A


### In der Beschreibung aufgeführte Nicht-Patentliteratur


- **SILICONE.** Chemie und Technologie. Vulkan-Verlag, 1989, 82-84
- **B.MARCINIEC.** Comprehensive Handbook on Hydrosilylation. Pergamon Press, 1992, 122-124
- **B.MARCINIEC.** Comprehensive Handbook on Hydrosilylation. Pergamon Press, 1992, 116-121, 127-130, 134-137, 151-155
- Organikum, Organischchemisches Grundpraktikum. VEB Deutscher Verlag der Wissenschaften, 1988, 189-190
- **H.J. LAAS ; R.HALPAAP ; J. PEDAIN.** *Journal f. Prakt. Chemie,* 1994, vol. 336, 185-200
- **H.J. LAAS ; R.HALPAAP ; J. PEDAIN.** *Farbe+Lack,* 1994, vol. 100, 330-336